# EUROPEAN PATENT SPECIFICATION

(11) **EP 2 155 739 B1**
(45) Date of publication and mention of the grant of the patent: **06.10.2010**
(21) Application number: 08738121.6
(22) Date of filing: 13.05.2008
(51) Int. Cl.: C07D 417/06

(54) **2-CYCLOPROPYL-THIAZOLE DERIVATIVES**
2-CYCLOPROPYLTHIAZOLDERIVATE
DÉRIVÉS DE 2-CYCLOPROPYL-THIAZOLE

(30) Priority: 14.05.2007 WO PCT/IB2007/051808
(43) Date of publication of application: 24.02.2010
(73) Proprietor: Actelion Pharmaceuticals Ltd., 4123 Allschwil (CH)
(72) Inventor: AISSAOUI, Hamed, F-68840 Pulversheim (FR); BOSS, Christoph, CH-4123 Allschwil (CH); GUDE, Markus, CH-4123 Allschwil (CH); KOBERSTEIN, Ralf, 79539 Lörrach (DE); SIFFERLEN, Thierry, F-68220 Wentzwiller (FR)
(74) Representative: Schager, Frank
(86) International application number: PCT/IB2008/051883
(87) International publication number: WO 2008/139416

(56) References cited:
- WO-A-01/96302
- WO-A-03/051368

## Description

The present invention relates to selected 2-cyclopropyl-thiazole derivatives of formula (I), and their use as pharmaceuticals. The invention also concerns related aspects including processes for the preparation of the compounds, pharmaceutical compositions containing one or more compounds of formula (I), and especially their use as orexin receptor antagonists.

Orexins (orexin A or OX-A and orexin B or OX-B) are novel neuropeptides found in 1998 by two research groups, orexin A is a 33 amino acid peptide and orexin B is a 28 amino acid peptide (Sakurai T. et al., Cell, 1998, 92, 573-585). Orexins are produced in discrete neurons of the lateral hypothalamus and bind to G-protein-coupled receptors (OX₁ and OX₂ receptors). The orexin-1 receptor (OX₁) is selective for OX-A, and the orexin-2 receptor (OX₂) is capable to bind OX-A as well as OX-B. Orexins are found to stimulate food consumption in rats suggesting a physiological role for these peptides as mediators in the central feedback mechanism that regulates feeding behaviour (Sakurai T. et al., Cell, 1998, 92, 573-585). On the other hand, it was also observed that orexins regulate states of sleep and wakefulness opening potentially novel therapeutic approaches to narcolepsy as well as insomnia and other sleep disorders (Chemelli R.M. et al., Cell, 1999, 98, 437-451).

Orexin receptors are found in the mammalian brain and may have numerous implications in pathologies as known from the literature.

The present invention provides 2-cyclopropyl-thiazole derivatives, which are non-peptide antagonists of human orexin receptors. These compounds are in particular of potential use in the treatment of e.g. eating disorders, drinking disorders, sleep disorders, or cognitive dysfunctions in psychiatric and neurologic disorders.

Up to now, several low molecular weight compounds are known having a potential to antagonise either specifically OX₁ or OX₂, or both receptors at the same time. Piperidine derivatives useful as orexin receptor antagonists are disclosed in WO 01/96302 and WO 03/051368.
i) A first aspect of the invention relates to compounds of formula (I) 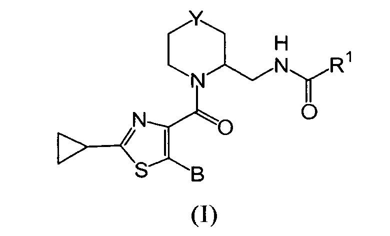 wherein
   Y represents (CH₂)ₙ, wherein n represents 0 or 1;
   B represents phenyl, wherein the phenyl ring is unsubstituted or mono-, di- or tri-substituted, wherein the substituents are independently selected from the group consisting of (C₁₋₄)alkyl, (C₁₋₄)alkoxy, trifluoromethyl, and halogen; and
   R¹ represents aryl or heterocyclyl, wherein the aryl or heterocyclyl is independently unsubstituted or mono-, di-, or tri-substituted wherein the substituents are independently selected from the group consisting of (C₁₋₄)alkyl, (C₁₋₄)alkoxy, halogen, cyano, trifluoromethyl and -NH-CO-(C₁₋₄)alkyl;
   or R¹ represents a 2,3-dihydro-benzofuranyl-, a benzo[1,3]dioxolyl-, a 2,3-dihydrobenzo[1,4]dioxinyl-, a 4*H*-benzo[1,3]dioxinyl-, a 2*H*-chromenyl, a chromanyl-, a 2,3-dihydro-thieno[3,4-b][I,4]dioxinyl-, a 3,4-dihydro-2*H*-benzo[1,4]oxazinyl-, or an indenyl group; which groups are unsubstituted or mono- or di-substituted wherein the substituents are independently selected from the group consisting of (C₁₋₄)alkyl, (C₁₋₄)alkoxy and halogen.
ii) A second embodiment of the invention relates to compounds of formula (I) according to embodiment i), wherein
   Y represents (CH₂)ₙ, wherein n represents 0 or 1;
   B represents phenyl, wherein the phenyl ring is unsubstituted or mono-, di- or tri-substituted, wherein the substituents are independently selected from the group consisting of (C₁₋₄)alkyl, (C₁₋₄)alkoxy, trifluoromethyl, and halogen; and
   R¹ represents aryl or heterocyclyl, wherein the aryl or heterocyclyl is independently unsubstituted or mono-, di-, or tri-substituted wherein the substituents are independently selected from the group consisting of (C₁₄)alkyl, (C₁₋₄)alkoxy, halogen, cyano and trifluoromethyl;
   or R¹ represents a 2,3-dihydro-benzofuranyl-, a benzo[1,3]dioxolyl-, a 2,3-dihydro-benzo[ 1,4]dioxinyl-, or a 4*H*-benzo[1,3]dioxinyl group which groups are unsubstituted or mono- or di-substituted wherein the substituents are independently selected from the group consisting of (C₁-₄)alkyl, (C₁₋₄)alkoxy and halogen.

   Also part of the invention are compounds of formula (I) (and Ia; see below) and pharmaceutically acceptable salts thereof.
   In this patent application, a dotted line shows the point of attachment of the radical drawn. For example, the radical drawn below 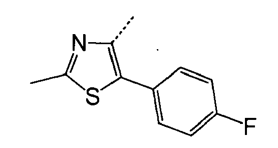 is the 5-(4-fluoro-phenyl)-2-methyl-thiazol-4-yl group.
   The general terms used hereinbefore and hereinafter preferably have, within this disclosure, the following meanings, unless otherwise indicated:
   The term "halogen" means fluorine, chlorine, or bromine; preferably it means fluorine and chlorine, especially fluorine.
   The term "(C₁₋₄)alkyl", alone or in combination, means a straight-chain or branched-chain alkyl group with 1 to 4 carbon atoms. Examples of (C₁₋₄)alkyl groups are methyl, ethyl, propyl, isopropyl, n-butyl, isobutyl, sec.-butyl and tert.-butyl. Preferred are methyl and ethyl.
   For B the term "(C₁₋₄)alkyl" has the above meaning; preferably it means methyl and ethyl, especially methyl.
   The term "(C₁₋₄)alkoxy", alone or in combination, means a group of the formula (C₁₋₄)alkyl-O- in which the term "(C₁₋₄)alkyl" has the previously given significance, such as methoxy, ethoxy, n-propoxy, isopropoxy, n-butoxy, isobutoxy, sec.-butoxy and tert.-butoxy. Preferred are methoxy and ethoxy, especially methoxy.
   An example of a -NH-CO-(C₁₋₄)alkyl group is -NH-CO-CH₃.
   The term "aryl", alone or in combination, means a phenyl or a naphthyl group. Preferred is a phenyl group. The aryl group is unsubstituted or mono-, di-, or tri-substituted wherein the substituents are independently selected from the group consisting of (C₁₋₄)alkyl, (C₁₋₄)alkoxy, halogen, cyano, trifluoromethyl and -NH-CO-(C₁₋₄)alkyl. Especially, the aryl group is unsubstituted or mono-, di-, or tri-substituted wherein the substituents are independently selected from the group consisting of (C₁₋₄)alkyl, (C₁₋₄)alkoxy, trifluoromethyl, and halogen.
   Examples wherein R¹ represents "aryl" are phenyl, naphthyl (notably 1-naphthyl), 2-methylphenyl, 3-methylphenyl, 4-methylphenyl, 2,3-dimethylphenyl, 2,4-dimethylphenyl, 3,4-dimethylphenyl, 3,5-dimethylphenyl, 4-methyl-3-trifluoromethylphenyl, 2-methoxyphenyl, 3-methoxyphenyl, 4-methoxyphenyl, 2,5-dimethoxyphenyl, 3,4-dimethoxyphenyl, 3,5-dimethoxyphenyl, 2-fluorophenyl, 3-fluorophenyl, 4-fluorophenyl, 3,4-difluorophenyl, 3-fluoro-2-methylphenyl, 2-chlorophenyl, 3-chlorophenyl, 4-chlorophenyl, 2,3-dichlorophenyl, 3,4-dichlorophenyl, 3-chloro-2-methylphenyl, 2-chloro-3-methylphenyl, 2-chloro-3-fluorophenyl, 2-bromophenyl, 3-bromophenyl, 4-bromophenyl, 2-bromo-3-methylphenyl, 2-cyanophenyl, 3-cyanophenyl, 4-cyanophenyl, 2-trifluoromethylphenyl, 3-trifluoromethylphenyl, 4-trifluoromethylphenyl, and 3-acetylaminophenyl. Especially, examples wherein R¹ represents "aryl" are phenyl, naphthyl (notably 1-naphthyl), 3-methylphenyl, 2,3-dimethylphenyl, 4-methyl-3-trifluoromethylphenyl, 3-methoxyphenyl, 2,5-dimethoxyphenyl, 3,4-dimethoxyphenyl, 3,5-dimethoxyphenyl, 3-fluoro-2-methylphenyl, 3-chlorophenyl, 3,4-dichlorophenyl, 3-chloro-2-methylphenyl, 2-chloro-3-methylphenyl, 2-chloro-3-fluorophenyl, 3-bromophenyl, 2-bromo-3-methylphenyl, and 3-trifluoromethylphenyl. Most preferred are 3-methylphenyl, 3-chlorophenyl and 3-bromophenyl.
   Examples wherein "B" represents "phenyl, wherein the phenyl ring is unsubstituted or mono-, di- or tri-substituted, wherein the substituents are independently selected from the group consisting of (C₁₋₄)alkyl, (C₁₋₄)alkoxy, trifluoromethyl, and halogen" are: phenyl, 2-methylphenyl, 3-methylphenyl, 4-methylphenyl, 2,3-dimethylphenyl, 2,4-dimethylphenyl, 3,4-dimethylphenyl, 3,5-dimethylphenyl, 2-methoxyphenyl, 3-methoxyphenyl, 4-methoxyphenyl, 2-fluorophenyl, 3-fluorophenyl, 4-fluorophenyl, 3,4-difluorophenyl, 2-chlorophenyl, 3-chlorophenyl, 4-chlorophenyl, 2,3-dichlorophenyl, 3,4-dichlorophenyl, 2-bromophenyl, 3-bromophenyl, 4-bromophenyl, 2-trifluoromethylphenyl, 3-trifluoromethylphenyl, and 4-trifluoromethylphenyl. Especially, examples are phenyl, 3-methylphenyl, 4-methylphenyl, 2-fluorophenyl, 4-fluorophenyl, 3-chlorophenyl, and 3-trifluoromethylphenyl.
   The term "heterocyclyl", alone or in combination, means a 5- to 10-membered monocyclic or bicyclic aromatic ring containing for example 1, 2 or 3 heteroatoms independently selected from oxygen, nitrogen and sulfur. Examples of such heterocyclyl groups are furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidyl, pyridazinyl, pyrazinyl, indolyl, isoindolyl, benzofuranyl, isobenzofuranyl, benzothiophenyl, indazolyl, benzimidazolyl, benzoxazolyl, benzisoxazolyl, benzothiazolyl, benzotriazolyl, benzoxadiazolyl, benzothiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidyl, imidazo[1,2-a]pyridyl and (especially) imidazo[2,1-b]thiazolyl. In addition to the above list of examples, further examples are benzoisothiazolyl, thienopyrazinyl, furopyrrolyl, and pyrrolo[2,1-b] thiazolyl. In another embodiment, preferred examples are isoxazolyl, pyrazolyl, pyridyl, indolyl, benzofuranyl, indazolyl, benzisoxazolyl, quinolinyl, isoquinolinyl, imidazo [1,2-a]pyridyl, thienopyrazinyl, furopyrrolyl and (especially) imidazo[2,1-b]thiazolyl. Most preferred are indolyl, benzofuranyl, indazolyl, benzisoxazolyl, quinolinyl and (especially) imidazo[2,1-b]thiazolyl. The above-mentioned heterocyclyl groups are unsubstituted or mono-, di-, or tri-substituted wherein the substituents are independently selected from the group consisting of (C₁₋₄)alkyl, (C₁₋₄)alkoxy, halogen, cyano, trifluoromethyl and -NH-CO-(C₁₋₄)alkyl. Especially, the above-mentioned heterocyclyl groups are unsubstituted, mono-, di-, or tri-substituted wherein the substituents are independently selected from the group consisting of (C₁₋₄)alkyl, (C₁₋₄)alkoxy, halogen, and trifluoromethyl. Preferably, the above-mentioned heterocyclyl groups are unsubstituted, mono-, or di-substituted wherein the substituents are independently selected from the group consisting of (C₁₋₄)alkyl and halogen. In a further preferred embodiment the above-mentioned groups are unsubstituted or mono-substituted wherein the substituent is selected from (C₁₋₄)alkyl (especially the substituent is methyl).
   In another embodiment, preferred examples of such heterocyclyl groups are selected from the group consisting of isoxazol-5-yl, pyrazol-5-yl, pyridin-3-yl, indol-2-yl, indol-3-yl, indol-4-yl, indol-5-yl, benzofuran-4-yl, indazol-3-yl, benzisoxazol-3-yl, quinoline-8-yl, isoquinoline-1-yl, imidazo[1,2-a]pyridine-3-yl, imidazo[2,1-b]thiazol-5-yl; imidazo[2,1-b]thiazol-6-yl, thieno[2,3-b]pyrazin-6-yl, and 4H-furo[3,2-b]pyrrol-5-yl (especially imidazo[2,1-b]thiazol-5-yl); wherein the above-mentioned heterocyclyl groups are unsubstituted, mono-, or di-substituted, wherein the substituents are independently selected from (C₁₋₄)alkyl and halogen (especially (C₁₋₄)alkyl).
   In particular, the above mentioned heterocyclyl groups as used for the substituent "R¹" are preferably substituted as follows: isoxazolyl groups are mono-substituted with (C₁₋₄)alkyl; pyrazolyl groups are di-substituted with (C₁₋₄)alkyl; pyridyl groups are mono-substituted with (C₁₋₄)alkyl; indolyl groups are unsubstituted, or independently mono- or di-substituted with (C₁₋₄)alkyl or halogen (especially unsubstituted, or mono- or di-substituted with methyl); benzofuranyl groups are unsubstituted; indazolyl groups are unsubstituted, or mono-substituted with (C₁₋₄)alkyl (especially methyl); benzisoxazolyl groups are unsubstituted; quinolinyl groups are unsubstituted; isoquinolinyl groups are unsubstituted; imidazo[1,2-a]pyridinyl groups are unsubstituted; imidazo[2,1-b]thiazolyl groups are unsubstituted, or mono-substituted with (C₁₋₄)alkyl; thienopyrazinyl groups are unsubstituted; and furopyrrolyl groups are mono-substituted with (C₁₋₄)alkyl;.
   In another embodiment, preferred examples wherein R¹ represents "heterocyclyl" are: 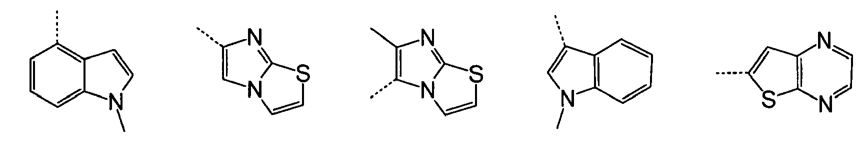 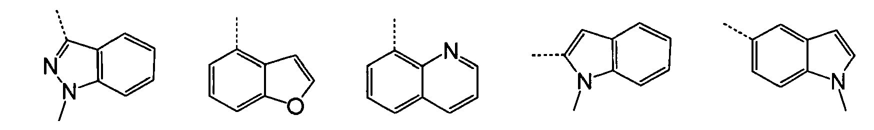 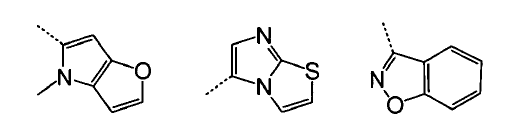
   In case R¹ is different from "aryl" and "heterocyclyl", it presents a 2,3-dihydro-benzofuranyl-, a benzo[1,3]dioxolyl-, a 2,3-dihydro-benzo[1,4]dioxinyl-, a 4*H*-benzo[1,3]dioxinyl-, a 2*H*-chromenyl, a chromanyl-, a 2,3-dihydro-thieno[3,4-b] [1,4]dioxinyl-, a 3,4-dihydro-2*H*-benzo[1,4]oxazinyl-, or an indenyl group. Especially it presents 2,3-dihydro-benzofuranyl-, a benzo[1,3]dioxolyl-, a 2,3-dihydro-benzo[1,4]dioxinyl-, a 4*H*-benzo[1,3]dioxinyl-, a 2,3-dihydro-thieno[3,4-b] [1,4]dioxinyl-, a 3,4-dihydro-2*H*-benzo[1,4]oxazinyl-, or an indenyl group. Preferably it presents a 2,3-dihydro-benzofuranyl-, a benzo[1,3]dioxolyl-, a 2,3-dihydro-benzo[1,4]dioxinyl-, or a 4*H*-benzo[1,3]dioxinyl-group. Most preferably it presents a 2,3-dihydro-benzofuranyl-, or a 2,3-dihydro-benzo[1,4]dioxinyl-group. The above-mentioned groups as used for the substituent R¹ are unsubstituted or mono- or di-substituted wherein the substituents are independently selected from the group consisting of (C₁₋₄)alkyl, (C₁₋₄)alkoxy and halogen. Preferably the above-mentioned groups are unsubstituted or mono-substituted with (C₁₋₄)alkyl.
   In particular, in case R¹ is different from "aryl" and "heterocyclyl", the above mentioned groups as used for the substituent "R¹" carry attachment points to the rest of the molecule, and are preferably substituted as follows: 2,3-dihydro-benzofuranyl-groups (especially 2,3-dihydro-benzofuran-4-yl or 2,3-dihydro-benzofuran-7-yl), benzo[1,3]dioxolyl-groups (especially benzo[1,3]dioxol-4-yl), 2,3-dihydro-benzo[1,4]dioxinyl- (especially 2,3-dihydro-benzo[1,4]dioxin-5-yl), 4*H*-benzo[1,3]dioxinyl-groups (especially 4*H*-benzo[1,3]dioxin-8-yl or 4*H*-benzo[1,3]dioxin-5-yl), 2*H*-chromenyl (especially 2*H*-chromen-5-yl), chromanyl-(especially chroman-5-yl or chroman-8-yl), 2,3-dihydro-thieno[3,4-b][1,4]dioxinyl-(especially 2,3-dihydro-thieno[3,4-b][1,4]dioxine-5-yl), 3,4-dihydro-2*H*-benzo[1,4]oxazinyl-groups (especially 3,4-dihydro-2*H*-benzo[1,4]oxazin-5-yl or 3,4-dihydro-2*H-*benzo[1,4]oxazin-8-yl) are preferably unsubstituted; indenyl-groups (especially 1H-inden-2-yl) are preferably mono-substituted with methyl.
   Preferably, in case R¹ is different from "aryl" and "heterocyclyl", it represents 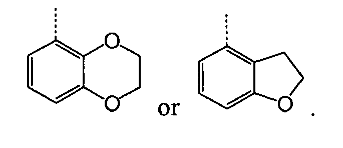
iii) A further embodiment of the invention comprises compounds of formula (I) according to embodiments i) or ii), which are also compounds of formula (Ia), wherein the indicated stereogenic center is in the (S)-configuration 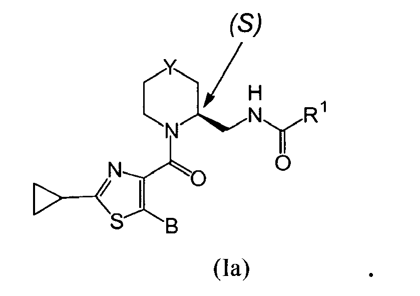
iv) A further embodiment of the invention comprises compounds of formula (I) according to any one of embodiments i) to iii), wherein B represents phenyl, wherein the phenyl ring is unsubstituted or mono- or di-substituted, wherein the substituents are independently selected from the group consisting of (C₁₋₄)alkyl, (C₁₋₄)alkoxy, trifluoromethyl, and halogen.
v) A further embodiment of the invention comprises compounds of formula (I) according to any one of embodiments i) to iv), wherein B represents phenyl, wherein the phenyl ring is unsubstituted or mono-substituted, wherein the substituent is selected from the group consisting of methyl, chloro, fluoro, and trifluoromethyl.
vi) A further embodiment of the invention comprises compounds of formula (I) according to any one of embodiments i) to v), wherein n represents 0.
vii) A further embodiment of the invention comprises compounds of formula (I) according to any one of embodiments i) to v), wherein n represents 1.
viii) A further embodiment of the invention comprises compounds of formula (I) according to any one of embodiments i) to vii), wherein
   R¹ represents phenyl, which is mono- (preferred) or di-substituted; wherein preferably one substituent is in position 3 and, if present, the other substituent in position 4; wherein the substituents are independently selected from methoxy, chloro, bromo and methyl (especially from chloro, bromo and methyl);
   or R¹ represents heterocyclyl, wherein the heterocyclyl is selected from the group consisting of indolyl, benzofuranyl, indazolyl, benzisoxazolyl, quinolinyl and (especially) imidazo[2,1-b]thiazolyl, wherein said heterocyclyl is unsubstituted or mono-substituted wherein the substituent is selected from (C₁₋₄)alkyl;
   or R¹ represents a 2,3-dihydro-benzofuranyl-, or a 2,3-dihydro-benzo[1,4]dioxinyl-group, which groups are unsubstituted.
ix) A further embodiment of the invention comprises compounds of formula (I) according to any one of embodiments i) to viii), wherein R¹ represents one of the following groups: 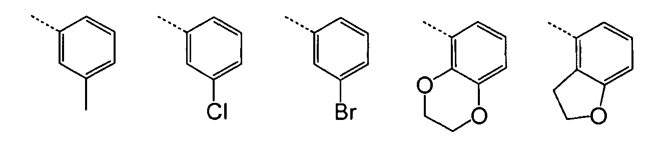 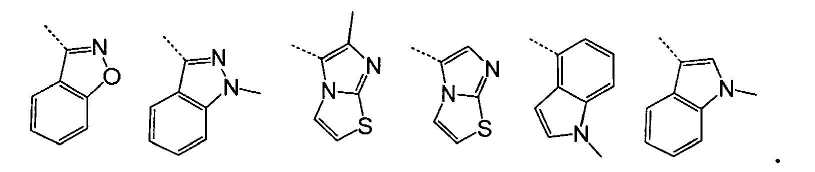
x) A further embodiment of the invention comprises compounds of formula (I) according to any one of embodiments i) to ix), wherein R¹ represents one of the following groups: 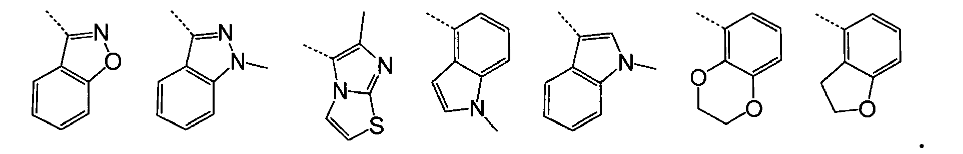
xi) A further embodiment of the invention comprises compounds of formula (I) according to any one of embodiments i) to x), wherein R¹ represents 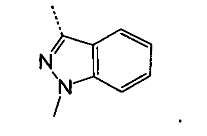
xii) A further embodiment of the invention comprises compounds of formula (I) according to any one of embodiments i) to x), wherein R¹ represents 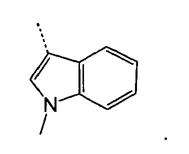
xiii) A further embodiment of the invention comprises compounds of formula (I) according to any one of embodiments i) to viii), wherein R¹ represents 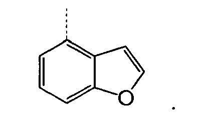
xiv) A further embodiment of the invention comprises compounds of formula (I) according to any one of embodiments i) to x), wherein R¹ represents 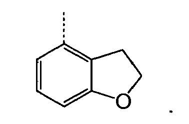
xv) A further embodiment of the invention comprises compounds of formula (I) according to any one of embodiments i) to ix), wherein R¹ represents 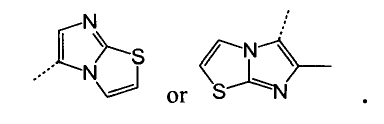
xvi) A further embodiment of the invention comprises compounds of formula (I) according to any one of embodiments i) to ix), wherein R¹ represents 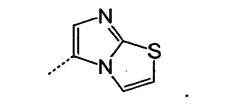
xvii) A further embodiment of the invention comprises compounds of formula (I) according to any one of embodiments i) to x), wherein R¹ represents 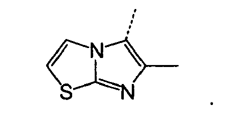
xviii) A further embodiment of the invention comprises compounds of formula (I) according to any one of embodiments i) to viii), wherein R¹ represents 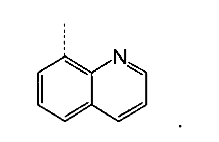
xix) A further embodiment of the invention comprises compounds of formula (I) according to any one of embodiments i) to vii), wherein R¹ represents 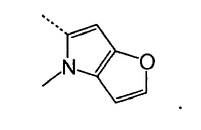
xx) A further embodiment of the invention comprises compounds of formula (I) according to any one of embodiments i) to x), wherein R¹ represents 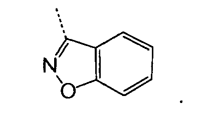
xxi) A further embodiment of the invention comprises compounds of formula (I) according to any one of embodiments i) to x), wherein R¹ represents 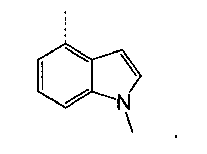
xxii) A further embodiment of the invention comprises compounds of formula (I) according to any one of embodiments i) to x), wherein R¹ represents 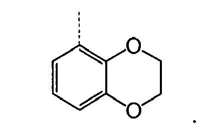
xxiii) A further embodiment of the invention comprises compounds of formula (I) according to any one of embodiments i) to viii), wherein R¹ represents phenyl which is mono-substituted (especially in position 3) wherein the substituent is selected from methoxy, chloro, bromo and methyl (especially chloro, bromo and methyl).
xxiv) A further embodiment of the invention comprises compounds of formula (I) according to any one of embodiments i) to viii), wherein R¹ represents 3-chloro-phenyl.
xxv) A further embodiment of the invention comprises compounds of formula (I) according to any one of embodiments i) to viii), wherein R¹ represents 3-bromo-phenyl.
xxvi) A further embodiment of the invention comprises compounds of formula (I) according to any one of embodiments i) to viii), wherein R¹ represents 3-methyl-phenyl.
xxvii) A further embodiment of the invention comprises compounds of formula (1) according to any one of embodiments i) to viii), wherein
   R¹ represents heterocyclyl, wherein the heterocyclyl is selected from the group consisting of indolyl, benzofuranyl, indazolyl, benzisoxazolyl, quinolinyl and (especially) imidazo[2,1-b]thiazolyl, wherein said heterocyclyl is unsubstituted or mono-substituted wherein the substituent is selected from (C₁₋₄)alkyl;
   or R¹ represents a 2,3-dihydro-benzofuranyl-, or a 2,3-dihydro-benzo[1,4]dioxinyl-group, which groups are unsubstituted.
xxviii) A further embodiment of the invention comprises compounds of formula (I) according to any one of embodiments i) to viii), wherein
   R¹ represents heterocyclyl, wherein the heterocyclyl is selected from the group consisting of indolyl, benzofuranyl, indazolyl, benzisoxazolyl, quinolinyl and (especially) imidazo[2,1-b]thiazolyl, wherein said heterocyclyl is unsubstituted or mono-substituted wherein the substituent is selected from (C₁₋₄)alkyl.
   xxix) A further embodiment of the invention comprises compounds of formula (I) according to any one of embodiments i) to viii), wherein
   R¹ represents a 2,3-dihydro-benzofuranyl-, or a 2,3-dihydro-benzo[1,4]dioxinyl-group, which groups are unsubstituted.
xxx) A further embodiment of the invention comprises compounds of formula (I) according to any one of embodiments i) to iii), wherein at least one, preferably all of the following characteristics are present:
   Y represents (CH₂)ₙ, wherein n represents 0 or 1;
   B represents phenyl, wherein the phenyl ring is mono-substituted with (C₁₋₄)alkyl (such as especially methyl), trifluoromethyl, or halogen (such as especially fluoro or chloro); and
   R¹ represents phenyl which is mono-substituted with (C₁₋₄)alkyl (such as especially methyl) or halogen (such as especially chloro or bromo); or unsubstituted naphthyl (such as especially 1-naphthyl); or R¹ is selected from the following groups:

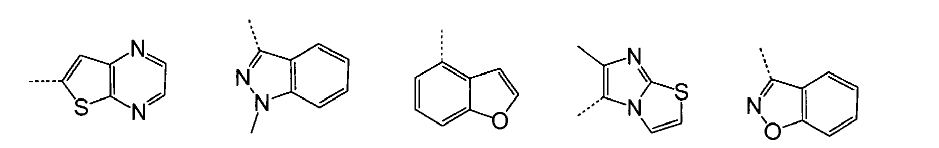

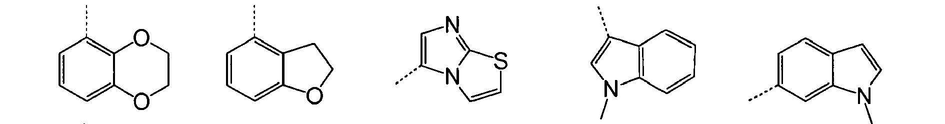

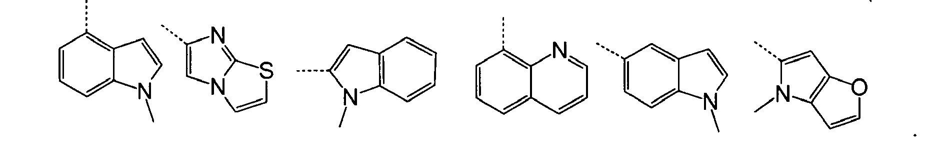

   The compounds of formula (I) may contain one or more stereogenic or asymmetric centers, such as one or more asymmetric carbon atoms. The compounds of formula (I) may thus be present as mixtures of stereoisomers or preferably as pure stereoisomers. Mixtures of stereoisomers may be separated in a manner known to a person skilled in the art.
xxxi) Examples of compounds of formula (I) according to embodiment i) are selected from the group consisting of:
   (S)-4-Methyl-4H-furo[3,2-b]pyrrole-5-carboxylic acid {1-[2-cyclopropyl-5-(2-fluorophenyl)-thiazole-4-carbonyl]-piperidin-2-ylmethyl}-amide;
   (S)-Thieno[2,3-b]pyrazine-6-carboxylic acid {1-[2-cyclopropyl-5-(2-fluoro-phenyl)-thiazole-4-carbonyl]-piperidin-2-ylmethyl}-amide;
   (S)-3-Bromo-N- {1-[2-cyclopropyl-5-(4-fluoro-phenyl)-thiazole-4-carbonyl]-piperidin-2-ylmethyl}-benzamide;
   (S)-Naphthalene-1-carboxylic acid {1-[2-cyclopropyl-5-(4-fluoro-phenyl)-thiazole-4-carbonyl]-piperidin-2-ylmethyl}-amide;
   (S)-Quinoline-8-carboxylic acid {1-[2-cyclopropyl-5-(4-fluoro-phenyl)-thiazole-4-carbonyl]-piperidin-2-ylmethyl}-amide;
   (S)-2,3-Dihydro-benzo[1,4]dioxine-5-carboxylic acid {1-[2-cyclopropyl-5-(4-fluorophenyl)-thiazole-4-carbonyl]-piperidin-2-ylmethyl}-amide;
   (S)-Benzofuran-4-carboxylic acid {1-[2-cyclopropyl-5-(4-fluoro-phenyl)-thiazole-4-carbonyl]-piperidin-2-ylmethyl}-amide;
   (S)-4-Methyl-4H-furo[3,2-b]pyrrole-5-carboxylic acid {1-[2-cyclopropyl-5-(4-fluorophenyl)-thiazole-4-carbonyl]-piperidin-2-ylmethyl}-amide;
   (S)-Imidazo[2,1-b]thiazole-5-carboxylic acid [1-(2-cyclopropyl-5-p-tolyl-thiazole-4-carbonyl)-piperidin-2-ylmethyl]-amide;
   (S)-Imidazo[2,1-b]thiazole-6-carboxylic acid [1-(2-cyclopropyl-5-p-tolyl-thiazole-4-carbonyl)-piperidin-2-ylmethyl]-amide;
   (S)-1-Methyl-1H-indole-2-carboxylic acid [1-(2-cyclopropyl-5-p-tolyl-thiazole-4-carbonyl)-piperidin-2-ylmethyl]-amide;
   (S)-Naphthalene-1-carboxylic acid [1-(2-cyclopropyl-5-m-tolyl-thiazole-4-carbonyl)-piperidin-2-ylmethyl]-amide;
   (S)-Quinoline-8-carboxylic acid [1-(2-cyclopropyl-5-m-tolyl-thiazole-4-carbonyl)-piperidin-2-ylmethyl]-amide;
   (S)-2,3-Dihydro-benzo[1,4]dioxine-5-carboxylic acid [1-(2-cyclopropyl-5-m-tolyl-thiazole-4-carbonyl)-piperidin-2-ylmethyl]-amide;
   (S)-Benzofuran-4-carboxylic acid [1-(2-cyclopropyl-5-m-tolyl-thiazole-4-carbonyl)-piperidin-2-ylmethyl]-amide;
   (S)-1-Methyl-1H-indazole-3-carboxylic acid [1-(2-cyclopropyl-5-m-tolyl-thiazole-4-carbonyl)-piperidin-2-ylmethyl]-amide;
   (S)-6-Methyl-imidazo[2,1-b]thiazole-5-carboxylic acid [1-(2-cyclopropyl-5-m-tolyl-thiazole-4-carbonyl)-piperidin-2-ylmethyl]-amide;
   (S)-Imidazo[2,1-b]thiazole-5-carboxylic acid [1-(2-cyclopropyl-5-m-tolyl-thiazole-4-carbonyl)-piperidin-2-ylmethyl]-amide;
   (S)-1-Methyl-1H-indole-4-carboxylic acid [1-(2-cyclopropyl-5-m-tolyl-thiazole-4-carbonyl)-piperidin-2-ylmethyl]-amide;
   (S)-3-Chloro-N-{1-[5-(3-chloro-phenyl)-2-cyclopropyl-thiazole-4-carbonyl]-piperidin-2-ylmethyl}-benzamide:
   (S)-Quinoline-8-carboxylic acid {1-[5-(3-chloro-phenyl)-2-cyclopropyl-thiazole-4-carbonyl]-piperidin-2-ylmethyl}-amide;
   (S)-Benzofuran-4-carboxylic acid {1-[5-(3-chloro-phenyl)-2-cyclopropyl-thiazole-4-carbonyl]-piperidin-2-ylmethyl}-amide;
   (S)-1-Methyl-1H-indole-3-carboxylic acid {1-[5-(3-chloro-phenyl)-2-cyclopropyl-thiazole-4-carbonyl]-piperidin-2-ylmethyl}-amide;
   (S)-Imidazo[2,1-b]thiazole-5-carboxylic acid {1-[5-(3-chloro-phenyl)-2-cyclopropyl-thiazole-4-carbonyl]-piperidin-2-ylmethyl }-amide;
   (S)-4-Methyl-4H-furo[3,2-b]pyrrole-5-carboxylic acid {1-[5-(3-chloro-phenyl)-2-cyclopropyl-thiazole-4-carbonyl]-piperidin-2-ylmethyl}-amide;
   (S)-1-Methyl-1H-indole-4-carboxylic acid {1-[5-(3-chloro-phenyl)-2-cyclopropyl-thiazole-4-carbonyl]-piperidin-2-ylmethyl}-amide;
   (S)-Benzo[d]isoxazole-3-carboxylic acid {1-[5-(3-chloro-phenyl)-2-cyclopropyl-thiazole-4-carbonyl]-piperidin-2-ylmethyl}-amide;
   (S)-N-{1-[2-Cyclopropyl-5-(3-trifluoromethyl-phenyl)-thiazole-4-carbonyl]-piperidin-2-ylmethyl}-3-methyl-benzamide;
   (S)-Quinoline-8-carboxylic acid {1-[2-cyclopropyl-5-(3-trifluoromethyl-phenyl)-thiazole-4-carbonyl]-piperidin-2-ylmethyl}-amide;
   (S)-2,3-Dihydro-benzo[1,4]dioxine-5-carboxylic acid {1-[2-cyclopropyl-5-(3-trifluoromethyl-phenyl)-thiazole-4-carbonyl]-piperidin-2-ylmethyl -amide;
   (S)-Benzofuran-4-carboxylic acid {1-[2-cyclopropyl-5-(3-trifluoromethyl-phenyl)-thiazole-4-carbonyl]-piperidin-2-ylmethyl}-amide;
   (S)-Imidazo[2, 1-b]thiazole-5-carboxylic acid {1-[2-cyclopropyl-5-(3-trifluoromethylphenyl)-thiazole-4-carbonyl]-piperidin-2-ylmethyl}-amide;
   (S)-3-Bromo-N-{1-[2-cyclopropyl-5-(4-fluoro-phenyl)-thiazole-4-carbonyl]-pyrrolidin-2-ylmethyl}-benzamide;
   (S)-Quinoline-8-carboxylic acid {1-[2-cyclopropyl-5-(4-fluoro-phenyl)-thiazole-4-carbonyl]-pyrrolidin-2-ylmethyl}-amide;
   (S)-2,3-Dihydro-benzo[1,4]dioxine-5-carboxylic acid {1-[2-cyclopropyl-5-(4-fluorophenyl)-thiazole-4-carbonyl]-pyrrolidin-2-ylmethyl}-amide;
   (S)-Benzofuran-4-carboxylic acid {1-[2-cyclopropyl-5-(4-fluoro-phenyl)-thiazole-4-carbonyl]-pyrrolidin-2-ylmethyl}-amide;
   (S)-1-Methyl-1H-indole-3-carboxylic acid {1-[2-cyclopropyl-5-(4-fluoro-phenyl)-thiazole-4-carbonyl]-pyrrolidin-2-ylmethyl}-amide;
   (S)-1-Methyl-1H-indazole-3-carboxylic acid {1-[2-cyclopropyl-5-(4-fluoro-phenyl)-thiazole-4-carbonyl]-pyrrolidin-2-ylmethyl}-amide; and
   (S)-Benzo[d]isoxazole-3-carboxylic acid {1-[2-cyclopropyl-5-(4-fluoro-phenyl)-thiazole-4-carbonyl]-pyrrolidin-2-ylmethyl}-amide.
xxxii) In addition to the compounds listed in embodiment xxxi), further examples of compounds of formula (I) according to embodiment i) are selected from the group consisting of:
   (S)-N-{1-[2-Cyclopropyl-5-(2-fluoro-phenyl)-thiazole-4-carbonyl]-piperidin-2-ylmethyl}-3-methyl-benzamide;
   (S)-3-Chloro-N-{1-[2-cyclopropyl-5-(2-fluoro-phenyl)-thiazole-4-carbonyl]-piperidin-2-ylmethyl}-benzamide;
   (S)-3-Bromo-N-{1-[2-cyclopropyl-5-(2-fluoro-phenyl)-thiazole-4-carbonyl]-piperidin-2-ylmethyl}-benzamide;
   (S)-Naphthalene-1-carboxylic acid {1-[2-cyclopropyl-5-(2-fluoro-phenyl)-thiazole-4-carbonyl]-piperidin-2-ylmethyl}-amide;
   (S)-2,3-Dihydro-benzo[1,4]dioxine-5-carboxylic acid {1-[2-cyclopropyl-5-(2-fluorophenyl)-thiazole-4-carbonyl]-piperidin-2-ylmethyl}-amide;
   (S)-1-Methyl-1H-indole-3-carboxylic acid {1-[2-cyclopropyl-5-(2-fluoro-phenyl)-thiazole-4-carbonyl]-piperidin-2-ylmethyl}-amide;
   (S)-1-Methyl-1H-indole-6-carboxylic acid {1-[2-cyclopropyl-5-(2-fluoro-phenyl)-thiazole-4-carbonyl]-piperidin-2-ylmethyl}-amide;
   (S)-1-Methyl-1H-indole-4-carboxylic acid {1-[2-cyclopropyl-5-(2-fluoro-phenyl)-thiazole-4-carbonyl]-piperidin-2-ylmethyl}-amide;
   (S)-N-{1-[2-Cyclopropyl-5-(4-fluoro-phenyl)-thiazole-4-carbonyl]-piperidin-2-ylmethyl}-3-methyl-benzamide;
   (S)-3-Chloro-N-{1-[2-cyclopropyl-5-(4-fluoro-phenyl)-thiazole-4-carbonyl]-piperidin-2-ylmethyl} -benzamide;
   (S)-1-Methyl-1H-indole-3-carboxylic acid {1-[2-cyclopropyl-5-(4-fluoro-phenyl)-thiazole-4-carbonyl]-piperidin-2-ylmethyl}-amide;
   (S)-1-Methyl-1H-indazole-3-carboxylic acid {1-[2-cyclopropyl-5-(4-fluoro-phenyl)-thiazole-4-carbonyl]-piperidin-2-ylmethyl}-amide;
   (S)-2,3-Dihydro-benzo[1,4]dioxine-5-carboxylic acid [1-(2-cyclopropyl-5-p-tolyl-thiazole-4-carbonyl)-piperidin-2-ylmethyl]-amide;
   (S)-N-[1-(2-Cyclopropyl-5-p-tolyl-thiazole-4-carbonyl)-piperidin-2-ylmethyl]-3-methyl-benzamide;
   (S)-3-Bromo-N-[1-(2-cyclopropyl-5-m-tolyl-thiazole-4-carbonyl)-piperidin-2-ylmethyl]-benzamide;
   (S)-4-Methyl-4H-furo[3,2-b]pyrrole-5-carboxylic acid [1-(2-cyclopropyl-5-m-tolyl-thiazole-4-carbonyl)-piperidin-2-ylmethyl]-amide;
   (S)-N-{1-[5-(3-Chloro-phenyl)-2-cyclopropyl-thiazole-4-carbonyl]-piperidin-2-ylmethyl}-3-methyl-benzamide;
   (S)-3-Bromo-N-{1-[5-(3-chloro-phenyl)-2-cyclopropyl-thiazole-4-carbonyl]-piperidin-2-ylmethyl} -benzamide;
   (S)-2,3-Dihydro-benzo[1,4]dioxine-5-carboxylic acid {1-[5-(3-chloro-phenyl)-2-cyclopropyl-thiazole-4-carbonyl]-piperidin-2-ylmethyl -amide;
   (S)-1-Methyl-1H-indazole-3-carboxylic acid {1-[5-(3-chloro-phenyl)-2-cyclopropyl-thiazole-4-carbonyl]-piperidin-2-ylmethyl}-amide;
   (S)-3-Chloro-N-{1-[2-cyclopropyl-5-(3-trifluoromethyl-phenyl)-thiazole-4-carbonyl]-piperidin-2-ylmethyl}-benzamide;
   (S)-3-Bromo-N-{1-[2-cyclopropyl-5-(3-trifluoromethyl-phenyl)-thiazole-4-carbonyl]-piperidin-2-ylmethyl} -benzamide;
   (S)-1-Methyl-1H-indole-3-carboxylic acid {1-[2-cyclopropyl-5-(3-trifluoromethylphenyl)-thiazole-4-carbonyl]-piperidin-2-ylmethyl}-amide;
   (S)-1-Methyl-1H-indazole-3-carboxylic acid {1-[2-cyclopropyl-5-(3-trifluoromethylphenyl)-thiazole-4-carbonyl]-piperidin-2-ylmethyl}-amide;
   (S)-6-Methyl-imidazo[2,1-b]thiazole-5-carboxylic acid {1-[2-cyclopropyl-5-(4-fluorophenyl)-thiazole-4-carbonyl]-pyrrolidin-2-ylmethyl}-amide; and
   (S)-Imidazo[2,1-b]thiazole-5-carboxylic acid {1-[2-cyclopropyl-5-(4-fluoro-phenyl)-thiazole-4-carbonyl]-pyrrolidin-2-ylmethyl}-amide.
xxxiii) In addition to the compounds listed in embodiments xxxi) to xxxii), further examples of compounds of formula (I) according to embodiment i) are selected from the group consisting of:
   (S)-Quinoline-8-carboxylic acid {1-[2-cyclopropyl-5-(2-fluoro-phenyl)-thiazole-4-carbonyl]-piperidin-2-ylmethyl}-amide;
   (S)-Benzofuran-4-carboxylic acid {1-[2-cyclopropyl-5-(2-fluoro-phenyl)-thiazole-4-carbonyl]-piperidin-2-ylmethyl}-amide;
   (S)-1-Methyl-1H-indazole-3-carboxylic acid {1-[2-cyclopropyl-5-(2-fluoro-phenyl)-thiazole-4-carbonyl]-piperidin-2-ylmethyl}-amide;
   (S)-6-Methyl-imidazo[2,1-b]thiazole-5-carboxylic acid {1-[2-cyclopropyl-5-(2-fluorophenyl)-thiazole-4-carbonyl]-piperidin-2-ylmethyl}-amide;
   (S)-Benzo[d]isoxazole-3-carboxylic acid {1-[2-cyclopropyl-5-(2-fluoro-phenyl)-thiazole-4-carbonyl]-piperidin-2-ylmethyl}-amide;
   (S)-6-Methyl-imidazo[2,1-b]thiazole-5-carboxylic acid {1-[2-cyclopropyl-5-(4-fluorophenyl)-thiazole-4-carbonyl]-piperidin-2-ylmethyl} -amide;
   (S)-6-Methyl-imidazo[2,1-b]thiazole-5-carboxylic acid [1-(2-cyclopropyl-5-p-tolyl-thiazole-4-carbonyl)-piperidin-2-ylmethyl]-amide;
   (S)-1-Methyl-1H-indazole-3-carboxylic acid [1-(2-cyclopropyl-5-p-tolyl-thiazole-4-carbonyl)-piperidin-2-ylmethyl]-amide;
   (S)-3-Bromo-N-[1-(2-cyclopropyl-5-p-tolyl-thiazole-4-carbonyl)-piperidin-2-ylmethyl]-benzamide;
   (S)-3-Chloro-N-[1-(2-cyclopropyl-5-p-tolyl-thiazole-4-carbonyl)-piperidin-2-ylmethyl]-benzamide
   (S)-N-[1-(2-Cyclopropyl-5-p-tolyl-thiazole-4-carbonyl)-piperidin-2-ylmethyl]-3-methyl-benzamide;
   (S)-3-Chloro-N-[1-(2-cyclopropyl-5-m-tolyl-thiazole-4-carbonyl)-piperidin-2-ylmethyl]-benzamide;
   (S)-1-Methyl-1H-indole-3-carboxylic acid [1-(2-cyclopropyl-5-m-tolyl-thiazole-4-carbonyl)-piperidin-2-ylmethyl]-amide;
   (S)-6-Methyl-imidazo[2,1 -b]thiazole-5-carboxylic acid {1-[5-(3-chloro-phenyl)-2-cyclopropyl-thiazole-4-carbonyl]-piperidin-2-ylmethyl}-amide; and
   (S)-6-Methyl-imidazo[2,1-b]thiazole-5-carboxylic acid {1-[2-cyclopropyl-5-(3-trifluoromethyl-phenyl)-thiazole-4-carbonyl]-piperidin-2-ylmethyl}-amide.
xxxiv) In addition to the compounds listed in embodiments xxxi) to xxxiii), further examples of compounds of formula (I) according to embodiment i) are selected from the group consisting of:
   (S)-Thieno[2,3-b]pyrazine-6-carboxylic acid {1-[2-cyclopropyl-5-(4-fluoro-phenyl)-thiazole-4-carbonyl]-piperidin-2-ylmethyl}-amide;
   (S)-1-Methyl-1H-indole-6-carboxylic acid {1-[2-cyclopropyl-5-(4-fluoro-phenyl)-thiazole-4-carbonyl]-piperidin-2-ylmethyl}-amid;
   (S)-1 -Methyl-1H-indole-4-carboxylic acid {1-[2-cyclopropyl-5-(4-fluoro-phenyl)-thiazole-4-carbonyl]-piperidin-2-ylmethyl}-amide;
   (S)-1-Methyl-1H-indole-5-carboxylic acid [1-(2-cyclopropyl-5-m-tolyl-thiazole-4-carbonyl)-piperidin-2-ylmethyl]-amide;
   (S)-N-{1-[5-(3-Chloro-phenyl)-2-cyclopropyl-thiazole-4-carbonyl]-piperidin-2-ylmethyl}-3-methyl-benzamide;
   (S)-Naphthalene-1-carboxylic acid {1-[5-(3-chloro-phenyl)-2-cyclopropyl-thiazole-4-carbonyl]-piperidin-2-ylmethyl}-amide;
   (S)-4-Methyl-4H-furo[3,2-b]pyrrole-5-carboxylic acid {1-[5-(3-chloro-phenyl)-2-cyclopropyl-thiazole-4-carbonyl]-piperidin-2-ylmethyl}-amide;
   (S)-1-Methyl-1H-indole-5-carboxylic acid {1-[5-(3-chloro-phenyl)-2-cyclopropyl-thiazole-4-carbonyl]-piperidin-2-ylmethyl}-amide;
   (S)-Naphthalene-1-carboxylic acid {1-[2-cyclopropyl-5-(3-trifluoromethyl-phenyl)-thiazole-4-carbonyl]-piperidin-2-ylmethyl}-amide;
   (S)-Benzo[d]isoxazole-3-carboxylic acid {1-[2-cyclopropyl-5-(3-trifluoromethylphenyl)-thiazole-4-carbonyl]-piperidin-2-ylmethyl}-amide;
   (S)-3-Chloro-N-{1-[2-cyclopropyl-5-(4-fluoro-phenyl)-thiazole-4-carbonyl]-pyrrolidin-2-ylmethyl}-benzamide;
   (S)-Naphthalene-1-carboxylic acid {1-[2-cyclopropyl-5-(4-fluoro-phenyl)-thiazole-4-carbonyl]-pyrrolidin-2-ylmethyl}-amide;
   (S)-Imidazo[2,1-b]thiazole-5-carboxylic acid {1-[2-cyclopropyl-5-(4-fluoro-phenyl)-thiazole-4-carbonyl]-pyrrolidin-2-ylmethyl}-amide;
   (S)-4-Methyl-4H-furo[3,2-b]pyrrole-5-carboxylic acid {1-[2-cyclopropyl-5-(4-fluorophenyl)-thiazole-4-carbonyl]-pyrrolidin-2-ylmethyl}-amide; and
   (S)-1-Methyl-1H-indole-4-carboxylic acid {1-[2-cyclopropyl-5-(4-fluoro-phenyl)-thiazole-4-carbonyl]-pyrrolidin-2-ylmethyl}-amide.
xxxv) In addition to the compounds listed in embodiments xxxi) to xxxiv), further examples of compounds of formula (I) according to embodiment i) are selected from the group consisting of: (S)-Imidazo[2,1-b]thiazole-5-carboxylic acid [1-(2-cyclopropyl-5-phenyl-thiazole-4-carbonyl)-piperidin-2-ylmethyl]-amide;
(S)-6-Methyl-imidazo[2,1-b]thiazole-5-carboxylic acid [1-(2-cyc\opropyl-5-phenyl-thiazole-4-carbonyl)-piperidin-2-ylmethyl]-amide;
(S)-2,3-Dihydro-benzofuran-4-carboxylic acid [1-(2-cyclopropyl-5-phenyl-thiazole-4-carbonyl)-piperidin-2-ylmethyl]-amide;
(S)-Benzofuran-4-carboxylic acid [1-(2-cyclopropyl-5-phenyl-thiazole-4-carbonyl)-piperidin-2-ylmethyl]-amide;
(S)-1-Methyl-1H-indazole-3-carboxylic acid [1-(2-cyclopropyl-5-phenyl-thiazole-4-carbonyl)-piperidin-2-ylmethyl]-amide;
(S)-Quinoline-8-carboxylic acid [1-(2-cyclopropyl-5-phenyl-thiazole-4-carbonyl)-piperidin-2-ylmethyl]-amide;
(S)-N-[1-(2-Cyclopropyl-5-phenyl-thiazole-4-carbonyl)-piperidin-2-ylmethyl]-3,4-dimethyl-benzamide;
(S)-N-[1-(2-Cyclopropyl-5-phenyl-thiazole-4-carbonyl)-piperidin-2-ylmethyl]-4-methyl-3-trifluoromethyl-benzamide;
(S)-3-Cyano-N-[1-(2-cyclopropyl-5-m-tolyl-thiazole-4-carbonyl)-piperidin-2-ylmethyl]-benzamide;
(S)-1-Methyl-1H-indole-3-carboxylic acid [1-(2-cyclopropyl-5-phenyl-thiazole-4-carbonyl)-piperidin-2-ylmethyl]-amide;
(S)-2,3-Dihydro-benzofuran-7-carboxylic acid [1-(2-cyclopropyl-5-m-tolyl-thiazole-4-carbonyl)-piperidin-2-ylmethyl]-amide;
(S)-2,3-Dihydro-benzofuran-4-carboxylic acid [1-(2-cyclopropyl-5-m-tolyl-thiazole-4-carbonyl)-piperidin-2-ylmethyl]-amide;
(S)-2-Ethyl-5-methyl-2H-pyrazole-3-carboxylic acid [1-(2-cyclopropyl-5-m-tolyl-thiazole-4-carbonyl)-piperidin-2-ylmethyl]-amide;
(S)-2,5-Dimethyl-2H-pyrazole-3-carboxylic acid [1-(2-cyclopropyl-5-m-tolyl-thiazole-4-carbonyl)-piperidin-2-ylmethyl]-amide;
(S)-3,4-Dihydro-2H-benzo[1,4]oxazine-5-carboxylic acid [1-(2-cyclopropyl-5-m-tolyl-thiazole-4-carbonyl)-piperidin-2-ylmethyl]-amide;
(S)-N-[1-(2-Cyclopropyl-5-m-tolyl-thiazole-4-carbonyl)-piperidin-2-ylmethyl]-3-methoxy-benzamide;
(S)-N-[1-(2-Cyclopropyl-5-m-tolyl-thiazole-4-carbonyl)-piperidin-2-ylmethyl]-3-trifluoromethyl-benzamide;
(S)-2,3-Dihydro-thieno[3,4-b][1,4]dioxine-5-carboxylic acid [1-(2-cyclopropyl-5-m-tolyl-thiazole-4-carbonyl)-piperidin-2-ylmethyl]-amide;
(S)-N-[1-(2-Cyclopropyl-5-m-tolyl-thiazole-4-carbonyl)-piperidin-2-ylmethyl]-3-fluoro-2-methyl-benzamide;
(S)-Benzo[d]isoxazole-3-carboxylic acid [1-(2-cyclopropyl-5-m-tolyl-thiazole-4-carbonyl)-piperidin-2-ylmethyl]-amide;
(S)-Benzo[d]isoxazole-3-carboxylic acid [1-(2-cyclopropyl-5-phenyl-thiazole-4-carbonyl)-piperidin-2-ylmethyl]-amide;
(S)-3,4-Dichloro-N-[1-(2-cyclopropyl-5-phenyl-thiazole-4-carbonyl)-piperidin-2-ylmethyl]-benzamide;
(S)-3-Chloro-N-[1-(2-cyclopropyl-5-m-tolyl-thiazole-4-carbonyl)-piperidin-2-ylmethyl]-2-methyl-benzamide;
(S)-2-Chloro-N-[1-(2-cyclopropyl-5-m-tolyl-thiazole-4-carbonyl)-piperidin-2-ylmethyl]-3-fluoro-benzamide;
(S)-N-[1-(2-Cyclopropyl-5-phenyl-thiazole-4-carbonyl)-piperidin-2-ylmethyl]-3-methoxy-benzamide;
(S)-Chroman-8-carboxylic acid [1-(2-cyclopropyl-5-m-tolyl-thiazole-4-carbonyl)-piperidin-2-ylmethyl]-amide;
(S)-1H-Indazole-3-carboxylic acid [1-(2-cyclopropyl-5-m-tolyl-thiazole-4-carbonyl)-piperidin-2-ylmethyl]-amide;
(S)-N-[1-(2-Cyclopropyl-5-phenyl-thiazole-4-carbonyl)-piperidin-2-ylmethyl]-3,5-dimethoxy-benzamide;
(S)-Isoquinoline-1-carboxylic acid [1-(2-cyclopropyl-5-m-tolyl-thiazole-4-carbonyl)-piperidin-2-ylmethyl]-amide;
(S)-2,3-Dihydro-benzo[1,4]dioxine-5-carboxylic acid [1-(2-cyclopropyl-5-phenyl-thiazole-4-carbonyl)-piperidin-2-ylmethyl]-amide;
(S)-N-[1-(2-Cyclopropyl-5-phenyl-thiazole-4-carbonyl)-piperidin-2-ylmethyl]-benzamide;
(S)-N-[1-(2-Cyclopropyl-5-phenyl-thiazole-4-carbonyl)-piperidin-2-ylmethyl]-2,5-dimethoxy-benzamide;
(S)-3-Methyl-isoxazole-5-carboxylic acid [1-(2-cyclopropyl-5-m-tolyl-thiazole-4-carbonyl)-piperidin-2-ylmethyl]-amide;
(S)-1,2-Dimethyl-1H-indole-3-carboxylic acid [1-(2-cyclopropyl-5-m-tolyl-thiazole-4-carbonyl)-piperidin-2-ylmethyl]-amide;
(S)-3-Acetylamino-N-[1-(2-cyclopropyl-5-m-tolyl-thiazole-4-carbonyl)-piperidin-2-ylmethyl]-benzamide;
(S)-4-Methyl-4H-furo[3,2-b]pyrrole-5-carboxylic acid [1-(2-cyclopropyl-5-phenyl-thiazole-4-carbonyl)-piperidin-2-ylmethyl]-amide;
(S)-3-Methyl-1H-indene-2-carboxylic acid [1-(2-cyclopropyl-5-m-tolyl-thiazole-4-carbonyl)-piperidin-2-ylmethyl]-amide;
(S)-1-Methyl-1H-indole-4-carboxylic acid [1-(2-cyclopropyl-5-phenyl-thiazole-4-carbonyl)-piperidin-2-ylmethyl]-amide;
(S)-Imidazo[1,2-a]pyridine-3-carboxylic acid [1-(2-cyclopropyl-5-m-tolyl-thiazole-4-carbonyl)-piperidin-2-ylmethyl]-amide;
(S)-Naphthalene-1-carboxylic acid [1-(2-cyclopropyl-5-phenyl-thiazole-4-carbonyl)-piperidin-2-ylmethyl]-amide;
(S)-5-Fluoro-1-methyl-1H-indole-2-carboxylic acid [1-(2-cyclopropyl-5-m-tolyl-thiazole-4-carbonyl)-piperidin-2-ylmethyl]-amide;
(S)-2,3-Dihydro-benzo[1,4]dioxine-6-carboxylic acid [1-(2-cyclopropyl-5-m-tolyl-thiazole-4-carbonyl)-piperidin-2-ylmethyl]-amide;
(S)-1-Methyl-1H-indole-5-carboxylic acid [1-(2-cyclopropyl-5-phenyl-thiazole-4-carbonyl)-piperidin-2-ylmethyl]-amide;
(S)-N-[1-(2-Cyclopropyl-5-m-tolyl-thiazole-4-carbonyl)-piperidin-2-ylmethyl]-4-methyl-nicotinamide;
(S)-2-Bromo-N-[1-(2-cyclopropyl-5-m-tolyl-thiazole-4-carbonyl)-piperidin-2-ylmethyl]-3-methyl-benzamide;
(S)-N-[1-(2-Cyclopropyl-5-phenyl-thiazole-4-carbonyl)-piperidin-2-ylmethyl]-3,4-dimethoxy-benzamide;
(S)-N-[1-(2-Cyclopropyl-5-m-tolyl-thiazole-4-carbonyl)-piperidin-2-ylmethyl]-2,3-dimethyl-benzamide;
(S)-Thieno[2,3-b]pyrazine-6-carboxylic acid {1-[5-(3-chloro-phenyl)-2-cyclopropyl-thiazole-4-carbonyl]-piperidin-2-ylmethyl}-amide;
(S)-1-Methyl-1H-indole-4-carboxylic acid {1-[2-cyclopropyl-5-(3-trifluoromethylphenyl)-thiazole-4-carbonyl]-piperidin-2-ylmethyl}-amide; and
(S)-2-Chloro-N-[1-(2-cyclopropyl-5-m-tolyl-thiazolc-4-carbonyl)-piperidin-2- . ylmethyl]-3-methyl-benzamide.

Any reference to a compound of formula (I) and/or (Ia) is to be understood as referring also to salts (especially pharmaceutically acceptable salts) of a compound of formula (I) and/or (Ia), respectively, as appropriate and expedient.

The term "pharmaceutically acceptable salts" refers to non-toxic, inorganic or organic acid and/or base addition salts. Reference can be made to "Salt selection for basic drugs", Int. J. Pharm. (1986), 33, 201-217.

Where the plural form is used for compounds, salts, pharmaceutical compositions, diseases and the like, this is intended to mean also a single compound, salt, or the like.

The compounds of formula (I) and/or (Ia) and their pharmaceutically acceptable salts can be used as medicaments, e.g. in the form of pharmaceutical compositions for enteral or parenteral administration.

The present invention also relates to the use of a compound of formula (I) for the preparation of a pharmaceutical composition for the prevention or treatment of the diseases and disorders mentioned herein.

The present invention further also relates to pharmaceutical compositions comprising a compound of formula (I), or a pharmaceutically acceptable salt thereof, and a pharmaceutically acceptable carrier.

The production of the pharmaceutical compositions can be effected in a manner which will be familiar to any person skilled in the art (see for example Remington, The Science and Practice of Pharmacy, 21st Edition (2005), Part 5, "Pharmaceutical Manufacturing" [published by Lippincott Williams & Wilkins]) by bringing the described compounds of formula (I) or their pharmaceutically acceptable salts, optionally in combination with other therapeutically valuable substances, into a galenical administration form together with suitable, non-toxic, inert, therapeutically compatible solid or liquid carrier materials and, if desired, usual pharmaceutical adjuvants.

The compounds according to formula (I) and/or (Ia) may be used for the preparation of a medicament and are suitable for the prevention and/or treatment of diseases or disorders selected from the group consisting of dysthymic disorders including major depression and cyclothymia, affective neurosis, all types of manic depressive disorders, delirium, psychotic disorders, schizophrenia, catatonic schizophrenia, delusional paranoia, adjustment disorders and all clusters of personality disorders; schizoaffective disorders; anxiety disorders including generalized anxiety, obsessive compulsive disorder, posttraumatic stress disorder, panic attacks, all types of phobic anxiety and avoidance; separation anxiety; all psychoactive substance use, abuse, seeking and reinstatement; all types of psychological or physical addictions, dissociative disorders including multiple personality syndromes and psychogenic amnesias; sexual and reproductive dysfunction; psychosexual dysfunction and addiction; tolerance to narcotics or withdrawal from narcotics; increased anaesthetic risk, anaesthetic responsiveness; hypothalamic-adrenal dysfunctions; disturbed biological and circadian rhythms; sleep disturbances associated with diseases such as neurological disorders including neuropathic pain and restless leg syndrome; sleep apnea; narcolepsy; chronic fatigue syndrome; insomnias related to psychiatric disorders; all types of idiopathic insomnias and parasomnias; sleep-wake schedule disorders including jet-lag; all dementias and cognitive dysfunctions in the healthy population and in psychiatric and neurological disorders; mental dysfunctions of aging; all types of amnesia; severe mental retardation; dyskinesias and muscular diseases; muscle spasticity, tremors, movement disorders; spontaneous and medication-induced dyskinesias; neurodegenerative disorders including Huntington's, Creutzfeld-Jacob's, Alzheimer's diseases and Tourette syndrome; Amyotrophic lateral sclerosis; Parkinson's disease; Cushing's syndrome; traumatic lesions; spinal cord trauma; head trauma; perinatal hypoxia; hearing loss; tinnitus; demyelinating diseases; spinal and cranial nerve diseases; ocular damage; retinopathy; epilepsy; seizure disorders; absence seizures, complex partial and generalized seizures; Lennox-Gastaut syndrome; migraine and headache; pain disorders; anaesthesia and analgesia; enhanced or exaggerated sensitivity to pain such as hyperalgesia, causalgia, and allodynia; acute pain; burn pain; atypical facial pain; neuropathic pain; back pain; complex regional pain syndrome I and II; arthritic pain; sports injury pain; dental pain; pain related to infection e.g. by HIV; post-chemotherapy pain; post-stroke pain; post-operative pain; neuralgia; osteoarthritis; conditions associated with visceral pain such as irritable bowel syndrome; eating disorders; diabetes; toxic and dysmetabolic disorders including cerebral anoxia, diabetic neuropathies and alcoholism; appetite, taste, eating, or drinking disorders; somatoform disorders including hypochondriasis; vomiting/nausea; emesis; gastric dyskinesia; gastric ulcers; Kallman's syndrome (anosmia); impaired glucose tolerance; intestinal motility dyskinesias; hypothalamic diseases; hypophysis diseases; hyperthermia syndromes, pyrexia, febrile seizures, idiopathic growth deficiency; dwarfism; gigantism; acromegaly; basophil adenoma; prolactinoma; hyperprolactinemia; brain tumors, adenomas; benign prostatic hypertrophy, prostate cancer; endometrial, breast, colon cancer; all types of testicular dysfunctions, fertility control; reproductive hormone abnormalities; hot flashes; hypothalamic hypogonadism, functional or psychogenic amenorrhea; urinary bladder incontinence asthma; allergies; all types of dermatitis, acne and cysts, sebaceous gland dysfunctions; cardiovascular disorders; heart and lung diseases, acute and congestive heart failure; hypotension; hypertension; dyslipidemias, hyperlipidemias, insulin resistance; urinary retention; osteoporosis; angina pectoris; myocardial infarction; arrhythmias, coronary diseases, left ventricular hypertrophy; ischemic or haemorrhagic stroke; all types of cerebrovascular disorders including subarachnoid haemorrhage, ischemic and hemorrhagic stroke and vascular dementia; chronic renal failure and other renal diseases; gout; kidney cancer; urinary incontinence; and other diseases related to general orexin system dysfunctions.

Compounds of formula (I) and/or (Ia) are particularly suitable for use in the treatment of diseases or disorders selected from the group consisting of all types of sleep disorders, of stress-related syndromes, of psychoactive substance use and abuse, of cognitive dysfunctions in the healthy population and in psychiatric and neurologic disorders, of eating or drinking disorders.

Eating disorders may be defined as comprising metabolic dysfunction; dysregulated appetite control; compulsive obesities; emeto-bulimia or anorexia nervosa. Pathologically modified food intake may result from disturbed appetite (attraction or aversion for food); altered energy balance (intake vs. expenditure); disturbed perception of food quality (high fat or carbohydrates, high palatability); disturbed food availability (unrestricted diet or deprivation) or disrupted water balance. Drinking disorders include polydipsias in psychiatric disorders and all other types of excessive fluid intake. Sleep disorders include all types of parasomnias, insomnias, narcolepsy and other disorders of excessive sleepiness, sleep-related dystonias; restless leg syndrome; sleep apneas; jet-lag syndrome; shift-work syndrome, delayed or advanced sleep phase syndrome or insomnias related to psychiatric disorders. Insomnias are defined as comprising sleep disorders associated with aging; intermittent treatment of chronic insomnia; situational transient insomnia (new environment, noise) or short-term insomnia due to stress; grief; pain or illness. Insomnia also include stress-related syndromes including post-traumatic stress disorders as well as other types and subtypes of anxiety disorders such as generalized anxiety, obsessive compulsive disorder, panic attacks and all types of phobic anxiety and avoidance; psychoactive substance use, abuse, seeking and reinstatement are defined as all types of psychological or physical addictions and their related tolerance and dependence components. Cognitive dysfunctions include deficits in all types of attention, learning and memory functions occurring transiently or chronically in the normal, healthy, young, adult or aging population, and also occurring transiently or chronically in psychiatric, neurologic, cardiovascular and immune disorders.

In a further preferred embodiment of the invention compounds of formula (I) and/or (Ia) are particularly suitable for use in the treatment of diseases or disorders selected from the group consisting of sleep disorders that comprises all types of insomnias, narcolepsy and other disorders of excessive sleepiness, sleep-related dystonias, restless leg syndrome, sleep apneas, jet-lag syndrome, shift-work syndrome, delayed or advanced sleep phase syndrome and insomnias related to psychiatric disorders.

In another preferred embodiment of the invention compounds of formula (I) and/or (Ia) are particularly suitable for use in the treatment of diseases or disorders selected from the group consisting of cognitive dysfunctions that comprise deficits in all types of attention, learning and memory functions occurring transiently or chronically in the normal, healthy, young, adult or aging population, and also occurring transiently or chronically in psychiatric, neurologic, cardiovascular and immune disorders.

In another preferred embodiment of the invention compounds of formula (I) and/or (Ia) are particularly suitable for use in the treatment of diseases or disorders selected from the group consisting of eating disorders that comprise metabolic dysfunction; dysregulated appetite control; compulsive obesities; emeto-bulimia or anorexia nervosa.

In another preferred embodiment of the invention compounds of formula (I) and/or (Ia) are particularly suitable for use in the treatment of diseases or disorders selected from the group consisting of psychoactive substance use and abuse that comprise all types of psychological or physical addictions and their related tolerance and dependence components.

The present invention also relates to a method for the prevention or treatment of a disease or disorder mentioned herein comprising administering to a subject a pharmaceutically active amount of a compound of formula (I).

Unless used regarding temperatures, the term "about" placed before a numerical value "X" refers in the current application to an interval extending from X minus 10% of X to X plus 10% of X, and preferably to an interval extending from X minus 5% of X to X plus 5% of X. In the particular case of temperatures, the term "about" placed before a temperature "Y" refers in the current application to an interval extending from the temperature Y minus 10°C to Y plus 10 °C, and preferably to an interval extending from Y minus 5 °C to Y plus 5 °C.

A further aspect of the invention is a process for the preparation of compounds of formula (I). Compounds according to formula (I) of the present invention can be prepared according to the general sequence of reactions outlined in the schemes below wherein B, Y and R¹ are as defined in the description of formula (I). The compounds obtained may also be converted into pharmaceutically acceptable salts thereof in a manner known *per se.* In general, all chemical transformations can be performed according to well-known standard methodologies as described in the literature or as described in the procedures or in the experimental part below.

### Preparation of compounds of formula (I):

Pathway 1: The synthesis of the final compounds starts from N-Boc-protected-2-aminomethylazacycloalkane derivatives **1** (commercially available) which are coupled with carboxylic acid derivatives **2** (either commercially available or prepared as described in I-Chemistry, Section A.1.9.1 to A.1.9.4) under standard peptide coupling reaction conditions in the presence of an activating reagent (e.g. TBTU) and a base (e.g. DIPEA) to give the mono-amide intermediates **3.** Deprotection under standard conditions with a 4M solution of HCl in dioxane results in the amine intermediates **4** as hydrochloride salts. The bis-amide final compounds **6** are prepared by a second amide bond formation reaction under comparable conditions as described above by using the carboxylic acid derivatives **5** (prepared as described in Schemes 2 and 3 and in the experimental part, A.1.1 to A.1.5).

Pathway 2:The sequence can be inverted by starting from the template 7 (commercially available), Boc-protected at the exocyclic N-atom by first introducing the substituent at the endocyclic N-atom in a standard peptide bond forming reaction with the carboxylic acid derivatives **5** (prepared as described in Schemes 2 and 3 and in I-Chemistry, Section A.1.1 to A.1.5) to give compound **8** which after deprotection to **9** leads in a final amide bond forming step with carboxylic acid derivatives **2** (either commercially available or prepared as described in the experimental part, A.1.9.1 to A.1.9.4) to the final compounds **6.**

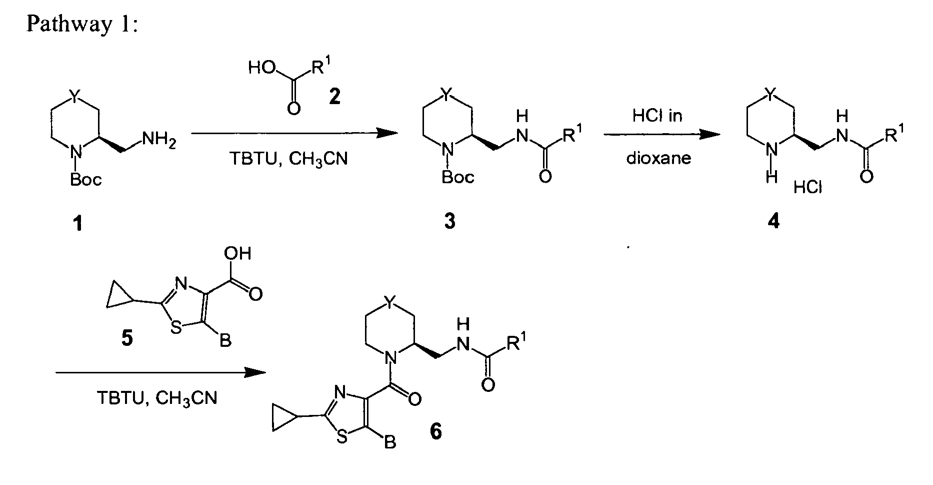

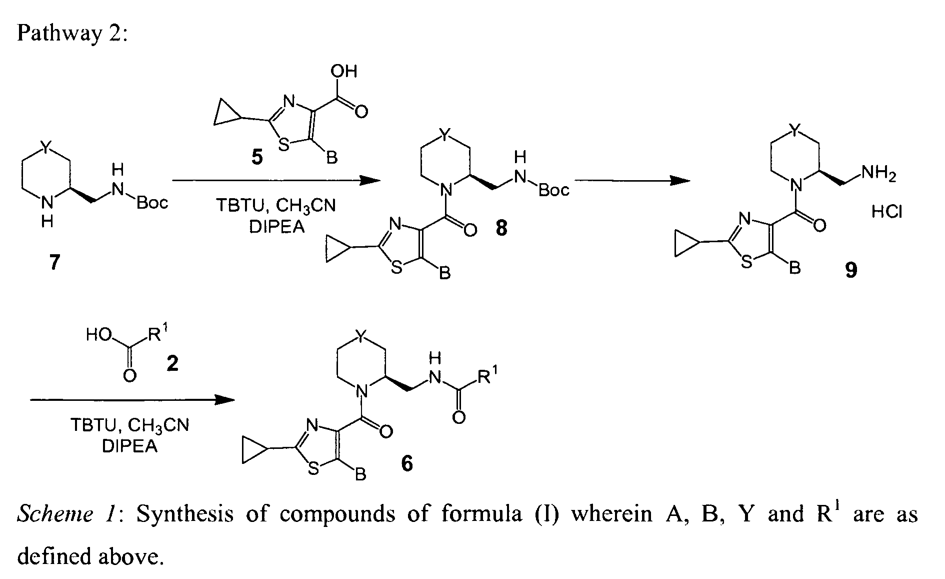

Carboxylic acid derivatives **16**, if not commercially available, are for example synthesized according to *scheme 2* and *scheme 3.*

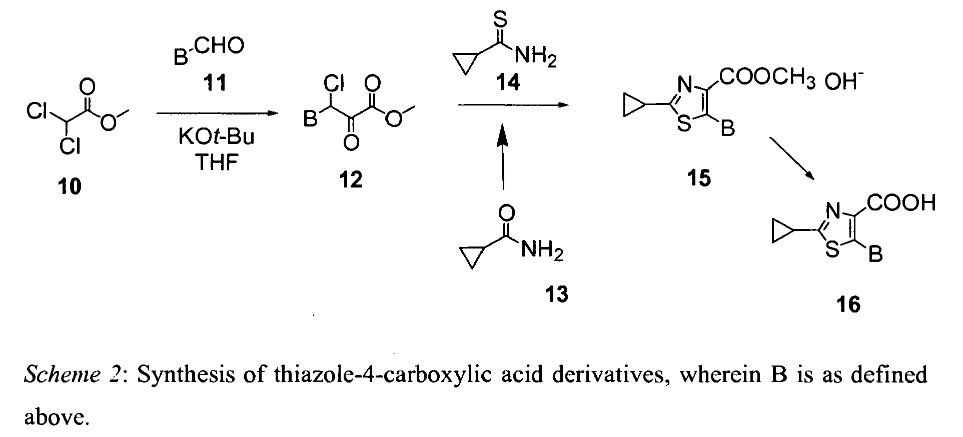

By reaction of methyl dichloroacetate **10** (commercially available) with an aldehyde **11** (commercially available) in the presence of a base like KOt-Bu the α-oxo-ester derivatives **12** are obtained which are transformed in a reaction with cyclopropyl-thiocarbamide **14** (prepared from commercially available cyclopropyl carboxamide **(13)** with *Lawesson's* reagent) to thiazole derivatives **15.** Hydrolysis of the ester function with an aq. solution of e.g. NaOH in a solvent like MeOH results in the formation of the desired carboxylic acids **16.**

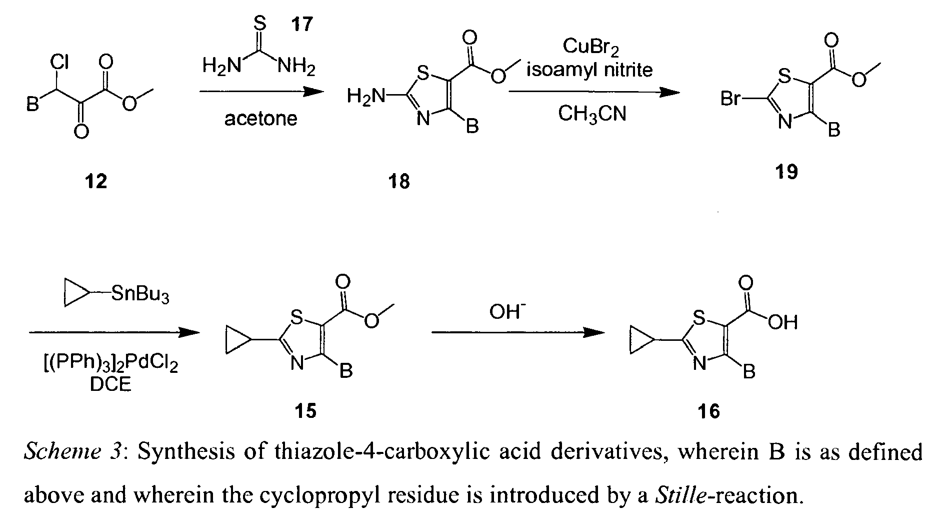

The synthetic sequence starts from the α-oxo-ester derivatives **12** (obtained as described in Scheme 2) by a *Hantzsch* cyclization step with thiourea **17** to obtain the 2-amino-thiazole intermediates **18** which are transformed into the 2-bromo-thiazole compounds **19** by a classical *Sandmeyer* reaction. The introduction of the cyclopropyl unit is achieved by the Pd-catalyzed *Stille*-coupling with tributyl-cyclopropyl-stannane (prepared from cyclopropyl magnesium bromide and tributyl tin chloride according to well established procedures) to give 2-cyclopropyl-thiazole-derivative **15** which is transformed into the carboxylic acid compound **16** according to the method described in Scheme 2.

Carboxylic acids of formula R¹-COOH are commercially available or well known in the field (Lit. e.g. WO2001/96302; T. Eicher, S. Hauptmann "The chemistry of Heterocycles: Structure, Reactions, Syntheses, and Applications", 2nd Edition 2003, Wiley, ISBN 978-3-527-30720-3).

Carboxylic acid derivatives R¹-COOH which represent an imidazo[2,1-b]thiazole-2-carboxylic acid derivative are commercially available or can be synthesised according to scheme 4.

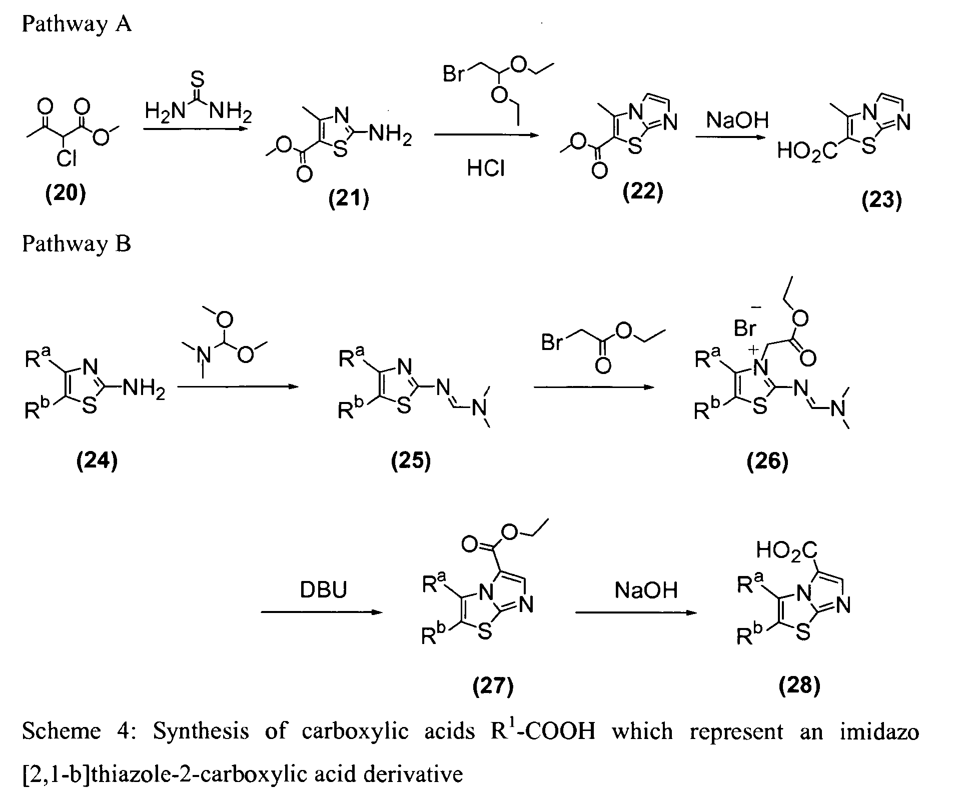

Pathway A: By reaction of 2-chloro-3-oxo-butyric acid methyl ester **(20)** with thiourea the amino-thiazole **(21)** can be obtained. Transformation to ester **(22)** can be accomplished with bromoacetaldehyde which can be generated *in-situ* from bromoacetaldehyde diethylacetal under acidic conditions. After saponification with bases such as NaOH the desired acid **(23)** can be obtained.

Pathway B: By heating a compound of structure **(24)** with *N,N*-dimethylformamide dimethylacetal in a solvent such as toluene formamidine derivatives **(25)** can be obtained. They can be alkylated with ethyl bromoacetate yielding the respective thiazolium bromide **(26)** which can be cyclised with strong bases such as DBU to the ester **(27).** Saponification of the ester function using methods known in the field such as treatment with a base such as NaOH in a solvent such as ethanol/water provides the corresponding imidazo[2,1-b]thiazole-2-carboxylic acid derivatives **(28).**

Carboxylic acid derivatives R¹-COOH which represent a pyrrolo[2,1-*b*]thiazole-7-carboxylic acid derivative can be synthesised according to scheme 5.

By reaction of 2-methylsulfanylthiazole **(29)** with trimethylsilylmethyl trifluoromethanesulfonate followed by cyclisation of the resulting thiazolinium salt by reaction with ethyl propiolate in the presence of caesium fluoride, the pyrrolo[2,1-*b*]thiazole **(30)** can be obtained. Saponification of the ester function using methods known in the field such as treatment with a base such as NaOH in a solvent such as EtOH/ water provides the corresponding pyrrolo[2,1-*b*]thiazole-7-carboxylic acid derivative **(31)** (Berry C.R. et al., Organic Letters, 2007, 9, 21, 4099-4102).

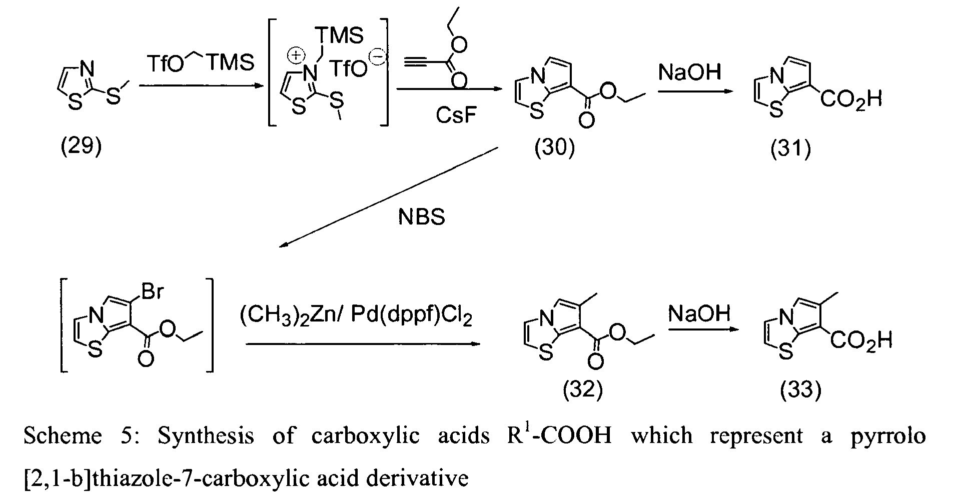

Bromination of **(30)** by reaction with NBS followed by methylation of the resulting crude ethyl 6-bromo-pyrrolo[2,1-*b*]thiazole-7-carboxylate by reaction with dimethylzinc in the presence of a palladium catalyst such as Pd(dppf)Cl₂ gave the ester **(32).** Saponification of the ester function using methods known in the field such as treatment with a base such as NaOH in a solvent such as EtOH/ water provides the corresponding 6-methyl-pyrrolo[2,1-*b*]thiazole-7-carboxylic acid derivative **(33).**

Carboxylic acid derivatives R¹-COOH which represent a 3,4-dihydro-2*H-*benzo[1,4]oxazinyl derivative can be synthesised according to the literature according to schemes 6 and 7.

Esterification of 3-hydroxy-anthranilic acid **(34)** with concentrated sulphuric acid in EtOH provides the corresponding ethyl ester **(35).** Cyclisation with acetyl chloride in presence of a base such as K₂CO₃ in a solvent such as DMF provides 3-oxo-3,4-dihydro-2*H*-benzo[1,4]oxazine derivatives **(36).** Compounds of structure **(36)** can optionally be alkylated with alkylating reagents such as methyl iodide in presence of a base such as K₂CO₃. Saponification with a base such as NaOH in a solvent such as EtOH/ water leads to the corresponding acids **(37)** or **(38).** Reduction of compounds of structure **(36)** with NaBH₄ in the presence of BF₃-diethyl etherate leads to the corresponding 3,4-dihydro-2*H*-benzo[1,4]oxazine derivative which can optionally be alkylated and/or saponified as described before to provide the corresponding acids **(40)** or **(41)** (Kuroita T. et al, Chemical Pharmaceutical Bulletin 1996,44,4,756-764).

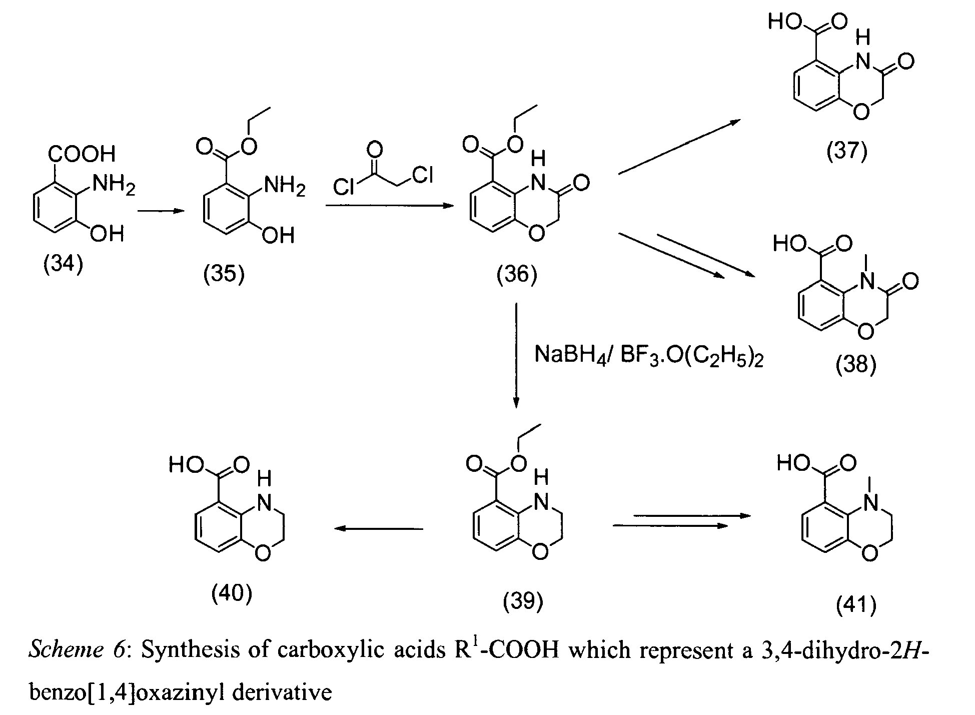

Hydrogenation of methyl 3-nitrosalicylate **(42)** in presence of a palladium catalyst provides the aniline derivative **(43)** which can be cyclized with chloroacetyl chloride as described before to the ester **(44).** Reduction of compounds of structure **(44)** with NaBH₄ in the presence of BF₃-diethyl etherate leads to the corresponding 3,4-dihydro-2*H*-benzo[1,4]oxazine derivative which can optionally be alkylated and/or saponified as described before to provide the corresponding acids **(45)** or **(46)** (Kuroita T. et al, Chemical Pharmaceutical Bulletin 1996, 44, 4, 756-764).

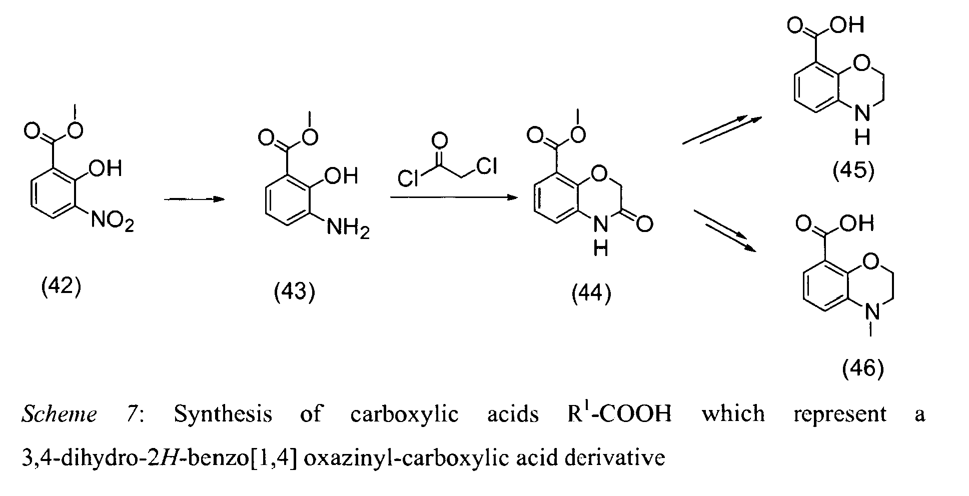

Carboxylic acid derivatives R¹-COOH which represent a benzooxazole-4-carboxylic acid derivative can be synthesised according to the literature according to schemes 8 and 9.

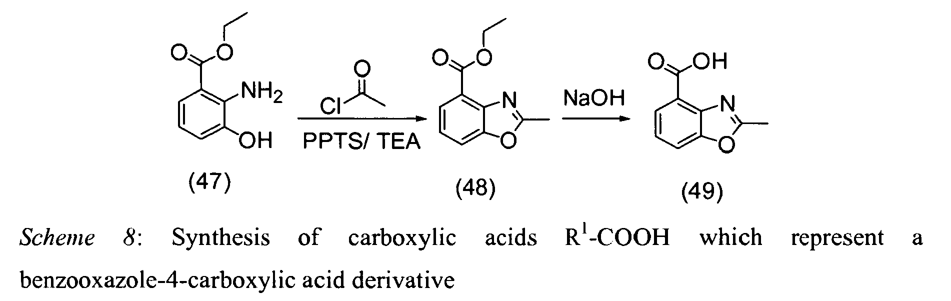

By cyclisation of ethyl 2-amino-3-hydroxybenzoate **(47)** with acetyl chloride in the presence of PPTS and TEA, the ester **(48)** can be obtained (Goldstein S.W. et al, Journal of Heterocyclic Chemistry, 1990, 27, 335-336). Saponification of the ester function using methods known in the art such as treatment with a base such as NaOH in a solvent such as EtOH / water provides the corresponding 2-methyl-benzooxazole-4-carboxylic acid derivative **(49).**

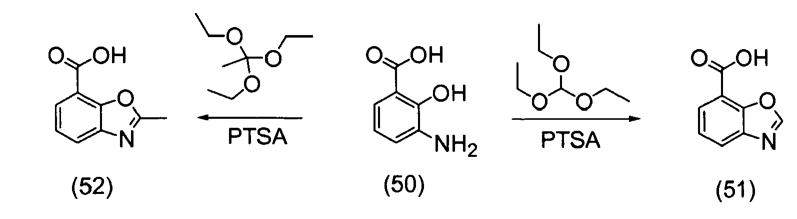

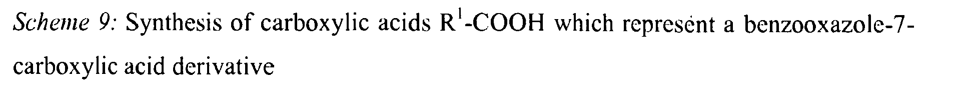

By cyclisation of 3-aminosalicylic acid **(50)** with triethyl orthoformate in the presence of PTSA, the benzooxazole-7-carboxylic acid **(51)** can be obtained (WO2006/069155). By cyclisation of 3-aminosalicylic acid **(50)** with triethyl orthoacetate in the presence of PTSA, the 2-methyl-benzooxazole-7-carboxylic acid **(52)** can be obtained (WO2006/069155)

Carboxylic acid derivatives R1-COOH which represent a benzothiazole-7-carboxylic acid derivative can be synthesised according to the literature according to scheme 10.

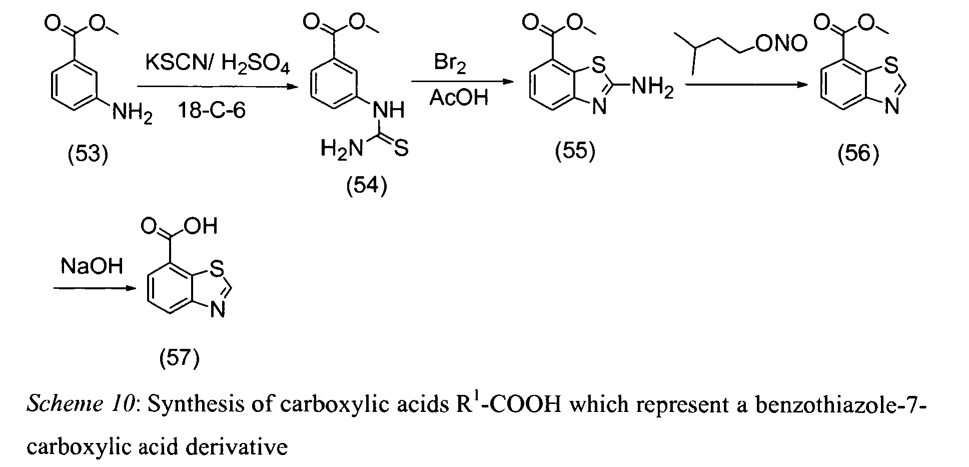

By reaction of methyl 3-aminobenzoate **(53)** with potassium thiocyanate in the presence of sulfuric acid and crown-ether 18-C-6, the thiourea **(54)** can be obtained. Cyclisation by reaction with bromine in acetic acid provides the 2-amino-benzothiazole derivative **(55).** Cleavage of the amino group by reaction with isoamyl nitrite furnishes the ester **(56)** (WO2005/092890). Saponification of the ester function using methods known in the field such as treatment with a base such as NaOH in a solvent such as MeOH/ water provides the corresponding benzothiazole-7-carboxylic acid derivative **(57).**

Carboxylic acid derivatives R¹-COOH which represent a benzofuran-4-carboxylic acid derivative can be synthesised according to the literature according to schemes 11 and 12.

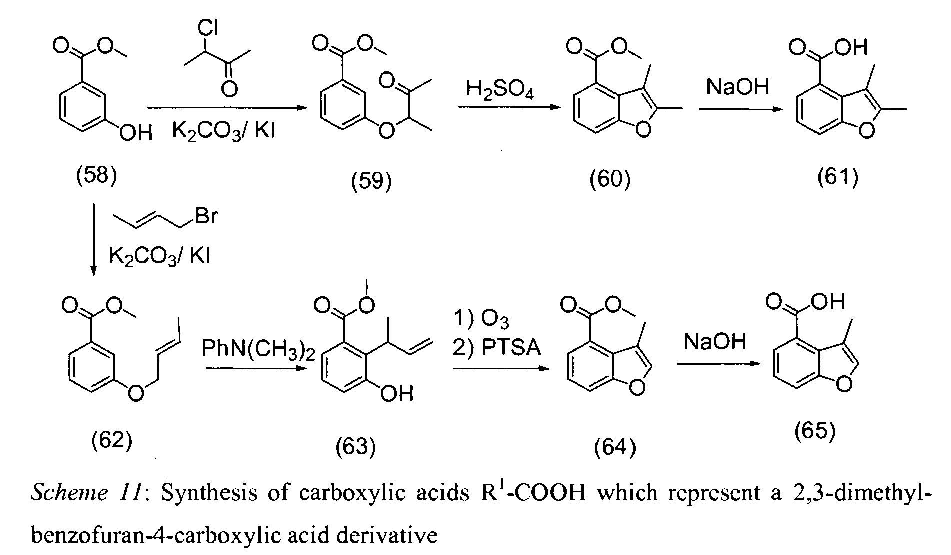

By reaction of methyl 3-hydroxybenzoate **(58)** with 3-chloro-2-butanone, the ester **(59)** can be obtained. Cyclisation with sulfuric acid provides the 2,3-dimethyl-benzofurane derivative **(60)** (Kawase Y. et al, Bulletin of the Chemical Sociaty of Japan, 1967, 40, 5, 1224-1231). Saponification of the ester function using methods known in the art such as treatment with a base such as NaOH in a solvent such as MeOH / water provides the corresponding 2,3-dimethyl-benzofuran-4-carboxylic acid derivative **(61).** On the other hand, reaction of methyl 3-hydroxybenzoate **(58)** with crotyl bromide furnishes the ester **(62)** which after reaction in N,N-dimethylaniline provides the ester **(63).** Ozonolysis followed by reaction with PTSA gives the 3-methyl-benzofurane derivative **(64)** (Mohamadi F. et al, Journal of Medicinal Chemistry, 1994, 37, 232-239 and EP58906). Saponification of the ester function using methods known in the art such as treatment with a base such as NaOH in a solvent such as MeOH/ water provides the corresponding 3-methylbenzofuran-4-carboxylic acid derivative **(65).**

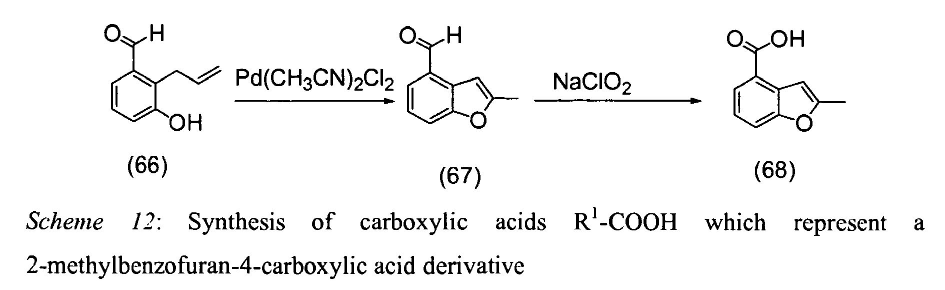

By cyclisation of 2-allyl-3-hydroxybenzaldehyde **(66)** with a palladium catalyst such as bis(acetonitrile)dichloropalladium in the presence of 1,4-benzoquinone and lithium chloride, the 2-methyl-benzofurane carbaldehyde **(67)** can be obtained (Danheiser R.L. et al, Organic Letters, 2005, 7, 18, 3905-3908). Oxidation of the aldehyde function with sodium chlorite in the presence of a scavenger such as 2-methyl-2-butene furnishes the corresponding 2-methylbenzofuran-4-carboxylic acid **(68).**

Carboxylic acid derivatives R¹-COOH which represent a benzofuran-4-carboxylic acid derivative and R represents Cl, F or CF₃ can be synthesised according to the literature or according to scheme 13.

By esterification of phenol derivative **(69)** with EtOH in the presence of an acid such as sulfuric acid followed by alkylation by reaction with allyl bromide in the presence of a K₂CO₃ and KI, the alkyl-ether derivative **(70)** can be obtained. Claisen rearrangement by reaction with N,N-dimethylaniline furnishes the phenol derivative **(71).** Ozonolysis followed by reaction with PTSA provides the benzofurane derivative **(72).** On the other hand ozonolysis of **(71)** in the presence of MeOH furnishes the dihydro-benzofurane derivative **(74).** Saponification of the ester function of **(72)** and **(74)** using methods known in the art such as treatment with a base such as NaOH in a solvent such as EtOH/water provide the corresponding benzofuran-4-carboxylic acid derivatives **(73)** and **(75).** Furthermore, cyclisation of **(71)** with a palladium catalyst such as bis(acetonitrile)dichloropalladium in the presence of 1,4-benzoquinone and lithium chloride, the 2-methylbenzofurane derivative **(76)** can be obtained (Danheiser R.L. et al, Organic Letters, 2005, 7, 18, 3905-3908). Saponification of the ester function of **(76)** using methods known in the art such as treatment with a base such as NaOH in a solvent such as EtOH / water provide the corresponding 2-methyl-benzofuran-4-carboxylic acid derivatives (77).

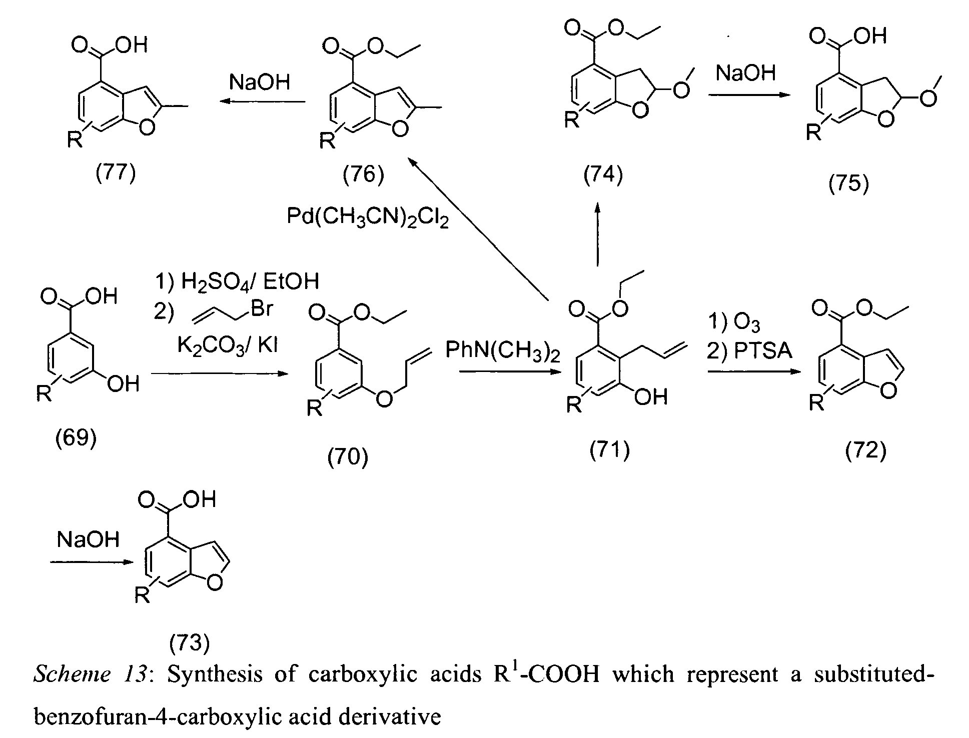

Carboxylic acid derivatives R¹-COOH which represent a 2-fluorobenzofuran-4-carboxylic acid derivative can be synthesised according to the literature according to scheme 14.

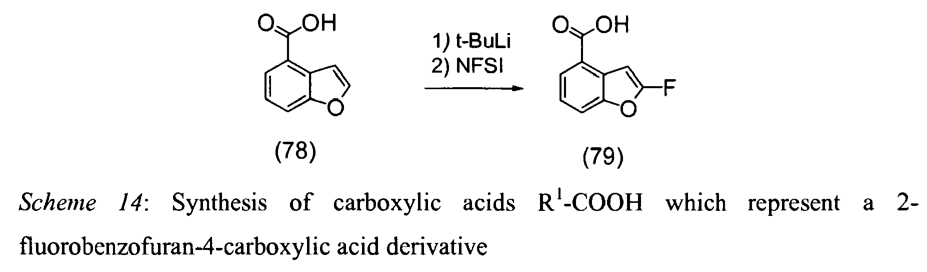

Specific electrophilic fluorination of benzofuran-4-carboxylic acid **(78)** (Eissenstat M.A. et al, Journal of Medicinal Chemistry 1995, 38, 16, 3094-3105) by reaction with tert-butyl lithium followed by reaction with NFSI (Torrado A. et al Bioorganic Medicinal Chemistry 2004, 12, 5277-5295 and Differling E. et al Synlett, 1991, 1, 187-189) provides the desired 2-fluorobenzofuran-4-carboxylic acid **(79).**

Derivatives of formula R¹-COOH wherein R¹ is chroman are for instance synthesised according to *scheme 15.*

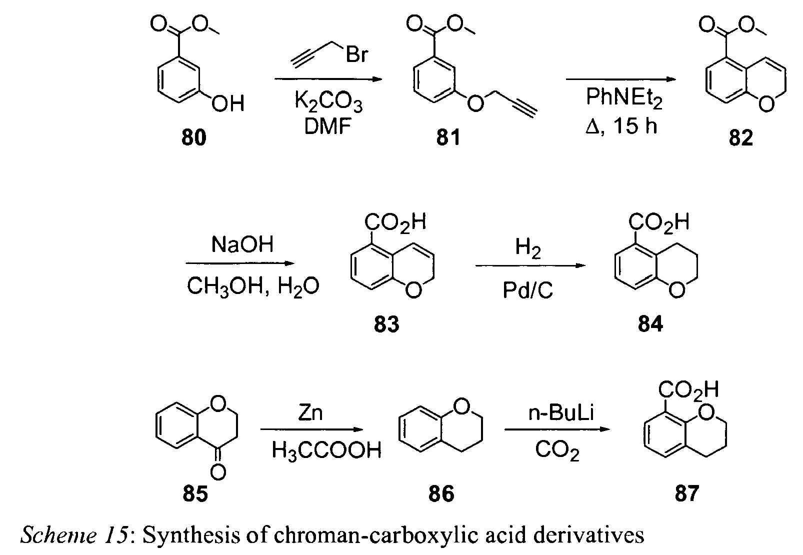

The synthesis of chroman-5-carboxylic acid derivatives starts with the alkylation of 3-hydroxy-benzoic acid methyl ester **(80;** commercially available) with propargyl bromide in the presence of K₂CO₃ to give phenylether **(81)** which is cyclised to the chromen derivative **(82)** by heating to reflux in N,N-diethylaniline. The carboxylic ester is saponified by treatment of **(82)** with NaOH in MeOH and water and the obtained chromen derivative **(83)** is hydrogenated to give the desired acid **(84).** The corresponding chroman-8-carboxylic acid derivatives are synthesized by reduction of 4-chromanone **(85;** commercially available) with zinc in acetic acid and subsequent *ortho-*metalation of the intermediate chroman derivative **(86)** with n-BuLi and trapping with carbon dioxide to give the desired acid **(87).**

Further alternative pathways for the synthesis of derivatives of formula R¹-COOH wherein R¹ is imidazo[2,1-b]thiazole are shown in *scheme 16.*

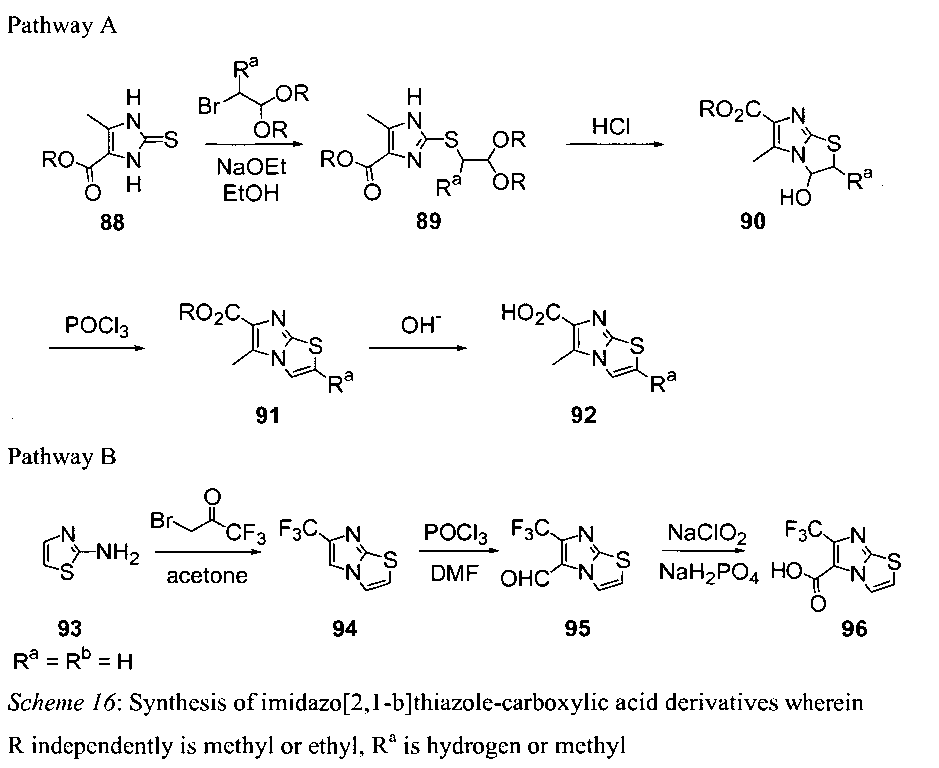

Following pathway A the imidazole derivative **(88)** may be transferred to the acetal **(89)** by alkylation with a bromoacetaldehyde dialkyl acetal derivative in the presence of a base like sodium ethoxide. Cyclization under acidic conditions (e.g. aq. hydrochloric acid) and dehydration of the intermediate **(90)** with for instance phosphorus oxychloride leads to ester **(91)** which is transformed to the desired acid **(92)** by saponification with for instance NaOH in solvents like THF and MeOH.

Pathway B starts with the alkylation of 2-amino-thiazole **(93)** with 3-bromo-1,1,1-trifluoroacetone to yield the trifluoromethyl-substituted imidazo[2,1-b]thiazole derivative **(94)** which is formylated to the aldehyde **(95)** by reaction with POCl₃ in a solvent like DMF. By oxidation of aldehyde **(95)** with sodium chlorite the desired imidazo[2,1-b]thiazole-carboxylic acid **(96)** is obtained. In analogy, the commercially available chlorinated aldehyde **(95,** being substituted with Cl instead of CF₃) may be oxidized to the corresponding acid.

Whenever the compounds of formula (I) are obtained in the form of mixtures of enantiomers, the enantiomers can be separated using methods known to one skilled in the art: e.g. by formation and separation of diastereomeric salts or by HPLC over a chiral stationary phase such as a Regis Whelk-O1(R,R) (10 µm) column, a Daicel ChiralCel OD-H (5-10 µm) column, or a Daicel ChiralPak IA (10 µm) or AD-H (5 µm) column. Typical conditions of chiral HPLC are an isocratic mixture of eluent A (EtOH, in presence or absence of an amine such as TEA, diethylamine) and eluent B (hexane), at a flow rate of 0.8 to 150 mL/min.

### Experimental Section

**Abbreviations** (as used herein and in the description before):
- AcOH: Acetic acid
- aq.: aqueous
- Boc: *tert*-Butoxycarbonyl
- BSA: Bovine serum albumine
- Bu: Butyl
- n-BuLi: n-Butyllithium
- conc.: concentrated
- d: Day(s)
- DBU: 1,8-Diazabicyclo[5.4.0]undec-7-ene
- DCE: 1,2-Dichloroethane
- DCM: Dichloromethane
- DIPEA: Diisopropylethylamine
- DMF: *N,N*-Dimethylformamide
- DMSO: Dimethylsulfoxide
- dppf: 1,1' -Bis(diphenylphosphanyl)ferrocene
- eq: Equivalent(s)
- Et: Ethyl
- EtOAc: Ethyl acetate
- EtOH: Ethanol
- FC: Flash column chromatography on silica gel
- FCS: Fetal calf serum
- FLIPR: Fluorescent imaging plate reader
- h: Hour(s)
- HBSS: Hank's balanced salt solution
- HEPES: 4-(2-Hydroxyethyl)-piperazine-1-ethanesulfonic acid
- HPLC: High performance liquid chromatography
- HV: High vacuum
- KOtBu: Potassium tert. butoxide
- LR: 2,4-bis(4-methoxyphenyl)-1,3,2,4-dithiadiphosphetane-2,4-disulfide (*Lawesson* reagent)
- LC: Liquid chromatography
- LC-MS: Liquid chromatography - Mass spectroscopy
- M: Molar(ity)
- Me: Methyl
- MeCN: Acetonitrile
- MeOH: Methanol
- min: Minute(s)
- MS: Mass spectroscopy
- NBS: N-Bromo-succinimide
- NFSI: N-Fluorobenzenesulfonimide
- Ph: Phenyl
- PPTS: Pyridinium 4-toluenesulfonate
- PTSA: para-Toluenesulfonic acid
- quant.: quantitative
- rt: room temperature
- sat.: saturated
- TBTU: *O*-Benzotriazol-1-yl-*N,N,N',N*'-tetramethyluronium tetrafluoroborate
- TEA: Triethylamine
- tert: Tertiary
- THF: Tetrahydrofuran
- Tf: Trifluoromethanesulfonyl
- TFA: Trifluoroacetic acid
- TMS: Trimethylsilyl
- t_{R}: Retention time

### 1-Chemistry

The following examples illustrate the preparation of biologically active compounds of the invention but do not at all limit the scope thereof.

All temperatures are stated in °C.

Compounds are characterized by:
- LC-MS:: Agilent 1100 series with DAD and MS detection (MS: Finnigan single quadrupole);
columns (4.6x50 mm, 5 µm): Zorbax SB-AQ, Zorbax Extend C 18 or Waters XBridge C 18;
conditions (if not otherwise stated the acidic gradient is used): acidic: eluent A: MeCN, eluent B: TFA in water (0.4 mL/L), 5% to 95% CH₃CN, flow rate 4.5 mL/min;
t_{R} is given in min.

Compounds are purified by FC, TLC or by preparative HPLC using RP-C₁₈ based columns with MeCN/water gradients and formic acid or ammonia additives.

### A. Preparation of precursors and intermediates

### A.1 Synthesis of thiazole-4-carboxylic acid derivatives

### A.1.1 Synthesis of 3-chloro-2-oxo-propionic ester derivatives

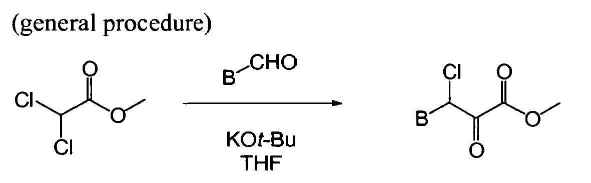

A solution of the respective aldehyde (338 mmol, 1.0 eq) and methyl dichloroacetate (338 mmol, 1.0 eq) in THF (100 mL) is added dropwise to a cold (-60°C) suspension of KOt-Bu (335 mmol, 1.0 eq) in THF (420 mL). After 4 h the mixture is allowed to reach rt, stirred over night and concentrated in vacuo. DCM and ice-cold water are added, the layers are separated and the aq. layer is extracted twice with DCM. The combined organic layers are washed with ice-cold water and brine, dried over MgSO₄ and concentrated in vacuo to give the desired α-oxo-ester which is used without further purification.
**3-Chloro-2-oxo-3-m-tolyl-propionic acid methyl ester**
   prepared by reaction of 3-methyl-benzaldehyde with methyl dichloroacetate.
**3-Chloro-2-oxo-3-p-tolyl-propionic acid methyl ester**
   prepared by reaction of 4-methyl-benzaldehyde with methyl dichloroacetate.
**3-Chloro-3-(4-fluoro-phenyl)-2-oxo-propionic acid methyl ester**
   prepared by reaction of 4-fluoro-benzaldehyde with methyl dichloroacetate.
**3-Chloro-3-(2-fluoro-phenyl)-2-oxo-propionic acid methyl ester**
   prepared by reaction of 2-fluoro-benzaldehyde with methyl dichloro-acetate.
**3-Chloro-3-(3-chloro-phenyl)-2-oxo-propionic acid methyl ester**
   prepared by reaction of 3-chloro-benzaldehyde with methyl dichloro-acetate.
**3-Chloro-2-oxo-3-(3-trifluoromethyl-phenyl)-propionic acid methyl ester**
   prepared by reaction of 3-trifluoromethyl-benzaldehyde with methyl dichloro-acetate.
**3-Chloro-3-(3-methoxy-phenyl)-2-oxo-propionic acid methyl ester**
   prepared by reaction of 3-methoxy-benzaldehyde with methyl dichloro-acetate.
**3-Chloro-3-(phenyl)-2-oxo-propionic acid methyl ester**
   prepared by reaction of benzaldehyde with methyl dichloro-acetate.

### A.1.2 Synthesis of cyclopropane thiaocarboxamide

Cyclopropanecarboxamide (115 mmol, 1 eq) was dissolved in THF (500 mL) followed by the addition of Na₂CO₃ (115 mmol, 1 eq) and LR (115 mmol, 1 eq). The reaction mixture was heated to 80°C for 2 h and concentrated under reduced pressure. The residue was taken up in diethylether (500 mL) and washed with water (500 mL) and brine (100 mL), dried over MgSO₄, filtered and concentrated in vacuo. The product was dried at HV to give cycloporpane thiocarboxamide in quant. yield. LC-MS: t_{R} = 0.36 min; [M+H+CH₃CN]⁺ = 143.18.

### A.1.3 Synthesis of 2-cyclopropyl-thiazole-4-carboxylic acid methyl ester derivatives (general procedure Hantzsch 1)

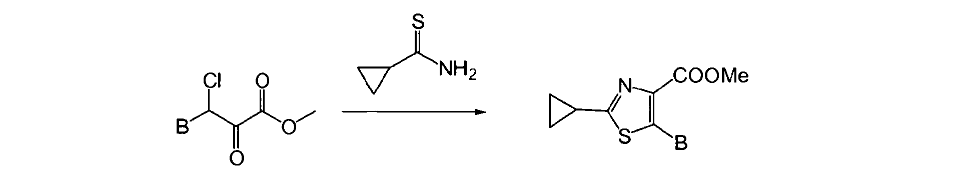

The respective α-oxo-ester (59 mmol, 1.0 eq) was dissolved in MeCN (90 mL) and sodium hydrogen carbonate was added (177 mmol, 3.0 eq) followed by the addition of a solution of cyclopropylthioacetamide (59 mmol, 1.0 eq) in MeCN (60 mL). Stirring was continued at rt for 3 h. The mixture was evaporated. The residue was taken up in EtOAc (200 mL) and washed with water (200 mL) and brine (200 mL). The organic layer was dried over magenisum sulphate, filtered and concentrated under reduced pressure. The residual oil was dissolved in MeOH (120 mL) followed by the addition of conc. sulphuric acid (5.9 mmol, 0.1 eq) and heated to 60°C overnight. The mixture was concentrated under reduced pressure and dried at HV to give the desired thiazole derivatives as white solids.
**2-Cyclopropyl-5-m-tolyl-thiazole-4-carboxylic acid methyl ester**
   prepared by reaction of 3-chloro-2-oxo-3-m-tolyl-propionic acid methyl ester with cyclopropylthioacetamide. LC-MS: t_{R} = 0.99 min; [M+H]⁺ = 274.27.
**2-Cyclopropyl-5-phenyl-thiazole-4-carboxylic acid methyl ester**
   prepared by reaction of 3-chloro-2-oxo-3-phenyl-propionic acid methyl ester with cyclopropylthioacetamide. LC-MS: t_{R} = 0.99 min; [M+H]⁺ = 260.45.

### A.1.4 Synthesis of 2-cyclopropyl-thiazole-4-carboxylic acid derivatives (general procedure)

The methyl ester derivative (73 mmol, 1 eq) was dissolved in a mixture of THF (165 mL) and iso-propanol (85 mL) followed by the addition of 1 M sodium hydroxide solution (145 mL, 2 eq). Stirring at rt was continued for 3 h. The reaction mixture was concentrated to 1/3 of the initial volume and acidified to pH = 1 by the addition of 1 M hydrochloric acid. The product was extracted with EtOAc (2 x 250 mL). The combined organic layers were washed with brine (100 mL), dried over MgSO₄ and concentrated under reduced pressure. The residue was dried at HV to give the carboxylic acid derivatives as white powders.
**2-Cyclopropylyl-5-m-tolyl-thiazole-4-carboxylic acid**
   prepared by saponification of 2-cyclopropyl-5-m-tolyl-thiazole-4-carboxylic acid methyl ester. LC-MS: t_{R} = 0.90 min; [M+H]⁺ = 260.23.
**2-Cyclopropylyl-5-phenyl-thiazole-4-carboxylic acid**
   prepared by saponification of 2-cyclopropyl-5-phenyl-thiazole-4-carboxylic acid methyl ester. LC-MS: t_{R} = 0.91 min; [M+H]⁺ = 246.39.

### A.1.5 Synthesis of 2-cyclopropyl-thiazole-4-carboxylic acid methyl ester derivatives via Stille pathway

### (general procedure)

### A.1.5.1 Synthesis of tributyl-cyclopropyl-stannane

Magnesium powder (0.26 mol, 1.05 eq) was activated with iodine followed by the addition of THF (100 mL). The reaction mixture was heated to 40°C and a solution of bromocyclopropane (0.248 mol, 1 eq) in THF (100 mL) was carefully added over a period of ca. 15 min. The reaction mixture was stirred for 90 min at 70°C, cooled to rt followed by the addition of a solution of tributylstannyl chloride (0.186 mol, 0.75 eq) in THF (150 mL) over ca. 30 min. The reaction mixture was refluxed for 16 h, cooled to 10°C and sat. NH₄Cl solution (150 mL) was carefully added followed by the addition of 1M KF solution (100 mL). The organic layer was separated, washed with 1M KF solution (50 mL) and brine (100 mL), dried over MgSO₄, filtered and concentrated under reduced pressure. The residual oil was distilled at HV to give tributyl-cyclopropyl-stannane as a colourless liquid.

### A.1.5.2 Synthesis of 2-amino-thiazole-4-carboxylic acid methyl ester derivatives (general procedure Hantzsch 2)

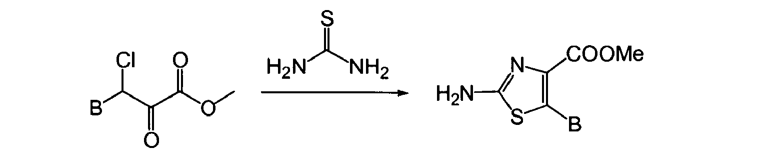

A solution of the respective α-oxo-ester (22.1 mmol, 1.0 eq) in acetone (25 mL) is added to a suspension of thiourea (22.1 mmol, 1.0 eq) in acetone (45 mL). The mixture is heated to 57°C (bath temperature), stirred for 24 h and concentrated to half of the volume. The obtained suspension is filtered and the residue is washed with acetone. After drying the desired amino-thiazole derivative is obtained as a solid.
**2-Amino-5-(4-fluoro-phenyl)-thiazole-4-carboxylic acid methyl ester**
   prepared by reaction of 3-chloro-2-oxo-(4-fluoro-phenyl)-propionic acid methyl ester with thiourea. LC-MS: t_{R} = 0.75 min; [M+H]⁺ = 253.17.
**2-Amino-5-(4-methyl-phenyl)-thiazole-4-carboxylic acid methyl ester**
   prepared by reaction of 3-chloro-3-(4-methyl-phenyl)-2-oxo-propionic acid methyl ester with thiourea. LC-MS: t_{R} = 0.77 min; [M+H]⁺ = 249.28.
**2-Amino-5-(3-methoxy-phenyl)-thiazole-4-carboxylic acid methyl ester**
   prepared by reaction of 3-chloro-3-(3-methoxy-phenyl)-2-oxo-propionic acid methyl ester with thiourea. LC-MS: t_{R} = 0.75 min; [M+H]⁺ = 265.25.
**2-Amino-5-(3-chloro-phenyl)-thiazole-4-carboxylic acid methyl ester**
   prepared by reaction of 3-chloro-3-(3-chloro-phenyl)-2-oxo-propionic acid methyl ester with thiourea. LC-MS: t_{R} = 0.82 min; [M+H]⁺ = 269.18.
**2-Amino-5-(2-fluoro-phenyl)-thiazole-4-carboxylic acid methyl ester**
   prepared by reaction of 3-chloro-3-(2-fluoro-phenyl)-2-oxo-propionic acid methyl ester with thiourea. LC-MS: t_{R} = 0.76 min; [M+H]⁺ = 253.20.
**2-Amino-5-(3-trifluoromethyl-phenyl)-thiazole-4-carboxylic acid methyl ester**
   prepared by reaction of 3-chloro-3-(3-trifluoromethyl-phenyl)-2-oxo-propionic acid methyl ester with thiourea. LC-MS: t_{R} = 0.86 min; [M+H]⁺ = 303.28.

### A.1.5.3 Synthesis of 2-bromo-thiazole-4-carboxylic acid methyl ester derivatives

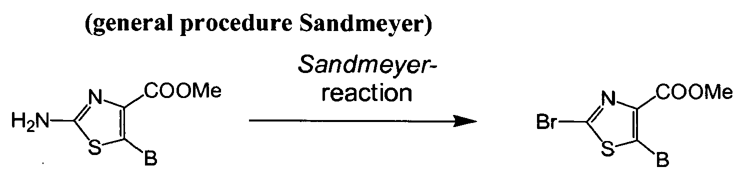

In an inert atmosphere, copper(II)bromide (47.3 mmol, 1.0 eq) was suspended in MeCN (200 mL) and cooled to 5-10°C followed by the addition of 3-methylbutylnitrite (71 mmol, 1.45 eq) over 15 min. To this reaction mixture the respective 2-aminothiazole derivative (47.3 mmol, 1 eq) was added in portions over 35 min at 5-10°C. The reaction mixture was then carefully heated to 65°C and stirring continued for 2 h. The volatiles were removed under reduced pressure and the black residue was purified by FC (heptane / EtOAc, as the appropriate mixture) to give the products as slightly yellow oils or solids.
**2-Bromo-5-(4-fluoro-phenyl)-thiazole-4-carboxylic acid methyl ester**
   LC-MS: t_{R} = 0.97 min; [M+H]⁺ = 316.09.
**2-Bromo-5-(4-methyl-phenyl)-thiazole-4-carboxylic acid methyl ester**
   LC-MS: t_{R} = 1.00 min; [M+H]⁺ = 314.25.
**2-Bromo-5-(3-methoxy-phenyl)-thiazole-4-carboxylic acid methyl ester**
   LC-MS: t_{R} = 0.97 min; [M+H]⁺ = 330.20.
**2-Bromo-5-(3-chloro-phenyl)-thiazole-4-carboxylic acid methyl ester**
   LC-MS: t_{R} = 1.00 min; [M+H]⁺ = 332.17.
**2-Bromo-5-(2-fluoro-phenyl)-thiazole-4-carboxylic acid methyl ester**
   LC-MS: t_{R} = 0.96 min; [M+H]⁺ = 316.11.
**2-Bromo-5-(3-trifluoromethyl-phenyl)-thiazole-4-carboxylic acid methyl ester**
   LC-MS: t_{R} = 1.03 min; [M+H]⁺ = 366.17.

### A.1.5.4 Synthesis of 2-cyclopropyl-thiazole-4-carboxylic acid methyl ester derivatives

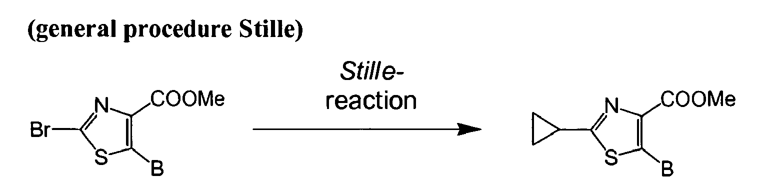

Cyclopropyl-tributyl-stannane (10.4 mmol, 1.1 eq) was dissolved in 1,2-dichloroethane followed by the addition of the respective 2-bromo-thiazole derivative (9.5 mmol, 1 eq). The mixture was degassed with Ar or N₂ for 5 min followed by the addition of bis(triphenylphosphine)palladium(II)dichloride (0.47 mmol, 0.05 eq). The reaction mixture was heated to 80°C and stirring continued for 24 h. The solvent was evaporated under reduced pressure and the residue was purified by FC (heptane : EtOAc = 2 : 1) to give the 2-cyclopropyl thiazole derivatives.
**2-Cyclopropyl-5-(4-fluoro-phenyl)-thiazole-4-carboxylic acid methyl ester**
   LC-MS: t_{R} = 0.97 min; [M+H]⁺ = 278.26.
**2-Cyclopropyl-5-(4-methyl-phenyl)-thiazole-4-carboxylic acid methyl ester**
   LC-MS: t_{R} = 0.99 min; [M+H]⁺ = 274.28.
**2-Cyclopropyl-5-(3-methoxy-phenyl)-thiazole-4-carboxylic acid methyl ester**
   LC-MS: t_{R} = 0.96 min; [M+H]⁺ = 290.30.
**2-Cyclopropyl-5-(3-chloro-phenyl)-thiazole-4-carboxylic acid methyl ester**
   LC-MS: t_{R} = 1.00 min; [M+H]⁺ = 294.23.
**2-Cyclopropyl-5-(2-fluoro-phenyl)-thiazole-4-carboxylic acid methyl ester**
   LC-MS: t_{R} = 0.95 min; [M+H]⁺ = 278.27.
**2-Cyclopropyl-5-(3-trifluoromethyl-phenyl)-thiazole-4-carboxylic acid methyl ester**
   LC-MS: t_{R} = 1.03 min; [M+H]⁺ = 328.24.

### A.1.5.5 Hydrolysis of the methyl esters to the carboxylic acids:

For experimental details: see A.1.4
**2-Cyclopropyl-5-(4-fluoro-phenyl)-thiazole-4-carboxylic acid**
   LC-MS: t_{R} = 0.87 min; [M+H]⁺ = 264.22.
**2-Cyclopropyl-5-(4-methyl-phenyl)-thiazole-4-carboxylic acid**
   LC-MS: t_{R} = 0.90 min; [M+H]⁺ = 260.24.
**2-Cyclopropyl-5-(3-methoxy-phenyl)-thiazole-4-carboxylic acid**
   LC-MS: t_{R} = 0.87 min; [M+H]⁺ = 276.27.
**2-Cyclopropyl-5-(3-chloro-phenyl)-thiazole-4-carboxylic acid**
   LC-MS: t_{R} = 0.91 min; [M+H]⁺ = 280.19.
**2-Cyclopropyl-5-(2-fluoro-phenyl)-thiazole-4-carboxylic acid**
   LC-MS: t_{R} = 0.86 min; [M+H]⁺ = 264.21.
**2-Cyclopropyl-5-(3-trifluoromethyl-phenyl)-thiazole-4-carboxylic acid**
   LC-MS: t_{R} = 0.94 min; [M+H]⁺ = 314.21.

### A.1.6 Synthesis of imidazo[2,1-b]thiazole-5-carboxylic acid derivatives:

### A.1.6.1 Synthesis of N,N-dimethyl-N-thiazol-2-yl-formamidine derivatives

### (general procedure)

To a solution of 2-aminothiazole derivative (4 g) in dry toluene (30 mL), was added dropwise *N,N*-dimethylformamide-dimethylacetal (2 eq). The reaction mixture was stirred at reflux under nitrogen for 20 h. After cooling to rt, the mixture was concentrated in vacuo and the residue was triturated with n-hexane to yield the formamidine intermediate as crystals.
***N*,*N*-Dimethyl-*N*-thiazol-2-yl-formamidine**
   Reaction with 2-aminothiazole
   LC-MS: t_{R} =0.56 min; [M+H]⁺ = 184.

### A.1.6.2 Synthesis of 2-(dimethylamino-methyleneamino)-3-ethoxycarbonylmethyl-thiazol-3-ium bromide derivatives

### (general procedure)

To a solution of *N*,*N*-dimethyl-*N*-thiazol-2-yl-formamidine derivative (5 g) in dry toluene (9 mL) was added dropwise ethyl bromoacetate (5 eq). The reaction mixture was stirred at rt under nitrogen for 20 h.

The resulting precipitate was filtered off and recrystallised over MeCN to give the desired thiazolinium bromide salt.
**2-(Dimethylamino-methyleneamino)-3-ethoxycarbonylmethyl-thiazol-3-ium bromide**
   Reaction with *N*,*N*-dimethyl-*N-*thiazol-2-yl-formamidine
   LC-MS: t_{R} = 0.58 min; [M+H]⁺ = 242.

### A.1.6.3 Synthesis of imidazo[2,1-b]thiazole-5-carboxylic ethyl ester derivatives

### (general procedure)

To a suspension of 2-(dimethylamino-methyleneamino)-3-ethoxycarbonylmethyl-thiazol-3-ium bromide derivative (10 g) in dry DMF (40 mL) was added DBU (1.6 eq). The resulting solution was stirred at rt under nitrogen for 20 h. The solution was poured into ice-water, the resulting precipitate was filtered, washed with cold water and dried in vacuo to give the desired imidazo[2,1*-b*]thiazole-5-carboxylic ethyl ester derivative as a solid.
**Imidazo[2,1-*b*]thiazole-5-carboxylic acid ethyl ester**
   Reaction with 2-(dimethylamino-methyleneamino)-3-ethoxycarbonylmethyl-thiazol-3-ium bromide
   LC-MS: t_{R} = 0.76 min; [M+H]⁺ = 197.

### A.1.6.4 Synthesis of imidazo[2,1-b]thiazole-5-carboxylic acid derivatives

### (general procedure)

To a solution of imidazo[2,1*-b*]thiazole-5-carboxylic ethyl ester derivative (1 g) in a mixture of THF/MeOH (3/1) (7.5 mL) was added dropwise NaOH IN (2 eq). The reaction mixture was stirred at rt for 20 h. The mixture was then concentrated in vacuo, the residue was diluted with water (2.5 mL) and cooled to 0°C. The pH was adjusted to 3-4 by addition of HCl 1N. The resulting white precipitate was filtered off and the solid was rinsed sparingly with cold water. After drying in vacuo, the desired imidazo[2,1-*b*]thiazole-carboxylic acid derivative was obtained as a white solid.
**Imidazo[2,1-b]thiazole-5-carboxylic acid**
   Reaction with imidazo[2,1*-b*]thiazole-5-carboxylic ethyl ester
   LC-MS: t_{R} = 0.39 min; [M+H]⁺ = 169.
**6-Methyl-imidazo[2,1-b]thiazole-5-carboxylic acid**
   is commercially available from Apollo Scientific as OR25897.

### B. Preparation of the Examples

### B.1.1 Example 1: (S)-N-{1-[2-Cyclopropyl-5-(2-fluoro-phenyl)-thiazole-4-carbonyl]-piperidin-2-ylmethyl}-3-methyl-benzamide

### STEP 1: (S)-{1-[2-Cyclopropyl-5-(2-fluoro-phenyl)-thiazole-4-carbonyl]-piperidin-2-ylmethyl}-carbamic acid tert-butyl ester

2-Cyclopropyl-5-(2-fluoro-phenyl)-thiazole-4-carboxylic acid (1.13 g, 4.29 mmol) was dissolved in MeCN (15 mL) followed by the addition of TBTU (1.5 g, 4.68 mmol) and DIPEA (1.7 mL, 9.75 mmol). The reaction mixture was stirred at rt for 30 min. (S)-Piperidin-2-ylmethyl-carbamic acid tert-butyl ester (836 mg, 3.9 mmol) dissolved in DCM (10 mL) was subsequently added to the reaction mixture. Stirring at rt was continued for 16 h. The reaction mixture was diluted with DCM and extracted with water. The organic layer was dried over MgSO₄, filtered and concentrated under reduced pressure to give 1.79 g (quant. yield) of (S)-{1-[2-cyclopropyl-5-(2-fluorophenyl)-thiazole-4-carbonyl]-piperidin-2-ylmethyl}-carbamic acid tert-butyl ester which was used in the next step without further purification. LC-MS: t_{R} = 1.06 min; [M+H]⁺ = 460.21.

### STEP 2: (S)-(2-Aminomethyl-piperidin-1-yl)-[2-cyclopropyl-5-(2-fluoro-phenyl)-thiazol-4-yl]-methanone hydrochloride

(S)**-**{1-[2-Cyclopropyl-5-(2-fluoro-phcnyl)-thiazole-4-carbonyl]-piperidin-2-ylmethyl}-carbamic acid tert-butyl ester (1.79 g, 3.9 mmol) was dissolved in dioxane (25 mL) followed by the addition of HCl in dioxane (4M, 25 mL). Stirring was continued at rt for 2 h. The reaction mixture was concentrated under reduced pressure and the residue dried at HV to give 1.4 g (quant. yield) of (S)-(2-aminomethyl-piperidin-1-yl)-[2-cyclopropyl-5-(2-fluoro-phenyl)-thiazol-4-yl]-methanone hydrochloride which was used in the next step without further purification. LC-MS: t_{R} = 0.76 min; [M+H]⁺ = 360. 1.

### STEP 3: (S)-N-{1-[2-Cyclopropyl-5-(2-fluoro-phenyl)-thiazole-4-carbonyl]-piperidin-2-ylmethyl}-3-methyl-benzamide (Example 1)

3-Methyl-benzoic acid (0.14 mmol) was dissolved in MeCN (1 mL) followed by the addition of TBTU (50 mg, 0.15 mmol) and DIPEA (0.65 mL, 0.38 mmol). The mixture was stirred at rt for 15 min. (S)-(2-Aminomethyl-piperidin-1-yl)-[2-cyclopropyl-5-(2-fluoro-phenyl)-thiazol-4-yl]-methanone hydrochloride (100 mg, 0.127 mmol) dissolved in DCM (1 mL) was added and stirring was continued for 16 h at rt. The reaction mixture was concentrated under reduced pressure, dissolved in MeCN and purified by preparative HPLC to give (S)-N-{1-[2-cyclopropyl-5-(2-fluoro-phenyl)-thiazole-4-carbonyl]-piperidin-2-ylmethyl}-3-methyl-benzamide (33.2 mg) as a white solid. LC-MS: t_{R} = 1.07 min; [M+H]⁺ = 478.19.

Examples 2 to 15 were prepared according to the procedures described above by using the appropriate carboxylic acid in STEP 3.

### B.1.2 Example 2: (S)-3-Chloro-N-{1-[2-cyclopropyl-5-(2-fluoro-phenyl)-thiazole-4-carbonyl]-piperidin-2-ylmethyl}-benzamide

LC-MS: t_{R} = 1.09 min; [M+H]⁺ = 498.14.

### B.1.3 Example 3: (S)-3-Bromo-N-{1-[2-cyclopropyl-5-(2-fluoro-phenyl)-thiazole-4-carbonyl]-piperidin-2-ylmethyl}-benzamide

LC-MS: t_{R} = 1.10 min; [M+H]⁺ = 542.06.

### B.1.4 Example 4: (S)-Naphthalene-1-carboxylic acid {1-[2-cyclopropyl-5-(2-fluorophenyl)-thiazole-4-carbonyl]-piperidin-2-ylmethyl}-amide

LC-MS: t_{R} = 1.11 min; [M+H]⁺ = 514.30.

### B.1.5 Example 5: (S)-Quinoline-8-carboxylic acid {1-[2-cyclopropyl-5-(2-fluorophenyl)-thiazole-4-carbonyl]-piperidin-2-ylmethyl}-amide

LC-MS: t_{R} = 0.97 min; [M+H]⁺ = 515.13.

### B.1.6 Example 6: (S)-2,3-Dihydro-benzo[1,4]dioxine-5-carboxylic acid {1-[2-cyclopropyl-5-(2-fluoro-phenyl)-thiazole-4-carbonyl]-piperidin-2-ylmethyl}-amide

LC-MS: t_{R} = 1.06 min; [M+H]⁺ = 522.11.

### B.1.7 Example 7: (S)-Benzofuran-4-carboxylic acid {1-[2-cyclopropyl-5-(2-fluorophenyl)-thiazole-4-carbonyl]-piperidin-2-ylmethyl}-amide

LC-MS: t_{R} = 1.07 min; [M+H]⁺ = 504.21.

### B.1.8 Example 8: (S)-1-Methyl-1H-indole-3-carboxylic acid {1-[2-cyclopropyl-5-(2-fluoro-phenyl)-thiazole-4-carbonyl]-piperidin-2-ylmethyl}-amide

LC-MS: t_{R} = 1.06 min; [M+H]⁺ = 517.20.

### B.1.9 Example 9: (S)-1-Methyl-1H-indazole-3-carboxylic acid {1-[2-cyclopropyl-5-(2-fluoro-phenyl)-thiazole-4-carbonyl]-piperidin-2-ylmethyl}-amide

LC-MS: t_{R} = 1.07 min; [M+H]⁺ = 518.17.

### B.1.10 Example 10: (S)-6-Methyl-imidazo[2,1-b]thiazole-5-carboxylic acid {1-[2-cyclopropyl-5-(2-fluoro-phenyl)-thiazole-4-carbonyl]-piperidin-2-ylmethyl}-amide

LC-MS: t_{R} = 0.92 min; [M+H]⁺ = 524.11.

### B.1.11 Example 11: (S)-4-Methyl-4H-furo[3,2-b]pyrrole-5-carboxylic acid {1-[2-cyclopropyl-5-(2-fluoro-phenyl)-thiazole-4-carbonyl]-piperidin-2-ylmethyl}-amide

LC-MS: t_{R} = 1.07 min; [M+H]⁺ = 507.13.

### B.1.12 Example 12: (S)-Thieno[2,3-b]pyrazine-6-carboxylic acid {1-[2-cyclopropyl-5-(2-fluoro-phenyl)-thiazole-4-carbonyl]-piperidin-2-ylmethyl}-amide

LC-MS: t_{R} = 1.03 min; [M+H]⁺ = 522.07.

### B.1.13 Example 13: (S)-1-Methyl-1H-indole-6-carboxylic acid {1-[2-cyclopropyl-5-(2-fluoro-phenyl)-thiazole-4-carbonyl]-piperidin-2-ylmethyl}-amide

LC-MS: t_{R} = 1.06 min; [M+H]⁺ = 517.20.

### B.1.14 Example 14: (S)-1-Methyl-1H-indole-4-carboxylic acid {1-[2-cyclopropyl-5-(2-fluoro-phenyl)-thiazole-4-carbonyl]-piperidin-2-ylmethyl}-amide

LC-MS: t_{R} = 1.06 min; [M+H]⁺ = 517.18.

### B.1.15 Example 15: (S)-Benzo[d]isoxazole-3-carboxylic acid {1-[2-cyclopropyl-5-(2-fluoro-phenyl)-thiazole-4-carbonyl]-piperidin-2-ylmethyl}-amide

LC-MS: t_{R} = 1.07 min; [M+H]⁺ = 505.15.

### B.1.16 Example 16: (S)-N-{1-[2-Cyclopropyl-5-(4-fluoro-phenyl)-thiazole-4-carbonyl]-piperidin-2-ylmethyl}-3-methyl-benzamide

### STEP 1: (S)-{1-[2-Cyclopropyl-5-(4-fluoro-phenyl)-thiazole-4-carbonyl]-piperidin-2-ylmethyl}-carbamic acid tert-butyl ester

According to the experimental procedure described in B.1.1 STEP 1;

LC-MS: t_{R} = 1.04 min; [M+H]⁺ = 460.24.

### STEP 2: (S)-(2-Aminomethyl-piperidin-1-yl)-[2-cyclopropyl-5-(4-fluoro-phenyl)-thiazol-4-yl]-methanone hydrochloride

According to the experimental procedure described in B.1.1 STEP 2;

LC-MS: t_{R} = 0.76 min; [M+H]⁺ = 360.18.

### STEP 3: (S)-N-{1-[2-Cyclopropyl-5-(4-fluoro-phenyl)-thiazole-4-carbonyl]-piperidin-2-ylmethyl}-3-methyl-benzamide (Example 16)

According to the experimental procedure described in B.1.1 STEP 3;

LC-MS: t_{R} = 1.07 min; [M+H]⁺ = 478.16.

Examples 17 to 30 were prepared according to the procedures described above by using the appropriate carboxylic acid in STEP 3.

### B.1.17 Example 17: (S)-3-Chloro-N-{1-[2-cyclopropyl-5-(4-fluoro-phenyl)-thiazole-4-carbonyl]-piperidin-2-ylmethyl}-benzamide

LC-MS: t_{R} = 1.08 min; [M+H]⁺ = 498.11.

### B.1.18 Example 18: (S)-3-Bromo-N-{1-[2-cyclopropyl-5-(4-fluoro-phenyl)-thiazole-4-carbonyl]-piperidin-2-ylmethyl}-benzamide

LC-MS: t_{R} = 1.09 min; [M+H]⁺ = 542.04.

### B.1.19 Example 19: (S)-Naphthalene-1-carboxylic acid {1-[2-cyclopropyl-5-(4-fluoro-phenyl)-thiazole-4-carbonyl]-piperidin-2-ylmethyl}-amide

LC-MS: t_{R} = 1.09 min; [M+H]⁺ = 514.23.

### B.1.20 Example 20: (S)-Quinoline-8-carboxylic acid {1-[2-cyclopropyl-5-(4-fluorophenyl)-thiazole-4-carbonyl]-piperidin-2-ylmethyl}-amide

LC-MS: t_{R} = 0.97 min; [M+H]⁺ = 515.21.

### B.1.21 Example 21: (S)-2,3-Dihydro-benzo[1,4]dioxine-5-carboxylic acid {1-[2-cyclopropyl-5-(4-fluoro-phenyl)-thiazole-4-carbonyl]-piperidin-2-ylmethyl}-amide

LC-MS: t_{R} = 1.06 min; [M+H]⁺ = 522.10.

### B.1.22 Example 22: (S)-Benzofuran-4-carboxylic acid {1-[2-cyclopropyl-5-(4-fluoro-phenyl)-thiazole-4-carbonyl]-piperidin-2-ylmethyl}-amide

LC-MS: t_{R} = 1.07 min; [M+H]⁺ = 504.15.

### B.1.23 Example 23: (S)-1-Methyl-1H-indole-3-carboxylic acid {1-[2-cyclopropyl-5-(4-fluoro-phenyl)-thiazole-4-carbonyl]-piperidin-2-ylmethyl}-amide

LC-MS: t_{R} = 1.05 min; [M+H]⁺ = 517.18.

### B.1.24 Example 24: (S)-1-Methyl-1H-indazole-3-carboxylic acid {1-[2-cyclopropyl-5-(4-fluoro-phenyl)-thiazole-4-carbonyl]-piperidin-2-ylmethyl}-amide

LC-MS: t_{R} = 1.07 min; [M+H]⁺ = 518.13.

### B.1.25 Example 25: (S)-6-Methyl-imidazo[2,1-b]thiazole-5-carboxylic acid {1-[2-cyclopropyl-5-(4-fluoro-phenyl)-thiazole-4-carbonyl]-piperidin-2-ylmethyl}-amide

LC-MS: t_{R} = 0.90 min; [M+H]⁺ = 524.13.

### B.1.26 Example 26: (S)-Imidazo[2,1-b]thiazole-5-carboxylic acid {1-[2-cyclopropyl-5-(4-fluoro-phenyl)-thiazole-4-carbonyl]-piperidin-2-ylmethyl}-amide

LC-MS: t_{R} = 0.93 min; [M+H]⁺ = 510.09.

### B.1.27 Example 27: (S)-4-Methyl-4H-furo[3,2-b]pyrrole-5-carboxylic acid {1-[2-cyclopropyl-5-(4-fluoro-phenyl)-thiazole-4-carbonyl]-piperidin-2-ylmethyl}-amide

LC-MS: t_{R} = 1.06 min; [M+H]⁺ = 507.13.

### B.1.28 Example 28: (S)-Thieno[2,3-b]pyrazine-6-carboxylic acid {1-[2-cyclopropyl-5-(4-fluoro-phenyl)-thiazole-4-carbonyl]-piperidin-2-ylmethyl}-amide

LC-MS: t_{R} = 1.01 min; [M+H]⁺ = 522.21.

### B.1.29 Example 29: (S)-1-Methyl-1H-indole-6-carboxylic acid {1-[2-cyclopropyl-5-(4-fluoro-phenyl)-thiazole-4-carbonyl]-piperidin-2-ylmethyl}-amide

LC-MS: t_{R} = 1.06 min; [M+H]⁺ = 517.17.

### B.1.30 Example 30: (S)-1-Methyl-1H-indole-4-carboxylic acid {1-[2-cyclopropyl-5-(4-fluoro-phenyl)-thiazole-4-carbonyl]-piperidin-2-ylmethyl}-amide

LC-MS: t_{R} = 1.06 min; [M+H]⁺ = 517.16.

### B.1.31 Example 31: (S)-6-Methyl-imidazo[2,1-b]thiazole-5-carboxylic acid [1-(2-cyclopropyl-5-p-tolyl-thiazole-4-carbonyl)-piperidin-2-ylmethyl]-amide

### STEP 1: (S)-[1-(2-Cyclopropyl-5-p-tolyl-thiazole-4-carbonyl)-piperidin-2-ylmethyl]-carbamic acid tert-butyl ester

According to the experimental procedure described in B.1.1 STEP 1;

LC-MS: t_{R} = 1.08 min; [M+H]⁺ = 456.22.

### STEP 2: (S)-(2-Aminomethyl-piperidin-1-yl)-(2-cyclopropyl-5-p-tolyl-thiazol-4-yl)-methanone hydrochloride

According to the experimental procedure described in B.1.1 STEP 2;

LC-MS: t_{R} = 0.79 min; [M+H]⁺ = 356.41.

### STEP 3 (Example 31): (S)-6-Methyl-imidazo[2,1-b]thiazole-5-carboxylic acid [1-(2-cyclopropyl-5-p-tolyl-thiazole-4-carbonyl)-piperidin-2-ylmethyl]-amide

According to the experimental procedure described in B.1.1 STEP 3;

LC-MS: t_{R} = 0.88 min; [M+H]⁺ = 520.47.

Examples 32 to 39 were prepared according to the procedures described above by using the appropriate carboxylic acid in STEP 3.

### B.1.32 Example 32: (S)-1-Methyl-1H-indazole-3-carboxylic acid [1-(2-cyclopropyl-5-p-tolyl-thiazole-4-carbonyl)-piperidin-2-ylmethyl]-amide

LC-MS: t_{R} = 1.05 min; [M+H]⁺ = 514.40.

### B.1.33 Example 33: (S)-Imidazo[2,1-b]thiazole-5-carboxylic acid [1-(2-cyclopropyl-5-p-tolyl-thiazole-4-carbonyl)-piperidin-2-ylmethyl]-amide

LC-MS: t_{R} = 0.90 min; [M+H]⁺ = 506.26.

### B.1.34 Example 34: (S)-Imidazo[2,1-b]thiazole-6-carboxylic acid [1-(2-cyclopropyl-5-p-tolyl-thiazole-4-carbonyl)-piperidin-2-ylmethyl]-amide

LC-MS: t_{R} = 0.95 min; [M+H]⁺ = 506.25.

### B.1.35 Example 35: (S)-1-Methyl-1H-indole-2-carboxylic acid [1-(2-cyclopropyl-5-p-tolyl-thiazole-4-carbonyl)-piperidin-2-ylmethyl]-amide

LC-MS: t_{R} = 1.09 min; [M+H]⁺ = 513.33.

### B.1.36 Example 36: (S)-3-Bromo-N-[1-(2-cyclopropyl-5-p-tolyl-thiazole-4-carbonyl)-piperidin-2-ylmethyl]-benzamide

LC-MS: t_{R} = 1.07 min; [M+H]⁺ = 540.15.

### B.1.37 Example 37: (S)-2,3-Dihydro-benzo[1,4]dioxine-5-carboxylic acid [1-(2-cyclopropyl-5-p-tolyl-thiazole-4-carbonyl)-piperidin-2-ylmethyl]-amide

LC-MS: t_{R} = 1.04 min; [M+H]⁺ = 518.30.

### B.1.38 Example 38: (S)-3-Chloro-N-[1-(2-cyclopropyl-5-p-tolyl-thiazole-4-carbonyl)-piperidin-2-ylmethyl]-benzamide

LC-MS: t_{R} = 1.06 min; [M+H]⁺ = 494.24.

### B.1.39 Example 39: (S)-N-[1-(2-Cyclopropyl-5-p-tolyl-thiazole-4-carbonyl)-piperidin-2-ylmethyl]-3-methyl-benzamide

LC-MS: t_{R} = 1 .05 min; [M+H]⁺ = 474.29.

### B.1.40 Example 40: (S)-N-[1-(2-Cyclopropyl-5-p-tolyl-thiazole-4-carbonyl)-piperidin-2-ylmethyl]-3-methyl-benzamide

### STEP 1: (S)-[1-(2-Cyclopropyl-5-m-tolyl-thiazole-4-carbonyl)-piperidin-2-ylmethyl]-carbamic acid tert-butyl ester

According to the experimental procedure described in B.1.1 STEP 1;

LC-MS: t_{R} = 1.08 min; [M+H]⁺ = 456.41.

### STEP 2: (S)-(2-Aminomethyl-piperidin-1-yl)-(2-cyclopropyl-5-m-tolyl-thiazol-4-yl)-methanone hydrochloride

According to the experimental procedure described in B.1.1 STEP 2;

LC-MS: t_{R} = 0.80 min; [M+H]⁺ = 356.13.

### STEP 3 (Example 40): (S)-N-[1-(2-Cyclopropyl-5-p-tolyl-thiazole-4-carbonyl) piperidin-2-ylmethyl]-3-methyl-benzamide

According to the experimental procedure described in B.1.1 STEP 3;

LC-MS: t_{R} = 0.98 min; [M+H]⁺ = 474.37.

Examples 41 to 53 were prepared according to the procedures described above by using the appropriate carboxylic acid in STEP 3.

### B.1.41 Example 41: (S)-3-Chloro-N-[1-(2-cyclopropyl-5-m-tolyl-thiazole-4-carbonyl)-piperidin-2-ylmethyl]-benzamide

LC-MS: t_{R} = 1.00 min; [M+H]⁺ = 494.36.

### B.1.42 Example 42: (S)-3-Bromo-N-[1-(2-cyclopropyl-5-m-tolyl-thiazole-4-carbonyl)-piperidin-2-ylmethyl]-benzamide

LC-MS: t_{R} = 1.01 min; [M+H]⁺ = 540.34.

### B.1.43 Example 43: (S)-Naphthalene-1-carboxylic acid [1-(2-cyclopropyl-5-m-tolyl-thiazole-4-carbonyl)-piperidin-2-ylmethyl]-amide

LC-MS: t_{R} = 1.01 min; [M+H]⁺ = 510.41.

### B.1.44 Example 44: (S)-Quinoline-8-carboxylic acid [1-(2-cyclopropyl-5-m-tolyl-thiazole-4-carbonyl)-piperidin-2-ylmethyl]-amide

LC-MS: t_{R} = 0.98 min; [M+H]⁺ = 511.41.

### B.1.45 Example 45: (S)-2,3-Dihydro-benzo[1,4]dioxine-5-carboxylic acid [1-(2-cyclopropyl-5-m-tolyl-thiazole-4-carbonyl)-piperidin-2-ylmethyl]-amide

LC-MS: t_{R} = 0.96 min; [M+H]⁺ = 518.41.

### B.1.46 Example 46: (S)-Benzofuran-4-carboxylic acid [I-(2-cyclopropyl-5-m-tolyl-thiazole-4-carbonyl)-piperidin-2-ylmethyl]-amide

LC-MS: t_{R} = 0.98 min; [M+H]⁺ = 500.39.

### B.1.47 Example 47: (S)-1-Methyl-1H-indole-3-carboxylic acid [1-(2-cyclopropyl-5-m-tolyl-thiazole-4-carbonyl)-piperidin-2-ylmethyl]-amide

LC-MS: t_{R} = 0.95 min; [M+H]⁺ = 513.42.

### B.1.48 Example 48: (S)-1-Methyl-1H-indazole-3-carboxylic acid [1-(2-cyclopropyl-5-m-tolyl-thiazole-4-carbonyl)-piperidin-2-ylmethyl]-amide

LC-MS: t_{R} = 0.98 min; [M+H]⁺ = 514.43.

### B.1.49 Example 49: (S)-6-Methyl-imidazo[2,1-b]thiazole-5-carboxylic acid [1-(2-cyclopropyl-5-m-tolyl-thiazole-4-carbonyl)-piperidin-2-ylmethyl]-amide

LC-MS: t_{R} = 0.88 min; [M+H]⁺ = 520.38.

### B.1.50 Example 50: (S)-Imidazo[2,1-b]thiazole-5-carboxylic acid [1-(2-cyclopropyl-5-m-tolyl-thiazole-4-carbonyl)-piperidin-2-ylmethyl]-amide

LC-MS: t_{R} = 0.86 min; [M+H]⁺ = 506.38.

### B.1.51 Example 51: (S)-4-Methyl-4H-furo[3,2-b]pyrrole-5-carboxylic acid [1-(2-cyclopropyl-5-m-tolyl-thiazole-4-carbonyl)-piperidin-2-ylmethyl]-amide

LC-MS: t_{R} = 0.97 min; [M+H]⁺ = 503.39.

### B.1.52 Example 52: (S)-1-Methyl-1H-indole-5-carboxylic acid [1-(2-cyclopropyl-5-m-tolyl-thiazole-4-carbonyl)-piperidin-2-ylmethyl]-amide

LC-MS: t_{R} = 0.95 min; [M+H]⁺ = 513.44.

### B.1.53 Example 53: (S)-1-Methyl-1H-indole-4-carboxylic acid [1-(2-cyclopropyl-5-m-tolyl-thiazole-4-carbonyl)-piperidin-2-ylmethyl]-amide

LC-MS: t_{R} = 0.96 min; [M+H]⁺ = 513.43.

### B.1.54 Example 54: (S)-N-{1-[5-(3-Chloro-phenyl)-2-cyclopropyl-thiazole-4-carbonyl]-piperidin-2-ylmethyl}-3-methyl-benzamide

### STEP 1: (S)-{1-[5-(3-Chloro-phenyl)-2-cyclopropyl-thiazole-4-carbonyl]-piperidin-2-ylmethyl}-carbamic acid tert-butyl ester

According to the experimental procedure described in B.1.1 STEP 1;

LC-MS: t_{R} = 1.08 min; [M+H]⁺ = 476.19.

### STEP 2: (S)-(2-Aminomethyl-piperidin-1-yl)-[5-(3-chloro-phenyl)-2-cyclopropyl-thiazol-4-yl]-methanone hydrochloride

According to the experimental procedure described in B.1.1 STEP 2;

LC-MS: t_{R} = 0.80 min; [M+H]⁺ = 376.05.

### STEP 3 (Example 54): (S)-N-{1-[5-(3-Chloro-phenyl)-2-cyclopropyl-thiazole-4-carbonyl]-piperidin-2-ylmethyl}-3-methyl-benzamide

According to the experimental procedure described in B.1.1 STEP 3;

LC-MS: t_{R} = 1.10 min; [M+H]⁺ = 494.17.

Examples 55 to 68 were prepared according to the procedures described above by using the appropriate carboxylic acid in STEP 3.

### B.1.55 Example 55: (S)-3-Chloro-N-{1-[5-(3-chloro-phenyl)-2-cyclopropyl-thiazole-4-carbonyl]-piperidin-2-ylmethyl}-benzamide

LC-MS: t_{R} = 1.11 min; [M+H]⁺ = 514.08.

### B.1.56 Example 56: (S)-3-Bromo-N-{1-[5-(3-chloro-phenyl)-2-cyclopropyl-thiazole-4-carbonyl]-piperidin-2-ylmethyl}-benzamide

LC-MS: t_{R} = 1.12 min; [M+H]⁺ = 559.99.

### B.1.57 Example 57: (S)-Naphthalene-1-carboxylic acid {1-[5-(3-chloro-phenyl)-2-cyclopropyl-thiazole-4-carbonyl]-piperidin-2-ylmethyl}-amide

LC-MS: t_{R} = 1.13 min; [M+H]⁺ = 530.10.

### B.1.58 Example 58: (S)-Quinoline-8-carboxylic acid {1-[5-(3-chloro-phenyl)-2-cyclopropyl-thiazole-4-carbonyl]-piperidin-2-ylmethyl}-amide

LC-MS: t_{R} = 1.00 min; [M+H]⁺ = 531.15.

### B.1.59 Example 59: (S)-2,3-Dihydro-benzo[1,4]dioxine-5-carboxylic acid {1-[5-(3-chloro-phenyl)-2-cyclopropyl-thiazole-4-carbonyl]-piperidin-2-ylmethyl}-amide

LC-MS: t_{R} = 1.09 min; [M+H]⁺ = 538.10.

### B.1.60 Example 60: (S)-Benzofuran-4-carboxylic acid {1-[5-(3-chloro-phenyl)-2-cyclopropyl-thiazole-4-carbonyl]-piperidin-2-ylmethyl}-amide

LC-MS: t_{R} = 1.00 min; [M+H]⁺ = 520.10.

### B.1.61 Example 61: (S)-1-Methyl-1H-indole-3-carboxylic acid 11-[5-(3-chloro-phenyl)-2-cyclopropyl-thiazole-4-carbonyl]-piperidin-2-ylmethyl}-amide

LC-MS: t_{R} = 1.09 min; [M+H]⁺ = 533.13.

### B.1.62 Example 62: (S)-1-Methyl-1H-indazole-3-carboxylic acid {1-[5-(3-chlorophenyl)-2-cyclopropyl-thiazole-4-carbonyl]-piperidin-2-ylmethyl}-amide

LC-MS: t_{R} = 1.10 min; [M+H]⁺ = 534.12.

### B.1.63 Example 63: (S)-6-Methyl-imidazo[2,1-b]thiazole-5-carboxylic acid {1-[5-(3-chloro-phenyl)-2-cyclopropyl-thiazole-4-carbonyl]-piperidin-2-ylmethyl }-amide

LC-MS: t_{R} = 0.95 min; [M+H]⁺ = 540.07.

### B.1.64 Example 64: (S)-Imidazo[2,1-b]thiazole-5-carboxylic acid {1-[5-(3-chlorophenyl)-2-cyclopropyl-thiazole-4-carbonyl]-piperidin-2-ylmethyl}-amide

LC-MS: t_{R} = 0.96 min; [M+H]⁺ = 526.06.

### B.1.65 Example 65: (S)-4-Methyl-4H-furo[3,2-b]pyrrole-5-carboxylic acid {1-[5-(3-chloro-phenyl)-2-cyclopropyl-thiazole-4-carbonyl]-piperidin-2-ylmethyl}-amide

LC-MS: t_{R} = 1.10 min; [M+H]⁺ = 523.10.

### B.1.66 Example 66: (S)-1-Methyl-1H-indole-5-carboxylic acid {1-[5-(3-chlorophenyl)-2-cyclopropyl-thiazole-4-carbonyl]-piperidin-2-ylmethyl}-amide

LC-MS: t_{R} = 1.09 min; [M+H]⁺ = 533.11.

### B.1.67 Example 67: (S)-1-Methyl-1H-indole-4-carboxylic acid {1-[5-(3-chlorophenyl)-2-cyclopropyl-thiazole-4-carbonyl]-piperidin-2-ylmethyl}-amide

LC-MS: t_{R} = 1.09 min; [M+H]⁺ = 533.18.

### B.1.68 Example 68: (S)-Benzo[d]isoxazole-3-carboxylic acid {1-[5-(3-chlorophenyl)-2-cyclopropyl-thiazole-4-carbonyl]-piperidin-2-ylmethyl}-amide

LC-MS: t_{R} = 1.10 min; [M+H]⁺ = 521.10.

### B.1.69 Example 69: (S)-N-{1-[2-Cyclopropyl-5-(3-trifluoromethyl-phenyl)-thiazole-4-carbonyl]-piperidin-2-ylmethyl}-3-methyl-benzamide

### STEP 1: (S)-{1-[2-Cyclopropyl-5-(3-trifluoromethyl-phenyl)-thiazole-4-carbonyl]-piperidin-2-ylmethyl}-carbamic acid tert-butyl ester

According to the experimental procedure described in B.1.1 STEP 1;

LC-MS: t_{R} = 1.10 min; [M+H]⁺ = 510.12.

### STEP 2: (S)-(2-Aminomethyl-piperidin-1-yl)-[2-cyclopropyl-5-(3-trifluoromethylphenyl)-thiazol-4-yl]-methanone hydrochloride

According to the experimental procedure described in B.1.1 STEP 2;

LC-MS: t_{R} = 0.82 min; [M+H]⁺ = 410.11.

### STEP 3 (Example 69): (S)-N-{1-[2-Cyclopropyl-S-(3-trifluoromethyl-phenyl)-thiazole-4-carbonyl]-piperidin-2-ylmethyl}-3-methyl-benzamide

According to the experimental procedure described in B.1.1 STEP 3;

LC-MS: t_{R} = 1.12 min; [M+H]⁺ = 528.14.

Examples 70 to 81 were prepared according to the procedures described above by using the appropriate carboxylic acid in STEP 3.

### B.1.70 Example 70: (S)-3-Chloro-N-{1-[2-cyclopropyl-5-(3-trifluoromethylphenyl)-thiazole-4-carbonyl]-piperidin-2-ylmethyl}-benzamide

LC-MS: t_{R} = 1.13 min; [M+H]⁺ = 548.17.

### B.1.71 Example 71: (S)-3-Bromo-N-{1-[2-cyclopropyl-5-(3-trifluoromethylphenyl)-thiazole-4-carbonyl]-piperidin-2-ylmethyl}-benzamide

LC-MS: t_{R} = 1.14 min; [M+H]⁺ = 592.13.

### B.1.72 Example 72: (S)-Naphthalene-1-carboxylic acid {1-[2-cyclopropyl-5-(3-trifluoromethyl-phenyl)-thiazole-4-carbonyl]-piperidin-2-ylmethyl}-amide

LC-MS: t_{R} = 1.14 min; [M+H]⁺ = 564.26.

### B.1.73 Example 73: (S)-Quinoline-8-carboxylic acid {1-[2-cyclopropyl-5-(3-trifluoromethyl-phenyl)-thiazole-4-carbonyl]-piperidin-2-ylmethyl}-amide

LC-MS: t_{R} = 1.03 min; [M+H]⁺ = 565.35.

### B.1.74 Example 74: (S)-2,3-Dihydro-benzo[1,4]dioxine-5-carboxylic acid {1-[2-cyclopropyl-5-(3-trifluoromethyl-phenyl)-thiazole-4-carbonyl]-piperidin-2-ytmethyl}-amide

LC-MS: t_{R} = 1.11 min; [M+H]⁺ = 572.33.

### B.1.75 Example 75: (S)-Benzofuran-4-carboxylic acid {1-[2-cyclopropyl-5-(3-trifluoromethyl-phenyl)-thiazole-4-carbonyl]-piperidin-2-ylmethyl}-amide

LC-MS: t_{R} = 1.12 min; [M+H]⁺ = 554.07.

### B.1.76 Example 76: (S)-1-Methyl-1H-indole-3-carboxylic acid {1-[2-cyclopropyl-5-(3-trifluoromethyl-phenyl)-thiazole-4-carbonyl]-piperidin-2-ylmethyl}-amide

LC-MS: t_{R} = 1.10 min; [M+H]⁺ = 567.15.

### B.1.77 Example 77: (S)-1-Methyl-1H-indazole-3-carboxylic acid {1-[2-cyclopropyl5-(3-trifluoromethyl-phenyl)-thiazole-4-carbonyl]-piperidin-2-ylmethyl}-amide

LC-MS: t_{R} = 1.12 min; [M+H]⁺ = 568.20.

### B.1.78 Example 78: (S)-6-Methyl-imidazo[2,1-b]thiazole-5-carboxylic acid {1-[2-cyclopropyl-5-(3-trifluoromethyl-phenyl)-thiazole-4-carbonyl]-piperidin-2-ylmethyl}-amide

LC-MS: t_{R} = 0.97 min; [M+H]⁺ = 574.13.

### B.1.79 Example 79: (S)-Imidazo[2,1-b]thiazole-5-carboxylic acid {1-[2-cyclopropyl-5-(3-trifluoromethyl-phenyl)-thiazole-4-carbonyl]-piperidin-2-ylmethyl}-amide

LC-MS: t_{R} = 0.99 min; [M+H]⁺ = 560.08.

### B.1.80 Example 80: (S)-4-Methyl-4H-furo[3,2-b]pyrrole-5-carboxylic acid {1-[2-cyclopropyl-5-(3-trifluoromethyl-phenyl)-thiazole-4-carbonyl]-piperidin-2-ylmethyl}-amide

LC-MS: t_{R} = 1.12 min; [M+H]⁺ = 557.30.

### B.1.81 Example 81: (S)-Benzo[d]isoxazole-3-carboxylic acid {1-[2-cyclopropyl-5-(3-trifluoromethyl-phenyl)-thiazole-4-carbonyl]-piperidin-2-ylmethyl}-amide

LC-MS: t_{R} = 1.12 min; [M+H]⁺ = 555.06.

### C.1.1 Example 82: (S)-N-{1-[2-Cyclopropyl-5-(4-fluoro-phenyl)-thiazole-4-carbonyl]-pyrrolidin-2-ylmethyl}-3-methyl-benzamide

### STEP 1: (S)-2-[(2,2,2-Trifluoro-acetylamino)-methyl]-pyrrolidine-1-carboxylic acid tert-butyl ester

(S)-1-Boc-2-(aminomethyl)pyrrolidine (10 g, 50 mmol) was dissolved in dry THF (200 mL) followed by the slow addition (within 5 min) of a solution of ethyl trifluoroacetate (9.75 g, 68 mmol) in THF (50 mL). Stirring at rt was continued for 2 h. The reaction mixture was evaporated to dryness and the residue dried at HV to give (S)-2-[(2,2,2-trifluoro-acetylamino)-methyl]-pyrrolidine-1-carboxylic acid tert-butyl ester (14.8 g, quant. yield). LC-MS: t_{R} = 0.93 min; [M+H]⁺ = 297.29.

### STEP 2: (S)-2,2,2-Trifluoro-N-pyrrolidin-2-ylmethyl-acetamide hydrochloride

(S)-2-[(2,2,2-Trifluoro-acetylamino)-methyl]-pyrrolidine-1-carboxylic acid tert-butyl ester (13.8 g, 46.8 mmol) was dissolved in THF (200 mL) and cooled to 0°C followed by the addition of a solution of HCl in dioxane (4M, 70 mL) over 5 min. Stirring was continued for 2 h at rt. The mixture was concentrated under reduced pressure and diethylether (100 mL) was added to the residue leading to a white precipitate which was filtered off and dried at HV to give (S)-2,2,2-trifluoro-N-pyrrolidin-2-ylmethyl-acetamide hydrochloride (11.2 g, quant. yield). LC-MS: t_{R} = 0.25 min; [M+H]⁺ - 197.29.

### STEP 3: (S)-N-{1-[2-cyclopropyl-5-(4-fluoro-phenyl)-thiazole-4-carbonyl]-pyrrolidin-2-ylmethyl}-2,2,2-trifluoro-acetamide

2-Cyclopropyl-5-(4-fluoro-phenyl)-thiazole-4-carboxylic acid (645 mg, 2.45 mmol) was dissolved in MeCN (10 mL). TBTU (860 mg, 2.67 mmol) and DIPEA (0.95 mL, 5.57 mmol) were added and stirring continued at rt for 15 min followed by the addition of a solution of (S)-2,2,2-trifluoro-N-pyrrolidin-2-ylmethyl-acetamide (520 mg, 2.23 mmol) in MeCN (10 mL). Stirring at rt was continued for 3 h. The reaction mixture was diluted with EtOAc (180 mL) and subsequently washed with 1 M aq. HCl (100 mL), sat. NaHCO₃ solution (100 mL) and brine (100 mL), dried over MgSO₄, filtered and concentrated under reduced pressure to give (S)-N-{1-[2-cyclopropyl-5-(4-fluorophenyl)-thiazole-4-carbonyl]-pyrrolidin-2-ylmethyl}-2,2,2-trifluoro-acetamide as a slightly coloured oil in quant. yield. LC-MS: t_{R} = 1.05 min; [M+H]⁺ = 442.29.

### STEP 4: (S)-(2-Aminomethyl-pyrrolidin-1-yl)-[2-cyclopropyl-5-(4-fluoro-phenyl)-thiazol-4-yl]-methanone

(S)-N-{1-[2-Cyclopropyl-5-(4-fluoro-phenyl)-thiazole-4-carbonyl]-pyrrolidin-2-ylmethyl}-2,2,2-trifluoro-acetamide (984 mg, 2.23 mmol) was dissolve in isopropanol (60 mL). Aq. NaOH solution (1M, 5 mL) was added and the reaction was stirred at rt for 3 h. The mixture was diluted with EtOAc (300 mL) and washed with brine (180 mL), dried over MgSO₄, filtered and concentrated in vacuo to give (S)-(2-aminomethyl-pyrrolidin-1-yl)-[2-cyclopropyl-5-(4-fluoro-phenyl)-thiazol-4-yl]-methanone in quant. yield. LC-MS: t_{R} = 0.80 min; [M+H]⁺ = 346.23.

### STEP 5: (S)-N-{1-[2-Cyclopropyl-5-(4-fluoro-phenyl)-thiazole-4-carbonyl]-pyrrolidin-2-ylmethyl}-3-methyl-benzamide (Example 82)

3-Methyl-benzoic acid (0.153 mmol, 1.1 eq) was dissolved in MeCN (1 mL) and TBTU (0.167 mmol, 1.2 eq) and DIPEA (0.417 mmol, 3 eq) were added and stirring continued at rt for 15 min followed by the addition of (S)-(2-aminomethyl-pyrroildin-1-yl)-[2-cyclopropyl-5-(4-fluoro-phenyl)-thiazol-4-yl]-methanone (0.14 mmol, 1 eq) dissolved in DCM (1 mL). Stirring at rt was continued for 16 h. The reaction mixture was concentrated, the residue was dissolved in MeCN (1 mL) and purified by preparative HPLC to give (S)-N-{1-[2-cyclopropyl-5-(4-fluoro-phenyl)-thiazole-4-carbonyl]-pyrrolidin-2-ylmethyl}-3-methyl-benzamide (33.3 mg, 51 %) as a white solid. LC-MS: t_{R} = 1.06 min; [M+H]⁺ = 464.18.

Examples 83 to 95 were prepared according to the experimental procedure described above in C.1.1 STEP 5 by using the appropriate carboxylic acid.

### C.1.2 Example 83: (S)-3-Chloro-N-{1-[2-cyclopropyl-5-(4-fluoro-phenyl)-thiazole-4-carbonyl]-pyrrolidin-2-ylmethyl}-benzamide

LC-MS: t_{R} = 1.07 min; [M+H]⁺ = 484.04.

### C.1.3 Example 84: (S)-3-Bromo-N-{1-[2-cyclopropyl-5-(4-fluoro-phenyl)-thiazole-4-carbonyl]-pyrrolidin-2-ylmethyl}-benzamide

LC-MS: t_{R} = 1.08 min; [M+H]⁺ = 528.06.

### C.1.4 Example 85: (S)-Naphthalene-1-carboxylic acid {1-[2-cyclopropyl-5-(4-fluoro-phenyl)-thiazole-4-carbonyl]-pyrrolidin-2-ylmethyl}-amide

LC-MS: t_{R} = 1.07 min; [M+H]⁺ = 500.16.

### C.1.5 Example 86: (S)-Quinoline-8-carboxylic acid {1-[2-cyclopropyl-5-(4-fluorophenyl)-thiazole-4-carbonyl]-pyrrolidin-2-ylmethyl}-amide

LC-MS: t_{R} = 0.98 min; [M+H]⁺ = 501.15.

### C.1.6 Example 87: (S)-2,3-Dihydro-benzo[1,4]dioxine-5-carboxylic acid {1-[2-cyclopropyl-5-(4-fluoro-phenyl)-thiazole-4-carbonyl]-pyrrolidin-2-ylmethyl}-amide

LC-MS: t_{R} = 1.03 min; [M+H]⁺ = 508.17.

### C.1.7 Example 88: (S)-Benzofuran-4-carboxylic acid {1-[2-cyclopropyl-5-(4-fluorophenyl)-thiazole-4-carbonyl]-pyrrolidin-2-ylmethyl}-amide

LC-MS: t_{R} = 1.06 min; [M+H]⁺ = 490.15.

### C.1.8 Example 89: (S)-1-Methyl-1H-indole-3-carboxylic acid {1-[2-cyclopropyl-5-(4-fluoro-phenyl)-thiazole-4-carbonyl]-pyrrolidin-2-ylmethyl}-amide

LC-MS: t_{R} = 1.04 min; [M+H]⁺ = 503.22.

### C.1.9 Example 90: (S)-1-Methyl-1H-indazole-3-carboxylic acid {1-[2-cyclopropyl-5-(4- fluoro-phenyl)-thiazole-4-carbonyl]-pyrrolidin-2-ylmethyl}-amide

LC-MS: t_{R} = 1.04 min; [M+H]⁺ = 504.22.

### C.1.10 Example 91: (S)-6-Methyl-imidazo[2,1-b]thiazole-5-carboxylic acid {1-[2-cyclopropyl-5-(4-fluoro-phenyl)-thiazole-4-carbonyl]-pyrrolidin-2-ylmethyl}-amide

LC-MS: t_{R} = 0.91 min; [M+H]⁺ = 510.11.

### C.1.11 Example 92: (S)-Imidazo[2,1-b]thiazole-5-carboxylic acid {1-[2-cyclopropyl-5-(4-fluoro-phenyl)-thiazole-4-carbonyl]-pyrrolidin-2-ylmethyl}-amide

LC-MS: t_{R} = 0.94 min; [M+H]⁺ = 496.11.

### C.1.12 Example 93: (S)-4-Methyl-4H-furo[3,2-b]pyrrole-5-carboxylic acid {1-[2-cyclopropyl-5-(4-fluoro-phenyl)-thiazole-4-carbonyl]-pyrrolidin-2-ylmethyl}-amide

LC-MS: t_{R} = 1.04 min; [M+H]⁺ = 493.16.

### C.1.13 Example 94: (S)-1-Methyl-1H-indole-4-carboxylic acid {1-[2-cyclopropyl-5-(4-fluoro-phenyl)-thiazole-4-carbonyl]-pyrrolidin-2-ylmethyl}-amide

LC-MS: t_{R} = 1.04 min; [M+H]⁺ = 503.18.

### C.1.14 Example 95: (S)-Benzo[d]isoxazole-3-carboxylic acid {1-[2-cyclopropyl-5-(4-fluoro-phenyl)-thiazole-4-carbonyl]-pyrrolidin-2-ylmethyl}-amide

LC-MS: t_{R} = 1.04 min; [M+H]⁺ = 491.14. **Second synthetic pathway to prepare derivatives of the present invention exemplified with Example 31**

### D.1.1 (S)-6-Methyl-imidazo[2,1-b]thiazole-5-carboxylic acid [1-(2-cyclopropyl-5-p-tolyl-thiazole-4-carbonyl)-piperidin-2-ylmethyl]-amide (Example 31):

### STEP 1:

6-Methyl-imidazo[2,1-b]thiazole-5-carboxylic acid (2.0 g, 10.975 mmol) was dissolved in MeCN (35 mL), TBTU (3.52 g, 10.975 mmol) was added followed by the addition of DIPEA (2.82 mL, 16.462 mmol) and a solution of (S)-2-aminomethyl-piperidine-1-carboxylic acid tert-butyl ester (2.35 g, 10.975 mmol) in MeCN (20 mL). Stirring at rt was continued for 20 h. The reaction mixture was concentrated under reduced pressure, again diluted with EtOAc and subsequently washed with a sat. citric acid solution, a sat. NaHCO₃ solution and brine. The organic layer was dried over MgSO₄, filtered and concentrated under reduced pressure to give 3.98 g (95%) of (S)-2-{[(6-methyl-imidazo[2,1-b]thiazole-5-carbonyl)-amino]-methyl}-piperidine-1-carboxylic acid tert-butyl ester as a slightly orange foam which was used without further purification. LC-MS: t_{R} = 0.80 min; [M+H]⁺ = 379.46.

### STEP 2:

(S)-2-{[(6-Methyl-imidazo[2,1-b]thiazole-5-carbonyl)-amino]-methyl}-piperidine-1-carboxylic acid tert-butyl ester (3.88 g, 10.251 mmol) was dissolved in dioxane (25 mL) followed by the addition of a 4 M solution of HCl in dioxane (25 mL). Stirring at rt was continued for 1 h. The reaction mixture was concentrated under reduced pressure and the residue was dried under HV to give 3.22 g (quant. yield) of (S)-6-methyl-imidazo[2,1-b]thiazole-5-carboxylic acid (piperidin-2-ylmethyl)-amide hydrochloride which was used without further purification. LC-MS: t_{R} = 0.49 min; [M+H]⁺ = 279.41.

### STEP 3:

5-(p-tolyl)-2-cyclopropyl-thiazole-4-carboxylic acid (48.46 mg, 0.187 mmol) was dissolved in DCM (1 mL), followed by the addition of 1-chloro-N,N,2-triemthylpropenylamine (28.4 mg, 0.21 mmol). The mixture was stirred for 30 min at rt followed by the addidion of DIPEA (0.087 mL, 0.51 mmol) and (S)-6-methyl-imidazo[2,1-b]thiazole-5-carboxylic acid (piperidin-2-ylmethyl)-amide hydrochloride (53.5 mg, 0.17 mmol) dissolved in DCM (1 mL). Stirring was continued for 30 min at rt. The reaction mixture was concentrated under reduced pressure, dissolved in MeCN (0.8 mL), and the product was isolated by preparative HPLC purification to give 54 mg (61%) of (S)-6-Methyl-imidazo[2,1-b]thiazole-5-carboxylic acid [1-(2-cyclopropyl-5-p-tolyl-thiazole-4-carbonyl)-piperidin-2-ylmcthyl]-amide (Example 31). LC-MS: t_{R} = 0.88 min; [M+H]⁺ = 520.37.

The preparation of the following Examples 96 to 145 was achieved according to the procedures described above for the preparation of Examples 1 to 95:

### Example 96: (S)-Imidazo[2,1-b]thiazole-5-carboxylic acid [1-(2-cyclopropyl-5-phenyl-thiazole-4-carbonyl)-piperidin-2-ylmethyl]-amide:

LC-MS: t_{R} = 0.91 min; [M+H]⁺ = 492.23.

### Example 97: (S)-6-Methyl-imidazo[2,1-b]thiazole-5-carboxylic acid [1-(2-cyclopropyl-5-phenyl-thiazole-4-carbonyl)-piperidin-2-ylmethyl]-amide:

LC-MS: t_{R} = 0.89 min; [M+H]⁺ = 506.26.

### Example 98: (S)-2,3-Dihydro-benzofuran-4-carboxylic acid [1-(2-cyclopropyl-5-phenyl-thiazole-4-carbonyl)-piperidin-2-ylmethyl]-amide:

LC-MS: t_{R} = 1.04 min; [M+H]⁺ = 488.30.

### Example 99: (S)-Benzofuran-4-carboxylic acid [1-(2-cyclopropyl-5-phenyl-thiazole-4-carbonyl)-piperidin-2-ylmethyl]-amide:

LC-MS: t_{R} = 1.05 min; [M+H]⁺ = 486.28.

### Example 100: (S)-1-Methyl-1H-indazote-3-carboxylic acid [1-(2-cyclopropyl-5-phenyl-thiazole-4-carbonyl)-piperidin-2-ylmethyl]-amide:

LC-MS: t_{R} = 1.05 min; [M+H]⁺ = 500.29.

### Example 101: (S)-Quinoline-8-carboxylic acid [1-(2-cyclopropyl-5-phenyl-thiazole-4-carbonyl)-piperidin-2-ylmethyl]-amide:

LC-MS: t_{R} = 0.95 min; [M+H]⁺ = 497.29.

### Example 102: (S)-N-[1-(2-Cyclopropyl-5-phenyl-thiazote-4-carbonyl)-piperidin-2-ylmethyl]-3,4-dimethyl-benzamide:

LC-MS: t_{R} = 1.08 min; [M+H]⁺ = 474.33.

### Example 103: (S)- N- [1-(2-Cyclopropyl-5-phenyl- thiazole-4-car bonyl)-piperidin-2-ylmethyl]-4-methyl-3-trifluoromethyl-benzamide:

LC-MS: t_{R} = 1.11 min; [M+H]⁺ = 528.25.

### Example 104: (S)-3-Cyano-N-[1-(2-cyclopropyl-5-m-tolyl-thiazole-4-carbonyl)-piperidin-2-ylmethyl]-benzamide:

LC-MS: t_{R} = 1.06 min; [M+H]⁺ = 485.10.

### Example 105: (S)-1-Methyl-1H-indole-3-carboxylic acid [1-(2-cyclopropyl-5-phenyl-thiazole-4-carbonyl)-piperidin-2-ylmethyl]-amide:

LC-MS: t_{R} = 1.03 min; [M+H]⁺ = 499.30.

### Example 106: (S)-2,3-Dihydro-benzofuran-7-carboxylic acid [1-(2-cyclopropyl-5-m-tolyl-thiazole-4-carbonyl)-piperidin-2-ylmethyl]-amide:

LC-MS: t_{R} = 1.08 min; [M+H]⁺ = 502.07.

### Example 107: (S)-2,3-Dihydro-benzofuran-4-carboxylic acid [1-(2-cyclopropyl-5-m-tolyl-thiazole-4-carbonyl)-piperidin-2-ylmethyl]-amide:

LC-MS: t_{R} = 1.13 min; [M+H]⁺ = 501.99.

### Example 108: (S)-2-Ethyl-5-methyl-2H-pyrazole-3-carboxylic acid [1-(2-cyclopropyl-5-m-tolyl-thiazole-4-carbonyl)-piperidin-2-ylmethyl]-amide:

LC-MS: t_{R} = 1.10 min; [M+H]⁺ = 492.06.

### Example 109: (S)-2,5-Dimethyl-2H-pyrazole-3-carboxylic acid [1-(2-cyclopropyl-5-m-tolyl-thiazole-4-carbonyl)-piperidin-2-ylmethyl]-amide:

LC-MS: t_{R} = 1.04 min; [M+H]⁺ = 478.04.

### Example 110: (S)-3,4-Dihydro-2H-benzo[1,4]oxazine-5-carboxylic acid [1-(2-cyclopropyl-5-m-tolyl-thiazole-4-carbonyl)-piperidin-2-ylmethyl]-amide:

LC-MS: t_{R} = 1.17 min; [M+H]⁺ = 517.03.

### Example 111: (S)-N-[1-(2-Cyclopropyl-5-m-tolyl-thiazole-4-carbonyl)-piperidin-2-ylmethyl]-3-methoxy-benzamide:

LC-MS: t_{R} = 1.07 min; [M+H]⁺ = 490.08.

### Example 112: (S)-N-[1-(2-Cyclopropyl-5-m-tolyl-thiazole-4-carbonyl)-piperidin-2-ylmethyl]-3-trifluoromethyl-benzamide:

LC-MS: t_{R} = 1.12 min; [M+H]⁺ = 528.08.

### Example 113: (S)-2,3-Dihydro-thieno[3,4-b][1,4]dioxine-5-carboxylic acid [1-(2-cyclopropyl-5-m-tolyl-thiazole-4-carbonyl)-piperidin-2-ylmethyl]-amide:

LC-MS: t_{R} = 1.10 min; [M+H]⁺ = 523.93.

### Example 114: (S)-N-[1-(2-Cyclopropyl-5-m-tolyl-thiazole-4-carbonyl)-piperidin-2-ylmethyl]-3-fluoro-2-methyl-benzamide:

LC-MS: t_{R} = 1.09 min; [M+H]⁺ = 492.08.

### Example 115: (S)-Benzo[d]isoxazole-3-carboxylic acid [1-(2-cyclopropyl-5-m-tolyl-thiazole-4-carbonyl)-piperidin-2-ylmethyl]-amide:

LC-MS: t_{R} = 1.16 min; [M+H]⁺ = 501.00.

### Example 116: (S)-Benzo[d]isoxazole-3-carboxylic acid [1-(2-cyclopropyl-5-phenyl-thiazole-4-carbonyl)-piperidin-2-ylmethyl]-amide:

LC-MS: t_{R} = 1.06 min; [M+H]⁺ = 487.23.

### Example 117: (S)-3,4-Dichloro-N-[1-(2-cyclopropyl-5-phenyl-thiazole-4-carbonyl)-piperidin-2-ylmethyl]-benzamide:

LC-MS: t_{R} = 1.11 min; [M+H]⁺ = 516.13.

### Example 118: (S)-3-Chloro-N-[1-(2-cyclopropyl-5-m-tolyl-thiazole-4-carbonyl)-piperidin-2-ylmethyl]-2-methyl-benzamide:

LC-MS: t_{R} = 1.13 min; [M+H]⁺ = 508.02.

### Example 119: (S)-2-Chloro-N-[1-(2-cyclopropyl-5-m-tolyl-thiazole-4-carbonyl)-piperidin-2-ylmethyl]-3-fluoro-benzamide:

LC-MS: t_{R} = 1.09 min; [M+H]⁺ = 512.24.

### Example 120: (S)-N-[1-(2-Cyclopropyl-5-phenyl-thiazole-4-carbonyl)-piperidin-2-ylmethyl]-3-methoxy-benzamide:

LC-MS: t_{R} = 1.04 min; [M+H]⁺ = 476.33.

### Example 121: (S)-Chroman-8-carboxylic acid [1-(2-cyclopropyl-5-m-tolyl-thiazole-4-carbonyl)-piperidin-2-ylmethyl]-amide:

LC-MS: t_{R} = 1.19 min; [M+H]⁺ = 515.93.

### Example 122: (S)-1H-Indazole-3-carboxylic acid [1-(2-cyclopropyl-5-m-tolyl-thiazole-4-carbonyl)-piperidin-2-ylmethyl]-amide:

LC-MS: t_{R} = 1.10 min; [M+H]⁺ = 500.00.

### Example 123: (S)-N-[1-(2-Cyclopropyl-5-phenyl-thiazole-4-carbonyl)-piperidin-2-ylmethyl]-3,5-dimethoxy-benzamide:

LC-MS: t_{R} = 1.05 min; [M+H]⁺ = 506.30.

### Example 124: (S)-Isoquinoline-1-carboxylic acid [1-(2-cyclopropyl-5-m-tolyl-thiazole-4-carbonyl)-piperidin-2-ylmethyl]-amide:

LC-MS: t_{R} = 1.18 min; [M+H]⁺ = 510.90.

### Example 125: (S)-2,3-Dihydro-benzo[1,4]dioxine-5-carboxylic acid [1-(2-cyclopropyl-5-phenyl-thiazole-4-carbonyl)-piperidin-2-ylmethyl]-amide:

LC-MS: t_{R} = 1.04 min; [M+H]⁺ = 504.28.

### Example 126: (S)-N-[1-(2-Cyclopropyl-5-phenyl-thiazole-4-carbonyl)-piperidin-2-ylmethyl]-benzamide:

LC-MS: t_{R} = 1.03 min; [M+H]⁺ = 446.31.

### Example 127: (S)-N-[1-(2-Cyclopropyl-5-phenyl-thiazole-4-carbonyl)-piperidin-2-ylmethyl]-2,5-dimethoxy-benzamide:

LC-MS: t_{R} = 1.05 min; [M+H]⁺ = 506.29.

### Example 128: (S)-3-Methyl-isoxazole-5-carboxylic acid [1-(2-cyclopropyl-5-m-tolyl-thiazole-4-carbonyl)-piperidin-2-ylmethyl]-amide:

LC-MS: t_{R} = 1.06 min; [M+H]⁺ = 465.02.

### Example 129: (S)-1,2-Dimethyl-1H-indole-3-carboxylic acid [1-(2-cyclopropyl-5-m-tolyl-thiazole-4-carbonyl)-piperidin-2-ylmethyl]-amide:

LC-MS: t_{R} = 1.15 min; [M+H]⁺ = 526.99.

### Example 130: (S)-3-Acetylamino-N-[1-(2-cyclopropyl-5-m-tolyl-thiazole-4-carbonyl)-piperidin-2-ylmethyl]-benzamide:

LC-MS: t_{R} = 0.99 min; [M+H]⁺ = 517.07.

### Example 131: (S)-4-Methyl-4H-furo[3,2-b]pyrrole-5-carboxylic acid [1-(2-cyclopropyl-5-phenyl-thiazole-4-carbonyl)-piperidin-2-ylmethyl]-amide:

LC-MS: t_{R} = 1.06 min; [M+H]⁺ = 489.29.

### Example 132: (S)-3-Methyl-1H-indene-2-carboxylic acid [1-(2-cyclopropyl-5-m-tolyl-thiazole-4-carbonyl)-piperidin-2-ylmethyl]-amide:

LC-MS: t_{R} = 1.21 min; [M+H]⁺ = 512.46.

### Example 133: (S)-1-Methyl-1H-indole-4-carboxylic acid [1-(2-cyclopropyl-5-phenyl-thiazole-4-carbonyl)-piperidin-2-ylmethyl]-amide:

LC-MS: t_{R} = 1.05 min; [M+H]⁺ = 499.29.

### Example 134: (S)-Imidazo[1,2-a]pyridine-3-carboxylic acid [1-(2-cyclopropyl-5-m-tolyl-thiazole-4-carbonyl)-piperidin-2-ylmethyl]-amide:

LC-MS: t_{R} = 0.90 min; [M+H]⁺ = 499.99.

### Example 135: (S)-Naphthalene-1-carboxylic acid [1-(2-cyclopropyl-5-phenyl-thiazole-4-carbonyl)-piperidin-2-ylmethyl]-amide:

LC-MS: t_{R} = 1.09 min; [M+H]⁺ = 496.29.

### Example 136: (S)-5-Fluoro-1-methyl-1H-indole-2-carboxylic acid [1-(2-cyclopropyl-5-m-tolyl-thiazole-4-carbonyl)-piperidin-2-ylmethyl]-amide:

LC-MS: t_{R} = 1.22 min; [M+H]⁺ = 530.96.

### Example 137: (S)-2,3-Dihydro-benzo[1,4]dioxine-6-carboxylic acid [1-(2-cyclopropyl-5-m-tolyl-thiazole-4-carbonyl)-piperidin-2-ylmethyl]-amide:

LC-MS: t_{R} = 1.10 min; [M+H]⁺ = 518.00.

### Example 138: (S)-1-Methyl-1H-indole-5-carboxylic acid [1-(2-cyclopropyl-5-phenyl-thiazole-4-carbonyl)-piperidin-2-ylmethyl]-amide:

LC-MS: t_{R} = 1.05 min; [M+H]⁺ = 499.29.

### Example 139: (S)-N-[1-(2-Cyclopropyl-5-m-tolyl-thiazole-4-carbonyl)-piperidin-2-ylmethyl]-4-methyl-nicotinamide:

LC-MS: t_{R} = 0.89 min; [M+H]⁺ = 475.05.

### Example 140: (S)-2-Bromo-N-[1-(2-cyclopropyl-5-m-tolyl-thiazole-4-carbonyl)-piperidin-2-ylmethyl]-3-methyl-benzamide:

LC-MS: t_{R} = 1.11 min; [M+H]⁺ = 551.90.

### Example 141: (S)-N-[1-(2-Cyclopropyl-5-phenyl-thiazole-4-carbonyl)-piperidin-2-ylmethyl]-3,4-dimethoxy-benzamide:

LC-MS: t_{R} = 1.00 min; [M+H]⁺ = 506.29.

### Example 142: (S)-N-[1-(2-Cyclopropyl-5-m-tolyl-thiazole-4-carbonyl)-piperidin-2-ylmethyl]-2,3-dimethyl-benzamide:

LC-MS: t_{R} = 1.10 min; [M+H]⁺ = 488.11.

### Example 143: (S)-Thieno[2,3-b]pyrazine-6-carboxylic acid {1-[5-(3-chloro-phenyl)-2-cyclopropyl-thiazole-4-carbonyl]-piperidin-2-ylmethyl}-amide:

LC-MS: t_{R} = 1.04 min; [M+H]⁺ = 538.01.

**Example 144: (S)-1-Methyl-1H-indole-4-carboxylic acid {1-[2-cyclopropyl-5-(3-trifluoromethyl-phenyl)-thiazole-4-carbonyl]-piperidin-2-ylmethyl}-amide:**

LC-MS: t_{R} = 1.11 min; [M+H]⁺ = 567.17.

### Example 145: (S)-2-Chloro-N-[1-(2-cyclopropyl-5-m-tolyl-thiazole-4-carbonyl)-piperidin-2-ylmethyl]-3-methyl-benzamide:

LC-MS: t_{R} = 1.10 min; [M+H]⁺ = 508.00.

### II. Biological assays

### In vitro assay

The orexin receptor antagonistic activity of the compounds of formula (I) is determined in accordance with the following experimental method.

### Experimental method:

### Intracellular calcium measurements:

Chinese hamster ovary (CHO) cells expressing the human orexin-1 receptor and the human orexin-2 receptor, respectively, are grown in culture medium (Ham F-12 with L-Glutamine) containing 300 µg/ml G418, 100 U/ml penicillin, 100 µg/ml streptomycin and 10 % heat inactivated fetal calf serum (FCS). The cells are seeded at 20'000 cells / well into 384-well black clear bottom sterile plates (Greiner). The seeded plates are incubated overnight at 37°C in 5% CO₂.

Human orexin-A as an agonist is prepared as 1 mM stock solution in MeOH: water (1:1), diluted in HBSS containing 0.1 % bovine serum albumin (BSA), NaHCO₃: 0.375g/l and 20 mM HEPES for use in the assay at a final concentration of 3 nM.

Antagonists are prepared as 10 mM stock solution in DMSO, then diluted in 384-well plates using DMSO followed by a transfer of the dilutions into in HBSS containing 0.1 % bovine serum albumin (BSA), NaHCO₃: 0.375g/l and 20 mM HEPES. On the day of the assay, 50 µl of staining buffer (HBSS containing 1% FCS, 20 mM HEPES, NaHCO₃: 0.375g/l, 5 mM probenecid (Sigma) and 3 µM of the fluorescent calcium indicator fluo-4 AM (1 mM stock solution in DMSO, containing 10% pluronic) is added to each well. The 384-well cell-plates are incubated for 50 min at 37° C in 5% CO₂ followed by equilibration at rt for 30 - 120 min before measurement.

Within the Fluorescent Imaging Plate Reader (FLIPR2 or FLIPR Tetra, Molecular Devices), antagonists are added to the plate in a volume of 10 µl/well, incubated for 10 min and finally 10 µl/well of agonist is added. Fluorescence is measured for each well at 1 second intervals, and the height of each fluorescence peak is compared to the height of the fluorescence peak induced by 3 nM orexin-A with vehicle in place of antagonist. For each antagonist, the IC₅₀ value (the concentration of compound needed to inhibit 50 % of the agonistic response) is determined. The calculated IC₅₀ values of the compounds may fluctuate depending on the daily cellular assay performance. Fluctuations of this kind are known to those skilled in the art.

Antagonistic activities (IC₅₀ values) of compounds of examples 1 to 95 (measured on FLIPR2) are in the range of 1.5-1208 nM with an average of 76 nM with respect to the OX1 receptor and in the range of 1.4-1367 nM with an average of 53 nM with respect to the OX2 receptor. Antagonistic activities (IC₅₀ values) of compounds of examples 96 to 145 (measured on FLIPR Tetra) are in the range of 3.6-820 nM with an average of 103 nM with respect to the OX1 receptor and in the range of 4.5-1743 nM with an average of 136 nM with respect to the OX2 receptor. Antagonistic activities of selected compounds are displayed in *Table 1.*

**Table 1**

| **Compound of** | **OX₁ IC₅₀ (nM)** | **OX₂ IC₅₀ (nM)** |
|---|---|---|
| Example 1 | 19 | 16 |
| Example 5 | 5.1 | 7.7 |
| Example 10 | 1.8 | 3.0 |
| Example 31 | 2.2¹ | 8.6¹ |
| Example 51 | 25 | 15 |
| Example 68 | 19 | 11 |
| Example 71 | 7.2 | 26 |
| Example 88 | 23 | 15 |
| Example 96 | 3.6 * | 4.5 * |
| Example 100 | 14 * | 20 * |
| Example 107 | 18 * | 22 * |
| Example 114 | 15 * | 19 * |
| Example 131 | 123 * | 17 * |

| | | |
|---|---|---|
| ¹ gemetric mean (n = 2); * IC₅₀ values measured on FLIPR Tetra | | |

## Claims

1. A compound of formula (1) 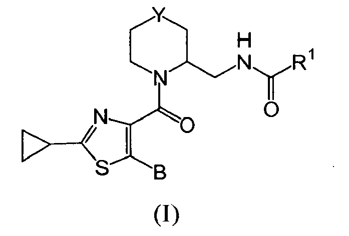 wherein
Y represents (CH₂)ₙ, wherein n represents 0 or 1;
B represents phenyl, wherein the phenyl ring is unsubstituted or mono-, di- or tri-substituted, wherein the substituents are independently selected from the group consisting of (C₁₋₄)alkyl, (C₁₋₄)alkoxy, trifluoromethyl, and halogen; and
R¹ represents aryl or heterocyclyl, wherein the aryl or heterocyclyl is independently unsubstituted or mono-, di-, or tri-substituted wherein the substituents are independently selected from the group consisting of (C₁₋₄)alkyl, (C₁₋₄)alkoxy, halogen, cyano, trifluoromethyl and -NH-CO-(C₁₋₄)alkyl;
or R¹ represents a 2,3-dihydro-benzofuranyl-, a benzo[1,3]dioxolyl-, a 2,3-dihydro-benzo[1,4]dioxinyl-, a 4*H* benzo[1,3]dioxinyl-, a 2*H*-chromenyl, a chromanyl-, a 2,3-dihydro-thieno[3,4-b][1,4]dioxinyl-, a 3,4-dihydro-2*H*-benzo[1,4]oxazinyl-, or an indenyl group; which groups are unsubstituted or mono- or di-substituted wherein the substituents are independently selected from the group consisting of (C₁₋₄)alkyl, (C₁₋₄)alkoxy and halogen;
or salts thereof.

2. A compound according to claim 1, wherein
Y represents (CH₂)ₙ, wherein n represents 0 or 1;
B represents phenyl, wherein the phenyl ring is unsubstituted or mono-, di- or tri-substituted, wherein the substituents are independently selected from the group consisting of (C₁₋₄)alkyl, (C₁₋₄)alkoxy, trifluoromethyl, and halogen; and
R¹ represents aryl or heterocyclyl, wherein the aryl or heterocyclyl is independently unsubstituted or mono-, di-, or tri-substituted wherein the substituents are independently selected from the group consisting of (C₁₋₄)alkyl, (C₁₋₄)alkoxy, halogen, cyano and trifluoromethyl;
or R¹ represents a 2,3-dihydro-benzofuranyl-, a benzo[1,3]dioxolyl-, a 2,3-dihydro-benzo[1,4]dioxinyl-, or a 4*H*-benzo[1,3]dioxinyl group which groups are unsubstituted or mono- or di-substituted wherein the substituents are independently selected from the group consisting of (C₁₋₄)alkyl, (C₁₋₄)alkoxy and halogen;
or a salt thereof.

3. A compound of formula (I) according to claims 1 or 2, which is also a compound of formula (Ia), wherein the indicated stereogenic center is in the (S)-configuration 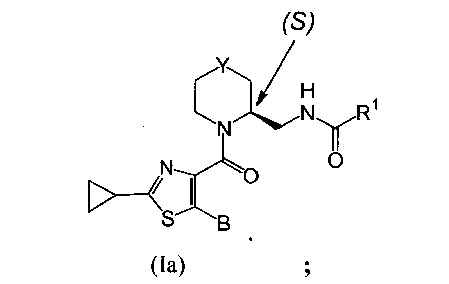 or a salt thereof.

4. A compound according to any one of claims 1 to 3, wherein B represents phenyl, wherein the phenyl ring is unsubstituted or mono- or di-substituted, wherein the substituents are independently selected from the group consisting of (C₁₋₄)alkyl, (C₁₋₄)alkoxy, trifluoromethyl, and halogen;
or a salt thereof.

5. A compound according to any one of claims 1 to 4, wherein B represents phenyl, wherein the phenyl ring is unsubstituted or mono-substituted, wherein the substituent is selected from the group consisting of methyl, chloro, fluoro, and trifluoromethyl;
or a salt thereof.

6. A compound according to any one of claims 1 to 5 wherein
R¹ represents phenyl which is mono-substituted, wherein the substituent is selected from chloro, bromo and methyl;
or R¹ represents heterocyclyl, wherein the heterocyclyl is selected from the group consisting of indolyl, benzofuranyl, indazolyl, benzisoxazolyl, quinolinyl and imidazo[2,1-b]thiazolyl, wherein said heterocyclyl is unsubstituted or mono-substituted wherein the substituent is selected from(C₁₋₄)alkyl;
or R¹ represents a 2,3-dihydro-benzofuranyl-, or a 2,3-dihydro-benzo[1,4]dioxinyl-group, which groups are unsubstituted;
or a salt thereof.

7. A compound according to any one of claims 1 to 6, or a salt thereof, wherein
R¹ represents one of the following groups: 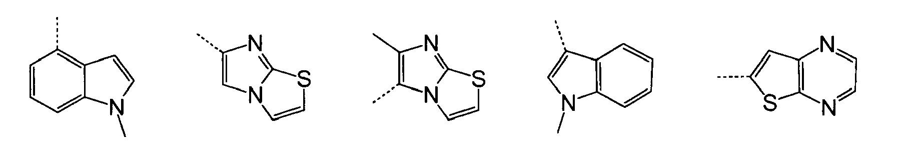 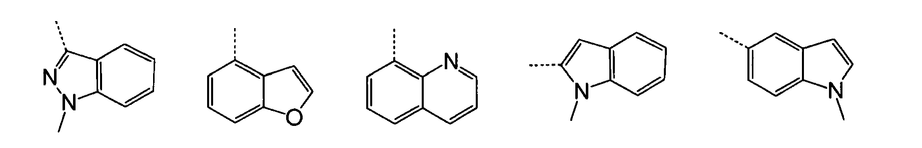 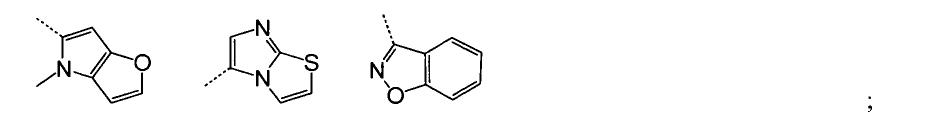 or a salt thereof.

8. A compound according to any one of claims 1 to 6, or a salt thereof, wherein
R¹ represents one of the following groups: 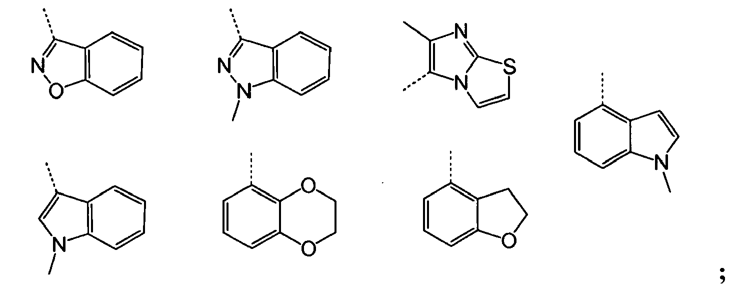 or a salt thereof.

9. A compound according to claim 1 selected from the group consisting of:
(S)-4-Methyl-4H-furo[3,2-b]pyrrole-5-carboxylic acid {1-[2-cyclopropyl-5-(2-fluorophenyl)-thiazole-4-carbonyl]-piperidin-2-ylmethyl}-amid;
(S)-Thieno[2,3-b]pyrazine-6-carboxylic acid {1-[2-cyclopropyl-5-(2-fluoro-phenyl)-thiazole-4-carbonyl]-piperidin-2-ylmethyl}-amid;
(S)-3-Bromo-N-{1-[2-cyclopropyl-5-(4-fluoro-phenyl)-thiazole-4-carbonyl]-piperidin-2-ylmethyl}-benzamide;
(S)-Naphthalene-1-carboxylic acid {1-[2-cyclopropyl-5-(4-fluoro-phenyl)-thiazole-4-carbonyl]-piperidin-2-ylmethyl}-amide;
(S)-Quinoline-8-carboxylic acid {1-[2-cyclopropyl-5-(4-fluoro-phenyl)-thiazole-4-carbonyl]-piperidin-2-ylmethyl}-amide;
(S)-2,3-Dihydro-benzo[1,4]dioxine-5-carboxylic acid {1-[2-cyclopropyl-5-(4-fluorophenyl)-thiazole-4-carbonyl]-piperidin-2-ylmethyl}-amide;
(S)-Benzofuran-4-carboxylic acid {1-[2-cyclopropyl-5-(4-fluoro-phenyl)-thiazole-4-carbonyl]-piperidin-2-ylmethyl}-amide;
(S)-4-Methyl-4H-furo[3,2-b]pyrrole-5-carboxylic acid {1-[2-cyclopropyl-5-(4-fluorophenyl)-thiazole-4-carbonyl]-piperidin-2-ylmethyl} -amide;
(S)-Imidazo[2,1-b]thiazole-5-carboxylic acid [1-(2-cyclopropyl-5-p-tolyl-thiazole-4-carbonyl)-piperidin-2-ylmethyl]-amide;
(S)-Imidazo[2,1-b]thiazole-6-carboxylic acid [1-(2-cyclopropyl-5-p-tolyl-thiazole-4-carbonyl)-piperidin-2-ylmethyl]-amide;
(S)-1-Methyl-1H-indole-2-carboxylic acid [1-(2-cyclopropyl-5-p-tolyl-thiazole-4-carbonyl)-piperidin-2-ylmethyl]-amide;
(S)-Naphthalene-1-carboxylic acid [1-(2-cyclopropyl-5-m-tolyl-thiazole-4-carbonyl)-piperidin-2-ylmethyl]-amide;
(S)-Quinoline-8-carboxylic acid [1-(2-cyclopropyl-5-m-tolyl-thiazole-4-carbonyl)-piperidin-2-ylmethyl]-amide;
(S)-2,3-Dihydro-benzo[1,4]dioxine-5-carboxylic acid [1-(2-cyclopropyl-5-m-tolyl-thiazole-4-carbonyl)-piperidin-2-ylmethyl]-amide;
(S)-Benzofuran-4-carboxylic acid [1-(2-cyclopropyl-5-m-tolyl-thiazole-4-carbonyl)-piperidin-2-ylmethyl]-amide;
(S)-1-Methyl-1H-indazole-3-carboxylic acid [1-(2-cyclopropyl-5-m-tolyl-thiazole-4-carbonyl)-piperidin-2-ylmethyl]-amide;
(S)-6-Methyl-imidazo[2,1-b]thiazole-5-carboxylic acid [1-(2-cyclopropyl-5-m-tolyl-thiazole-4-carbonyl)-piperidin-2-ylmethyl]-amide;
(S)-Imidazo[2,1-b]thiazole-5-carboxylic acid [1-(2-cyclopropyl-5-m-tolyl-thiazole-4-carbonyl)-piperidin-2-ylmethyl]-amide;
(S)-1-Methyl-1H-indole-4-carboxylic acid [1-(2-cyclopropyl-5-m-tolyl-thiazole-4-carbonyl)-piperidin-2-ylmethyl]-amide;
(S)-3-Chloro-N-{1-[5-(3-chloro-phenyl)-2-cyclopropyl-thiazole-4-carbonyl]-piperidin-2-ylmethyl}-benzamide:
(S)-Quinoline-8-carboxylic acid {1-[5-(3-chloro-phenyl)-2-cyclopropyl-thiazole-4-carbonyl]-piperidin-2-ylmethyl}-amide;
(S)-Benzofuran-4-carboxylic acid {1-[5-(3-chloro-phenyl)-2-cyclopropyl-thiazole-4-carbonyl]-piperidin-2-ylmethyl}-amide;
(S)-1-Methyl-1H-indole-3-carboxylic acid {1-[5-(3-chloro-phenyl)-2-cyclopropyl-thiazole-4-carbonyl]-piperidin-2-ylmethyl}-amide;
(S)-Imidazo[2,1-b]thiazole-5-carboxylic acid {1-[5-(3-chloro-phenyl)-2-cyclopropyl-thiazole-4-carbonyl]-piperidin-2-ylmethyl}-amide;
(S)-4-Methyl-4H-furo[3,2-b]pyrrole-5-carboxylic acid {1-[5-(3-chloro-phenyl)-2-cyclopropyl-thiazole-4-carbonyl]-piperidin-2-ylmethyl}-amide;
(S)-1-Methyl-1H-indole-4-carboxylic acid {1-[5-(3-chloro-phenyl)-2-cyclopropyl-thiazole-4-carbonyl]-piperidin-2-ylmethyl}-amide;
(S)-Benzo[d]isoxazole-3-carboxylie acid {1-[5-(3-chloro-phenyl)-2-cyclopropyl-thiazole-4-carbonyl]-piperidin-2-ylmethyl}-amide;
(S)-N-{1-1-[2-Cyclopropyl-5-(3-trifluoromethyl-phenyl)-thiazole-4-carbonyl]-piperidin-2-ylmethyl}-3-methyl-benzamide;
(S)-Quinoline-8-carboxylic acid {1-[2-cyclopropyl-5-(3-trifluoromethyl-phenyl)-thiazole-4-carbonyl]-piperidin-2-ylmethyl}-amide;
(S)-2,3-Dihydro-benzo[1,4]dioxine-5-carboxylic acid {1-[2-cyclopropyl-5-(3-trifluoromethyl-phenyl)-thiazole-4-carbonyl]-piperidin-2-ylmethyl -amide;
(S)-Benzofuran-4-carboxylic acid {1-[2-cyclopropyl-5-(3-trifluoromethyl-phenyl)-thiazole-4-carbonyl]-piperidin-2-ylmethyl}-amide;
(S)-Imidazo[2,1-b]thiazole-5-carboxylic acid {1-[2-cyclopropyl-5-(3-trifluoromethylphenyl)-thiazole-4-carbonyl]-piperidin-2-ylmethyl}-amide;
(S)-3-Bromo-N-{1-[2-cyclopropyl-5-(4-fluoro-phenyl)-thiazole-4-carbonyl]-pyrrolidin-2-ylmethyl}-benzamide;
(S)-Quinoline-8-carboxylic acid {1-[2-cyclopropyl-5-(4-fluoro-phenyl)-thiazole-4-carbonyl]-pyrrolidin-2-ylmethyl}-amide;
(S)-2,3-Dihydro-benzo[1,4]dioxine-5-carboxylic acid {1-[2-cyclopropyl-5-(4-fluorophenyl)-thiazole-4-carbonyl]-pyrrolidin-2-ylmethyl}-amide;
(S)-Benzofuran-4-carboxylic acid {1-[2-cyclopropyl-5-(4-fluoro-phenyl)-thiazole-4-carbonyl]-pyrrolidin-2-ylmethyl -amide;
(S)-1-Methyl-1H-indole-3-carboxylic acid {1-[2-cyclopropyl-5-(4-fluoro-phenyl)-thiazole-4-carbonyl]-pyrrolidin-2-ylmethyl}-amide;
(S)-1-Methyl-1H-indazole-3-carboxylic acid {1-[2-cyclopropyl-5-(4-fluoro-phenyl)-thiazole-4-carbonyl]-pyrrolidin-2-ylmethyl}-amide;
(S)-Benzo[d]isoxazole-3-carboxylic acid {1-[2-cyclopropyl-5-(4-fluoro-phenyl)-thiazole-4-carbonyl]-pyrrolidin-2-ylmethyl}-amide;
(S)-N- {1-[2-Cyclopropyl-5-(2-fluoro-phenyl)-thiazole-4-carbonyl]-piperidin-2-ylmethyl}-3-methyl-benzamide;
(S)-3-Chloro-N-{1-[2-cyclopropyl-5-(2-fluoro-phenyl)-thiazole-4-carbonyl]-piperidin-2-ylmethyl}-benzamide;
(S)-3-Bromo-N-{1-[2-cyclopropyl-5-(2-fluoro-phenyl)-thiazole-4-carbonyl]-piperidin-2-ylmethyl}--benzamide;
(S)-Naphthalene-1-carboxylic acid {1-[2-cyclopropyl-5-(2-fluoro-phenyl)-thiazole-4-carbonyl]-piperidin-2-ylmethyl}-amide;
(S)-2,3-Dihydro-benzo[1,4]dioxine-5-carboxylic acid {1-[2-cyclopropyl-5-(2-fluorophenyl)-thiazole-4-carbonyl]-piperidin-2-ylmethyl}-amide;
(S)-1-Methyl-1H-indole-3-carboxylic acid {1-[2-cyclopropyl-5-(2-fluoro-phenyl)-thiazole-4-carbonyl]-piperidin-2-ylmethyl}-amide;
(S)-1-Methyl-1H-indole-6-carboxylic acid {1-[2-cyclopropyl-5-(2-fluoro-phenyl)-thiazole-4-carbonyl]-piperidin-2-ylmethyl}-amide;
(S)-1-Methyl-1H-indole-4-carboxylic acid {1-[2-cyclopropyl-5-(2-fluoro-phenyl)-thiazole-4-carbonyl]-piperidin-2-ylmethyl}-amide;
(S)-N-{1-[2-Cyclopropyl-5-(4-fluoro-phenyl)-thiazole-4-carbonyl]-piperidin-2-ylmethyl}-3-methyl-benzamide;
(S)-3-Chloro-N-{1-[2-cyclopropyl-5-(4-fluoro-phenyl)-thiazole-4-carbonyl]-piperidin-2-ylmethyl}-benzamide;
(S)-1-Methyl-1H-indole-3-carboxylic acid {1-[2-cyclopropyl-5-(4-fluoro-phenyl)-thiazole-4-carbonyl]-piperidin-2-ylmethyl}-amide;
(S)-1-Methyl-1H-indazole-3-carboxylic acid {1-[2-cyclopropyl-5-(4-fluoro-phenyl)-thiazole-4-carbonyl]-piperidin-2-ylmethyl}-amide;
(S)-2,3-Dihydro-benzo[1,4]dioxine-5-carboxylic acid [1-(2-cyclopropyl-5-p-tolyl-thiazole-4-carbonyl)-piperidin-2-ylmethyl]-amide;
(S)-N-[1-(2-Cyclopropyl-5-p-tolyl-thiazole-4-carbonyl)-piperidin-2-ylmethyl]-3-methyl-benzamide;
(S)-3-Bromo-N-[1-(2-cyclopropyl-5-m-tolyl-thiazole-4-carbonyl)-piperidin-2-ylmethyl]-benzamide;
(S)-4-Methyl-4H-furo[3,2-b]pyrrole-5-carboxylic acid [1-(2-cyclopropyl-5-m-tolyl-thiazole-4-carbonyl)-piperidin-2-ylmethyl]-amide;
(S)-N-{1-[5-(3-Chloro-phenyl)-2-cyclopropyl-thiazole-4-carbonyl]-piperidin-2-ylmethyl}-3-methyl-benzamide;
(S)-3-Bromo-N-{1-[5-(3-chloro-phenyl)-2-cyclopropyl-thiazole-4-carbonyl]-piperidin-2-ylmethyl}-benzamide;
(S)-2,3-Dihydro-benzo[1,4]dioxine-5-carboxylic acid {1-[5-(3-chloro-phenyl)-2-cyclopropyl-thiazole-4-carbonyl]-piperidin-2-ylmethyl}-amide;
(S)-1-Methyl-1H-indazote-3-carboxylie acid {1-[5-(3-chloro-phenyl)-2-cyclopropyl-thiazole-4-carbonyl]-piperidin-2-ylmethyl}-amide;
(S)-3-Chloro-N-{1-[2-cyclopropyl-5-(3-trifluoromethyl-phenyl)-thiazole-4-carbonyl]-piperidin-2-ylmethyl}-benzamide;
(S)-3-Bromo-N-{1-[2-cyclopropyl-5-(3-trifluoromethyl-phenyl)-thiazole-4-carbonyl]-piperidin-2-ylmethyl}-benzamide;
(S)-1-Methyl-1H-indole-3-carboxylic acid {1-[2-cyclopropyl-5-(3-trifluoromethylphenyl)-thiazole-4-carbonyl]-piperidin-2-ylmethyl}-amide;
(S)-1-Methyl-1H-indazole-3-carboxylic acid {1-[2-cyclopropyl-5-(3-trifluoromethylphenyl)-thiazole-4-carbonyl]-piperidin-2-ylmethyl}-amide;
(S)-6-Methyl-imidazo[2,1-b]thiazole-5-carboxylic acid {1-[2-cyclopropyl-5-(4-fluorophenyl)-thiazole-4-carbonyl]-pyrrolidin-2-ylmethyl}-amide;
(S)-Imidazo[2,1-b]thiazole-5-carboxylic acid {1-[2-cyclopropyl-5-(4-fluoro-phenyl)-thiazole-4-carbonyl]-pyrrolidin-2-ylmethyl}-amide;
(S)-Quinoline-8-carboxylic acid {1-[2-cyclopropyl-5-(2-fluoro-phenyl)-thiazole-4-carbonyl]-piperidin-2-ylmethyl}-amide;
(S)-Benzofuran-4-carboxylic acid {1-[2-cyclopropyl-5-(2-fluoro-phenyl)-thiazole-4-carbonyl]-piperidin-2-ylmethyl}-amide;
(S)-1-Methyl-1H-indazole-3-carboxylic acid {1-[2-cyclopropyl-5-(2-fluoro-phenyl)-thiazole-4-carbonyl]-piperidin-2-ylmethyl}-amide;
(S)-6-Methyl-imidazo[2,1-b]thiazole-5-carboxylic acid {1-[2-cyclopropyl-5-(2-fluorophenyl)-thiazole-4-carbonyl]-piperidin-2-ylmethyl}-amide;
(S)-Benzo[d]isoxazole-3-carboxylic acid {1-[2-cyclopropyl-5-(2-fluoro-phenyl)-thiazole-4-carbonyl]-piperidin-2-ylmethyl}-amide;
(S)-6-Methyl-imidazo[2,1-b]thiazole-5-carboxylic acid {1-[2-cyclopropyl-5-(4-fluorophenyl)-thiazole-4-carbonyl]-piperidin-2-ylmethyl}-amide;
(S)-6-Methyl-imidazo[2,1-b]thiazole-5-carboxylic acid [1-(2-cyclopropyl-5-p-tolyl-thiazole-4-carbonyl)-piperidin-2-ylmethyl]-amide;
(S)-1-Methyl-1H-indazole-3-carboxylic acid [1-(2-cyclopropyl-5-p-tolyl-thiazole-4-carbonyl)-piperidin-2-ylmethyl]-amide;
(S)-3-Bromo-N-[1-(2-cyclopropyl-5-p-tolyl-thiazole-4-carbonyl)-piperidin-2-ylmethyl]-benzamide;
(S)-3-Chloro-N-[1-(2-cyclopropyl-5-p-tolyl-thiazole-4-carbonyl)-piperidin-2-ylmethyl]-benzamide
(S)-N-[1-(2-Cyclopropyl-5-p-tolyl-thiazole-4-carbonyl)-piperidin-2-ylmethyl]-3-methyl-benzamide;
(S)-3-Chloro-N-[1-(2-cyclopropyl-5-m-tolyl-thiazole-4-carbonyl)-piperidin-2-ylmethyl]-benzamide;
(S)-1-Methyl-1H-indole-3-carboxylic acid [1-(2-cyclopropyl-5-m-tolyl-thiazole-4-carbonyl)-piperidin-2-ylmethyl]-amide;
(S)-6-Methyl-imidazo[2,1-b]thiazole-5-carboxylic acid {1-[5-(3-chloro-phenyl)-2-cyclopropyl-thiazole-4-carbonyl]-piperidin-2-ylmethyl}-amide;
(S)-6-Methyl-imidazo[2,1-b]thiazole-5-carboxylic acid {1-[2-cyclopropyl-5-(3-trifluoromethyl-phenyl)-thiazole-4-carbonyl]-piperidin-2-ylmethyl}-amide;
(S)-Thieno[2,3-b]pyrazine-6-carboxylic acid {1-[2-cyclopropyl-5-(4-fluoro-phenyl)-thiazole-4-carbonyl]-piperidin-2-ylmethyl -amide;
(S)-1-Methyl-1H-indole-6-carboxylic acid {1-[2-cyclopropyl-5-(4-fluoro-phenyl)-thiazole-4-carbonyl]-piperidin-2-ylmethyl}-amide;
(S)-1-Methyl-1H-indole-4-carboxylic acid {1-[2-cyclopropyl-5-(4-fluoro-phenyl)-thiazole-4-carbonyl]-piperidin-2-ylmethyl}-amide;
(S)-1-Methyl-1H-indole-5-carboxylic acid [1-(2-cyclopropyl-5-m-tolyl-thiazole-4-carbonyl)-piperidin-2-ylmethyl]-amide;
(S)-N-{1-[S-(3-Chloro-phenyl)-2-cyclopropyl-thiazole-4-carbonyl]-piperidin-2-ylmethyl}-3-methyl-benzamide;
(S)-Naphthalene-1-carboxylic acid {1-[5-(3-chloro-phenyl)-2-cyclopropyl-thiazole-4-carbonyl]-piperidin-2-ylmethyl}-amide;
(S)-4-Methyl-4H-furo[3,2-b]pyrrole-5-carboxylic acid {1-[5-(3-chloro-phenyl)-2-cyclopropyl-thiazole-4-carbonyl]-piperidin-2-ylmethyl}-amide;
(S)-1-Methyl-1H-indole-5-carboxylic acid {1-[5-(3-chloro-phenyl)-2-cyclopropyl-thiazole-4-carbonyl]-piperidin-2-ylmethyl}-amide;
(S)-Naphthalene-1-carboxylic acid {1-[2-cyclopropyl-5-(3-trifluoromethyl-phenyl)-thiazole-4-carbonyl]-piperidin-2-ylmethyl}-amide;
(S)-Benzo[d]isoxazole-3-carboxylic acid {1-[2-cyclopropyl-5-(3-trifluoromethylphenyl)-thiazole-4-carbonyl]-piperidin-2-ylmethyl}-amide;
(S)-3-Chloro-N-{1-[2-cyclopropyl-5-(4-fluoro-phenyl)-thiazole-4-carbonyl]-pyrrolidin-2-ylmethyl}-benzamide;
(S)-Naphthalene-1-carboxylic acid {1-[2-cyclopropyl-5-(4-fluoro-phenyl)-thiazole-4-carbonyl]-pyrrolidin-2-ylmethyl}-amide;
(S)-Imidazo[2,1-b]thiazole-5-carboxylic acid {1-[2-cyclopropyl-5-(4-fluoro-phenyl)-thiazole-4-carbonyl]-pyrrolidin-2-ylmethyl}-amide;
(S)-4-Methyl-4H-furo[3,2-b]pyrrole-5-carboxylic acid {1-[2-cyclopropyl-5-(4-fluorophenyl)-thiazole-4-carbonyl]-pyrrolidin-2-ylmethyl}-amide;
(S)-1-Methyl-1H-indole-4-carboxylic acid {1-[2-cyclopropyl-5-(4-fluoro-phenyl)-thiazole-4-carbonyl]-pyrrolidin-2-ylmethyl}-amide;
(S)-Imidazo[2,1-b]thiazole-5-carboxylic acid [1-(2-cyclopropyl-5-phenyl-thiazole-4-carbonyl)-piperidin-2-ylmethyl]-amide;
(S)-6-Methyl-imidazo[2,1-b]thiazole-5-carboxylic acid [1-(2-cyclopropyl-5-phenyl-thiazole-4-carbonyl)-piperidin-2-ylmethyl]-amide;
(S)-2,3-Dihydro-benzofuran-4-carboxylic acid [1-(2-cyclopropyl-5-phenyl-thiazole-4-carbonyl)-piperidin-2-ylmethyl]-amide;
(S)-Benzofuran-4-carboxylic acid [1-(2-cyclopropyl-5-phenyl-thiazole-4-carbonyl)-piperidin-2-ylmethyl]-amide;
(S)-1-Methyl-1H-indazole-3-carboxylic acid [1-(2-cyclopropyl-5-phenyl-thiazole-4-carbonyl)-piperidin-2-ylmethyl]-amide;
(S)-Quinoline-8-carboxylic acid [1-(2-cyclopropyl-5-phenyl-thiazole-4-carbonyl)-piperidin-2-ylmethyl]-amide;
(S)-N-[1-(2-Cyclopropyl-5-phenyl-thiazole-4-carbonyl)-piperidin-2-ylmethyl]-3,4-dimethyl-benzamide;
(S)-N-[1-(2-Cyclopropyl-5-phenyl-thiazole-4-carbonyl)-piperidin-2-ylmethyl]-4-methyl-3-trifluoromethyl-benzamide;
(S)-3-Cyano-N-[1-(2-cyclopropyl-5-m-tolyl-thiazole-4-carbonyl)-piperidin-2-ylmethyl]-benzamide;
(S)-1-Methyl-1H-indole-3-carboxylic acid [1-(2-cyclopropyl-5-phenyl-thiazole-4-carbonyl)-piperidin-2-ylmethyl]-amide;
(S)-2,3-Dihydro-benzofuran-7-carboxylic acid [1-(2-cyclopropyl-5-m-tolyl-thiazole-4-carbonyl)-piperidin-2-ylmethyl]-amide;
(S)-2,3-Dihydro-benzofuran-4-carboxylic acid [1-(2-cyclopropyl-5-m-tolyl-thiazole-4-carbonyl)-piperidin-2-ylmethyl]-amide;
(S)-2-Ethyl-5-methyl-2H-pyrazole-3-carboxylic acid [1-(2-cyclopropyl-5-m-tolyl-thiazole-4-carbonyl)-piperidin-2-ylmethyl]-amide;
(S)-2,5-Dimethyl-2H-pyrazole-3-carboxylic acid [1-(2-cyclopropyl-5-m-tolyl-thiazole-4-carbonyl)-piperidin-2-ylmethyl]-amide;
(S)-3,4-Dihydro-2H-benzo[1,4]oxazine-5-carboxylic acid [1-(2-cyclopropyl-5-m-tolyl-thiazole-4-carbonyl)-piperidin-2-ylmethyl]-amide;
(S)-N-[1-(2-Cyclopropyl-5-m-tolyl-thiazole-4-carbonyl)-piperidin-2-ylmethyl]-3-methoxy-benzamide;
(S)-N-[1-(2-Cyclopropyl-5-m-tolyl-thiazole-4-carbonyl)-pipcridin-2-ylmethyl]-3-trifluoromethyl-benzamide;
(S)-2,3-Dihydro-thieno[3,4-b][1,4]dioxinc-5-carboxylic acid [1-(2-cyclopropyl-5-m-tolyl-thiazole-4-carbonyl)-piperidin-2-ylmethyl]-amide;
(S)-N-[1-(2-Cyclopropyl-5-m-tolyl-thiazole-4-carbonyl)-piperidin-2-ylmethyl]-3-fluoro-2-methyl-benzamide;
(S)-Benzo[d]isoxazole-3-carboxylic acid [1-(2-cyclopropyl-5-m-tolyl-thiazole-4-carbonyl)-piperidin-2-ylmethyl]-amide;
(S)-Benzo[d]isoxazole-3-carboxylic acid [1-(2-cyclopropyl-5-phenyl-thiazole-4-carbonyl)-piperidin-2-ylmethyl]-amide;
(S)-3,4-Dichloro-N-[1-(2-cyclopropyl-5-phenyl-thiazole-4-carbonyl)-piperidin-2-ylmethyl]-benzamide;
(S)-3-Chloro-N-[1-(2-cyclopropyl-5-m-tolyl-thiazole-4-carbonyl)-piperidin-2-ylmethyl]-2-methyl-benzamide;
(S)-2-Chloro-N-[1-(2-cyclopropyl-5-m-tolyl-thiazole-4-carbonyl)-piperidin-2-ylmethyl]-3-fluoro-benzamide;
(S)-N-[1-(2-Cyclopropyl-5-phenyl-thiazole-4-carbonyl)-piperidin-2-ylmethyl]-3-methoxy-benzamide;
(S)-Chroman-8-carboxylic acid [1-(2-cyclopropyl-5-m-tolyl-thiazole-4-carbonyl)-piperidin-2-ylmethyl]-amide;
(S)-1H-Indazole-3-carboxylic acid [1-(2-cyclopropyl-5-m-tolyl-thiazole-4-carbonyl)-piperidin-2-ylmethyl]-amide;
(S)-N-[1-(2-Cyclopropyl-5-phenyl-thiazole-4-carbonyl)-piperidin-2-ylmethyl]-3,5-dimethoxy-benzamide;
(S)-Isoquinoline-1-carboxylic acid [1-(2-cyclopropyl-5-m-tolyl-thiazole-4-carbonyl)-piperidin-2-ylmethyl]-amide;
(S)-2,3-Dihydro-benzo[1,4]dioxine-5-carboxylic acid [1-(2-cyclopropyl-5-phenyl-thiazole-4-carbonyl)-piperidin-2-ylmethyl]-amide;
(S)-N-[1-(2-Cyclopropyl-5-phenyl-thiazole-4-carbonyl)-piperidin-2-ylmethyl]-benzamide;
(S)-N-[1-(2-Cyclopropyl-5-phenyl-thiazole-4-carbonyl)-piperidin-2-ylmethyl]-2,5-dimethoxy-benzamide;
(S)-3-Methyl-isoxazole-5-carboxylic acid [1-(2-cyclopropyl-5-m-tolyl-thiazole-4-carbonyl)-piperidin-2-ylmethyl]-amide;
(S)-1,2-Dimethyl-1H-indole-3-carboxylic acid [1-(2-cyclopropyl-5-m-tolyl-thiazole-4-carbonyl)-piperidin-2-ylmethyl]-amide;
(S)-3-Acetylamino-N-[1-(2-cyclopropyl-5-m-tolyl-thiazole-4-carbonyl)-piperidin-2-ylmethyl]-benzamide;
(S)-4-Methyl-4H-furo[3,2-b]pyrrole-5-carboxylic acid [1-(2-cyclopropyl-5-phenyl-thiazole-4-carbonyl)-piperidin-2-ylmethyl]-amide;
(S)-3-Methyl-1H-indene-2-carboxylic acid [1-(2-cyclopropyl-5-m-tolyl-thiazole-4-carbonyl)-piperidin-2-ylmethyl]-amide;
(S)-1-Methyl-1H-indole-4-carboxylic acid [1-(2-cyclopropyl-5-phenyl-thiazole-4-carbonyl)-piperidin-2-ylmethyl]-amide;
(S)-Imidazo[1,2-a]pyridine-3-carboxylic acid [1-(2-cyclopropyl-5-m-tolyl-thiazole-4-carbonyl)-piperidin-2-ylmethyl]-amide;
(S)-Naphthalene-1-carboxylic acid [1-(2-cyclopropyl-5-phenyl-thiazole-4-carbonyl)-piperidin-2-ylmethyl]-amide;
(S)-5-Fluoro-1-methyl-1H-indole-2-carboxylic acid [1-(2-cyclopropyl-5-m-tolyl-thiazole-4-carbonyl)-piperidin-2-ylmethyl]-amide;
(S)-2,3-Dihydro-benzo[1,4]dioxine-6-carboxylic acid [1-(2-cyclopropyl-5-m-tolyl-thiazole-4-carbonyl)-piperidin-2-ylmethyl]-amide;
(S)-1-Methyl-1H-indole-5-carboxylic acid [1-(2-cyclopropyl-5-phenyl-thiazole-4-carbonyl)-piperidin-2-ylmethyl]-amide;
(S)-N-[1-(2-Cyclopropyl-5-m-tolyl-thiazole-4-carbonyl)-piperidin-2-ylmethyl]-4-methyl-nicotinamide;
(S)-2-Bromo-N-[1-(2-cyclopropyl-5-m-tolyl-thiazole-4-carbonyl)-piperidin-2-ylmethyl]-3-methyl-benzamide;
(S)-N-[1-(2-Cyclopropyl-5-phenyl-thiazole-4-carbonyl)-piperidin-2-ylmethyl]-3,4-dimethoxy-benzamide;
(S)-N-[1-(2-Cyclopropyl-5-m-tolyl-thiazole-4-carbonyl)-piperidin-2-ylmethyl]-2,3-dimethyl-benzamide;
(S)-Thieno[2,3-b]pyrazine-6-carboxylic acid {1-[5-(3-chloro-phenyl)-2-cyclopropyl-thiazole-4-carbonyl]-piperidin-2-ylmethyl}-amide;
(S)-1-Methyl-1H-indole-4-carboxylic acid {1-[2-cyclopropyl-5-(3-trifluoromethylphenyl)-thiazole-4-carbonyl]-piperidin-2-ylmethyl}-amide; and
(S)-2-Chloro-N-[1-(2-cyclopropyl-5-m-tolyl-thiazole-4-carbonyl)-piperidin-2-ylmethyl]-3-methyl-benzamide;
or a salt of such a compound.

10. A pharmaceutical composition comprising a compound of formula (I) according to any one of claims 1 to 9, or a pharmaceutically acceptable salt thereof, and a pharmaceutically acceptable carrier.

11. A compound according to any one of claims 1 to 9, or a pharmaceutically acceptable salt thereof, for use as a medicament.

12. A compound according to any of claims 1 to 9, or a pharmaceutically acceptable salt thereof, for the prevention or treatment of a disease selected from the group consisting of all types of sleep disorders, of stress-related syndromes, of psychoactive substance use and abuse, of cognitive dysfunctions in the healthy population and in psychiatric and neurologic disorders, of eating or drinking disorders.

13. Use of a compound according to any one of claims 1 to 9, or of a pharmaceutically acceptable salt thereof, for the preparation of a pharmaceutical composition for the treatment of diseases or disorders selected from the group consisting of sleep disorders that comprises all types of insomnias, narcolepsy and other disorders of excessive sleepiness, sleep-related dystonias, restless leg syndrome, sleep apneas, jet-lag syndrome, shift-work syndrome, delayed or advanced sleep phase syndrome and insomnias related to psychiatric disorders.

## Patentansprüche

1. Verbindung der Formel (I) 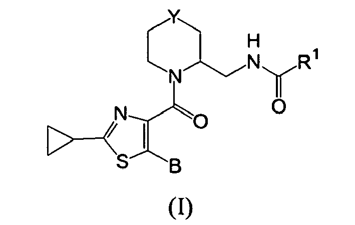 wobei
Y (CH₂)ₙ repräsentiert, wobei n 0 oder 1 repräsentiert;
B Phenyl repräsentiert, wobei der Phenylring unsubstituiert oder mono-, di- oder trisubstituiert ist, wobei die Substituenten unabhängig ausgewählt sind aus der Gruppe bestehend aus (C₁₋₄)-Alkyl, (C₁₋₄)-Alkoxy, Trifluormethyl und Halogen; und
R¹ Aryl oder Heterocyclyl repräsentiert, wobei das Aryl oder Heterocyclyl unabhängig unsubstituiert oder mono-, di- oder trisubstituiert ist, wobei die Substituenten unabhängig ausgewählt sind aus der Gruppe bestehend aus (C₁₋₄)-Alkyl, (C₁₋₄)-Alkoxy, Halogen, Cyano, Trifluormethyl und -NH-CO-(C₁₋₄)-Alkyl;
oder R¹ eine 2,3-Dihydro-benzofuranyl-, eine Benzo[1,3]dioxolyl-, eine 2,3-Dihydro-benzo[1,4]dioxinyl-, eine 4*H*-Benzo[1,3]dioxinyl-, eine 2*H*-Chromenyl, eine Chromanyl-, eine 2,3-Dihydro-thieno[3,4-b][1,4]dioxinyl-, eine 3,4-Dihydro-2*H*-benzo[1,4]oxazinyl- oder eine Indenyl-Gruppe repräsentiert; wobei die Gruppen unsubstituiert oder mono- oder disubstituiert sind, wobei die Substituenten unabhängig ausgewählt sind aus der Gruppe bestehend aus (C₁₋₄)-Alkyl, (C₁₋₄)-Alkoxy und Halogen;
oder Salze davon.

2. Verbindung nach Anspruch 1, wobei
Y (CH₂)ₙ repräsentiert, wobei n 0 oder 1 repräsentiert;
B Phenyl repräsentiert, wobei der Phenylring unsubstituiert oder mono-, di- oder trisubstitutiert ist, wobei die Substituenten unabhängig ausgewählt sind aus der Gruppe bestehend aus (C₁₋₄)-Alkyl, (C₁₋₄)-Alkoxy, Trifluormethyl und Halogen; und
R¹ Aryl oder Heterocyclyl repräsentiert, wobei das Aryl oder Heterocyclyl unabhängig unsubstituiert oder mono-, di oder trisubstituiert ist, wobei die Substituenten unabhängig ausgewählt sind aus der Gruppe bestehend aus (C₁₋₄)-Alkyl, (C₁₋₄)-Alkoxy, Halogen, Cyano und Trifluormethyl;
oder R¹ eine 2,3-Dihydro-benzofuranyl-, eine Benzo[1,3]dioxolyl-, eine 2,3-Dihydro-benzo[1,4]dioxinyl- oder eine 4*H*-Benzo[1,3]dioxinyl-Gruppe repräsentiert, wobei die Gruppen unsubstituiert oder mono- oder disubstituiert sind, wobei die Substituenten unabhängig ausgewählt sind aus der Gruppe bestehend aus (C₁₋₄)-Alkyl, (C₁₋₄)-Alkoxy und Halogen;
oder ein Salz davon.

3. Verbindung der Formel (I) nach Anspruch 1 oder 2, die auch eine Verbindung der Formel (Ia) ist, wobei das angegebene stereogene Zentrum in (S)-Konfiguration vorliegt 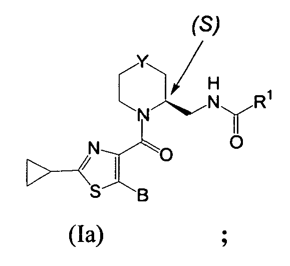 oder ein Salz davon.

4. Verbindung nach einem der Ansprüche 1 bis 3, wobei B Phenyl repräsentiert, wobei der Phenylring unsubstituiert oder mono- oder disubstituiert ist, wobei die Substituenten unabhängig ausgewählt sind aus der Gruppe bestehend aus (C₁₋₄)-Alkyl, (C₁₋₄)-Alkoxy, Trifluormethyl und Halogen;
oder ein Salz davon.

5. Verbindung nach einem der Ansprüche 1 bis 4, wobei B Phenyl repräsentiert, wobei der Phenylring unsubstituiert oder monosubstituiert ist, wobei der Substituent ausgewählt ist aus der Gruppe bestehend aus Methyl, Chlor, Fluor und Trifluormethyl;
oder ein Salz davon.

6. Verbindung nach einem der Ansprüche 1 bis 5, wobei
R¹ Phenyl repräsentiert, das monosubstituiert ist, wobei der Substituent ausgewählt ist aus Chlor, Brom und Methyl;
oder R¹ Heterocyclyl repräsentiert, wobei das Heterocyclyl ausgewählt ist aus der Gruppe bestehend aus Indolyl, Benzofuranyl, Indazolyl, Benzisoxazolyl, Chinolinyl und Imidazo[2,1-b]thiazolyl, wobei das genannte Heterocyclyl unsubstituiert oder monosubstituiert ist, wobei der Substituent ausgewählt ist aus (C₁₋₄)-Alkyl;
oder R¹ eine 2,3-Dihydro-benzofuranyl- oder eine 2,3-Dihydro-benzo[1,4]dioxinyl-Gruppe repräsentiert, wobei die Gruppen unsubstituiert sind;
oder ein Salz davon.

7. Verbindung nach einem der Ansprüche 1 bis 6 oder ein Salz davon, wobei R¹ eine der folgenden Gruppen repräsentiert: 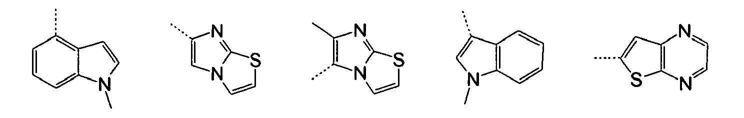 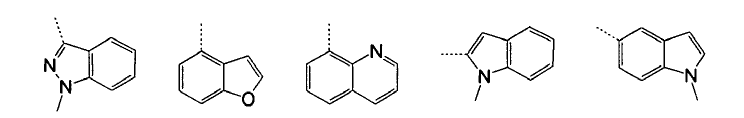 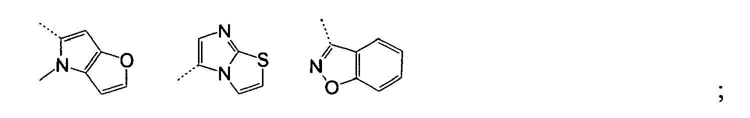 oder ein Salz davon.

8. Verbindung nach einem der Ansprüche 1 bis 6 oder ein Salz davon, wobei R¹ eine der folgenden Gruppen repräsentiert: 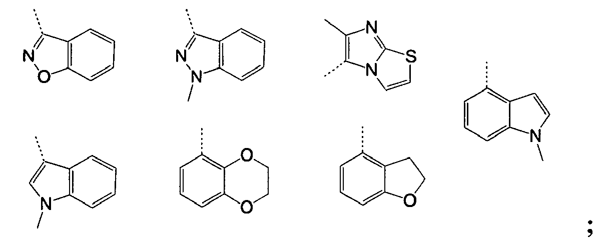 oder ein Salz davon.

9. Verbindung nach Anspruch 1, die ausgewählt ist aus der Gruppe bestehend aus:
(S)-4-Methyl-4H-furo[3,2-b]pyrrol-5-carbonsäure- {1-[2-cyclopropyl-5-(2-fluor-phenyl)-thiazol-4-carbonyl]-piperidin-2-ylmethyl}-amid;
(S)-Thieno[2,3-b]pyrazin-6-carbonsäure-{1-[2-cyclopropyl-5-(2-fluor-phenyl)-thiazol-4-carbonyl]-piperidin-2-ylmethyl}-amid;
(S)-3-Brom-N-{1-[2-cyclopropyl-5-(4-fluor-phenyl)-thiazol-4-carbonyl]-piperidin-2-ylmethyl}-benzamid;
(S)-Naphthalen-1-carbonsäure-{1-[2-cyclopropyl-5-(4-fluor-phenyl)-thiazol-4-carbonyl]-piperidin-2-ylmethyl}-amid;
(S)-Chinolin-8-carbonsäure-{1-[2-cyclopropyl-5-(4-fluor-phenyl)-thiazol-4-carbonyl]-piperidin-2-ylmethyl}-amid;
(S)-2,3-Dihydro-benzo[1,4]dioxin-5-carbonsäure-{1-[2-cyclopropyl-5-(4-fluor-phenyl)-thiazol-4-carbonyl]-piperidin-2-ylmethyl}-amid;
(S)-Benzofuran-4-carbonsäure-{1-[2-cyclopropyl-5-(4-fluor-phenyl)-thiazol-4-carbonyl]-piperidin-2-ylmethyl}-amid;
(S)-4-Methyl-4H-furo[3,2-b]pyrrol-5-carbonsäure-{1-[2-cyclopropyl-5-(4-fluor-phenyl)-thiazol-4-carbonyl]-piperidin-2-ylmethyl}amid;
(S)-Imidazo[2,1-b]thiazol-5-carbonsäure-[1-(2-cyclopropyl-5-p-tolyl-thiazol-4-carbonyl)-piperidin-2-ylmethyl]-amid;
(S)-Imidazo[2,1-b]thiazol-6-carbonsäure-[1-(2-cyclopropyl-5-p-tolyl-thiazol-4-carbonyl)-piperidin-2-ylmethyl]-amid;
(S)-1-Methyl-1H-indol-2-carbonsäure-[1-(2-cyclopropyl-5-p-tolyl-thiazol-4-carbonyl)-piperidin-2-ylmethyl]-amid;
(S)-Naphthalen-1-carbonsäure-[1-(2-cyclopropyl-5-m-tolyl-thiazol-4-carbonyl)-piperidin-2-ylmethyl]-amid;
(S)-Chinolin-8-carbonsäure-[1-(2-cyclopropyl-5-m-tolyl-thiazol-4-carbonyl)-piperidin-2-ylmethyl]-amid;
(S)-2,3-Dihydro-benzo[1,4]dioxin-5-carbonsäure-[1-(2-cyclopropyl-5-m-tolyl-thiazol-4-carbonyl)-piperidin-2-ylmethyl]-amid;
(S)-Benzofuran-4-carbonsäure-[1-(2-cyclopropyl-5-m-tolyl-thiazol-4-carbonyl)-piperidin-2-ylmethyl]-amid;
(S)-1-Methyl-1H-indazol-3-carbonsäure-[1-(2-cyclopropyl-5-m-tolyl-thiazol-4-carbonyl)-piperidin-2-ylmethyl]-amid;
(S)-6-Methyl-imidazo[2,1-b]thiazol-5-carbonsäure-[1-(2-cyclopropyl-5-m-tolyl-thiazol-4-carbonyl)-piperidin-2-ylmethyl]-amid;
(S)-Imidazo[2,1-b]thiazol-5-carbonsäure-[1-(2-cyclopropyl-5-m-tolyl-thiazol-4-carbonyl)-piperidin-2-ylmethyl]-amid;
(S)-1-Methyl-1H-indol-4-carbonsäure-[1-(2-cyclopropyl-5-m-tolyl-thiazol-4-carbonyl)-piperidin-2-ylmethyl]-amid;
(S)-3-Chlor-N-{1-[5-(3-chlor-phenyl)-2-cyclopropyl-thiazol-4-carbonyl]-piperidin-2-ylmethyl}-benzamid:
(S)-Chinolin-8-carbonsäure-{1-[5-(3-chlor-phenyl)-2-cyclopropyl-thiazol-4-carbonyl]-piperidin-2-ylmethyl}-amid;
(S)-Benzofuran-4-carbonsäure-{1-[5-(3-chlor-phenyl)-2-cyclopropyl-thiazol-4-carbonyl]-piperidin-2-ylmethyl}-amid;
(S)-1-Methyl-1H-indol-3-carbonsäure-{1-[5-(3-chlor-phenyl)-2-cyclopropyl-thiazol-4-carbonyl]-piperidin-2-ylmethyl}-amid;
(S)-Imidazo[2,1-b]thiazol-5-carbonsäure-{1-[5-(3-chlor-phenyl)-2-cyclopropyl-thiazol-4-carbonyl]-piperidin-2-ylmethyl}-amid;
(S)-4-Methyl-4H-furo[3,2-b]pyrrol-5-carbonsäure-{1-[5-(3-chlor-phenyl)-2-cyclopropyl-thiazol-4-carbonyl]-piperidin-2-ylmethyl}-amid;
(S)-1-Methyl-1H-indol-4-carbonsäure-{1-[5-(3-chlor-phenyl)-2-cyclopropyl-thiazol-4-carbonyl]-piperidin-2-ylmethyl}-amid;
(S)-Benzo[d]isoxazol-3-carbonsäure-{1-[5-(3-chlor-phenyl)-2-cyclopropyl-thiazol-4-carbonyl]-piperidin-2-ylmethyl}-amid;
(S)-N-{1-[2-Cyclopropyl-5-(3-trifluormethyl-phenyl)-thiazol-4-carbonyl]-piperidin-2-ylmethyl}-3-methyl-benzamid;
(S)-Chinolin-8-carbonsäure-{1-[2-cyclopropyl-5-(3-trifluormethyl-phenyl)-thiazol-4-carbonyl]-piperidin-2-ylmethyl}-amid;
(S)-2,3-Dihydro-benzo[1,4]dioxin-5-carbonsäure-{1-[2-cyclopropyl-5-(3-trifluormethylphenyl)-thiazol-4-carbonyl]-piperidin-2-ylmethyl}-amid;
(S)-Benzofuran-4-carbonsäure-{1-[2-cyclopropyl-5-(3-trifluormethyl-phenyl)-thiazol-4-carbonyl]-piperidin-2-ylmethyl}-amid;
(S)-Imidazo[2,1-b]thiazol-5-carbonsäure-{1-[2-cyclopropyl-5-(3-trifluormethyl-phenyl)-thiazol-4-carbonyl]-piperidin-2-ylmethyl}-amid;
(S)-3-Brom-N-{1-[2-cyclopropyl-5-(4-fluor-phenyl)-thiazol-4-carbonyl]-pyrrolidin-2-ylmethyl}-benzamid;
(S)-Chinolin-8-carbonsäure-{1-[2-cyclopropyl-5-(4-fluor-phenyl)-thiazol-4-carbonyl]-pyrrolidin-2-ylmethyl}-amid;
(S)-2,3-Dihydro-benzo[1,4]dioxin-5-carbonsäure-{1-[2-cyclopropyl-5-(4-fluor-phenyl)-thiazol-4-carbonyl]-pyrrolidin-2-ylmethyl}-amid;
(S)-Benzofuran-4-carbonsäure-{1-[2-cyclopropyl-5-(4-fluor-phenyl)-thiazol-4-carbonyl]-pyrrolidin-2-ylmethyl}-amid;
(S)-1-Methyl-1H-indol-3-carbonsäure-{1-[2-cyclopropyl-5-(4-fluor-phenyl)-thiazol-4-carbonyl]-pyrrolidin-2-ylmethyl}-amid;
(S)-1-Methyl-1H-indazol-3-carbonsäure-{1-[2-cyclopropyl-5-(4-fluor-phenyl)-thiazol-4-carbonyl]-pyrrolidin-2-ylmethyl}-amid;
(S)-Benzo[d]isoxazol-3-carbonsäure-{1-[2-cyclopropyl-5-(4-fluor-phenyl)-thiazol-4-carbonyl]-pyrrolidin-2-ylmethyl}-amid;
(S)-N-{1-[2-Cyclopropyl-5-(2-fluor-phenyl)-thiazol-4-carbonyl]-piperidin-2-ylmethyl}-3-methyl-benzamid;
(S)-3-Chlor-N-{1-[2-cyclopropyl-5-(2-fluor-phenyl)-thiazol-4-carbonyl]-piperidin-2-ylmethyl}-benzamid;
(S)-3-Brom-N-{1-[2-cyclopropyl-5-(2-fluor-phenyl)-thiazol-4-carbonyl]-piperidin-2-ylmethyl}-benzamid;
(S)-Naphthalen-1-carbonsäure-{1-[2-cyclopropyl-5-(2-fluor-phenyl)-thiazol-4-carbonyl]-piperidin-2-ylmethyl}-amid;
(S)-2,3-Dihydro-benzo[1,4]dioxin-5-carbonsäure-{1-[2-cyclopropyl-5-(2-fluor-phenyl)-thiazol-4-carbonyl]-piperidin-2-ylmethyl}-amid;
(S)-1-Methyl-1H-indol-3-carbonsäure-{1-[2-cyclopropyl-5-(2-fluor-phenyl)-thiazol-4-carbonyl]-piperidin-2-ylmethyl}-amid;
(S)-1-Methyl-1H-indol-6-carbonsäure-{1-[2-cyclopropyl-5-(2-fluor-phenyl)-thiazol-4-carbonyl]-piperidin-2-ylmethyl}-amid;
(S)-1-Methyl-1H-indol-4-carbonsäure-{1-[2-cyclopropyl-5-(2-fluor-phenyl)-thiazol-4-carbonyl]-piperidin-2-ylmethyl}-amid;
(S)-N-{1-[2-Cyclopropyl-5-(4-fluor-phenyl)-thiazol-4-carbonyl]-piperidin-2-ylmethyl}-3-methyl-benzamid;
(S)-3-Chlor-N-{1-[2-cyclopropyl-5-(4-fluor-phenyl)-thiazol-4-carbonyl]-piperidin-2-ylmethyl}-benzamid;
(S)-1-Methyl-1H-indol-3-carbonsäure-{1-[2-cyclopropyl-5-(4-fluor-phenyl)-thiazol-4-carbonyl]-piperidin-2-ylmethyl}-amid;
(S)-1-Methyl-1H-indazol-3-carbonsäure-{1-[2-cyclopropyl-5-(4-fluor-phenyl)-thiazol-4-carbonyl]-piperidin-2-ylmethyl}-amid;
(S)-2,3-Dihydro-benzo[1,4]dioxin-5-carbonsäure-[1-(2-cyclopropyl-5-p-tolyl-thiazol-4-carbonyl)-piperidin-2-ylmethyl]-amid;
(S)-N-[1-(2-Cyclopropyl-5-p-tolyl-thiazol-4-carbonyl)-piperidin-2-ylmethyl]-3-methyl-benzamid;
(S)-3-Brom-N-[1-(2-cyclopropyl-5-m-tolyl-thiazol-4-carbonyl)-piperidin-2-ylmethyl]-benzamid;
(S)-4-Methyl-4H-furo[3,2-b]pyrrol-5-carbonsäure-[1-(2-cyclopropyl-5-m-tolyl-thiazol-4-carbonyl)-piperidin-2-ylmethyl]-amid;
(S)-N-{1-[5-(3-Chlor-phenyl)-2-cyclopropyl-thiazol-4-carbonyl]-piperidin-2-ylmethyl}-3-methyl-benzamid;
(S)-3-Brom-N-{1-[5-(3-chlor-phenyl)-2-cyclopropyl-thiazol-4-carbonyl]-piperidin-2-ylmethyl}-benzamid;
(S)-2,3-Dihydro-benzo[1,4]dioxin-5-carbonsäure-{1-[5-(3-chlor-phenyl)-2-cyclopropyl-thiazol-4-carbonyl]-piperidin-2-ylmethyl}-amid;
(S)-1-Methyl-1H-indazol-3-carbonsäure-{1-[5-(3-chlor-phenyl)-2-cyclopropyl-thiazol-4-carbonyl]-piperidin-2-ylmethyl}-amid;
(S)-3-Chlor-N-{1-[2-cyclopropyl-5-(3-trifluormethyl-phenyl)-thiazol-4-carbonyl]-piperidin-2-ylmethyl}-benzamid;
(S)-3-Brom-N-{1-[2-cyclopropyl-5-(3-trifluormethyl-phenyl)-thiazol-4-carbonyl]-piperidin-2-ylmethyl}-benzamid;
(S)-1-Methyl-1H-indol-3-carbonsäure-{1-[2-cyclopropyl-5-(3-trifluormethyl-phenyl)-thiazol-4-carbonyl]-piperidin-2-ylmethyl}-amid;
(S)-1-Methyl-1H-indazol-3-carbonsäure-1-[2-cyclopropyl-5-(3-trifluormethyl-phenyl)-thiazol-4-carbonyl]-piperidin-2-ylmethyl}-amid;
(S)-6-Methyl-imidazo[2,1-b]thiazol-5-carbonsäure-{1-[2-cyclopropyl-5(4-fluor-phenyl)-thiazol-4-carbonyl]-pyrrolidin-2-ylmethyl}-amid;
(S)-Imidazo[2,1-b] thiazol-5-carbonsäure- {1-[2-cyclopropyl-5-(4-fluor-phenyl)-thiazol-4-carbonyl]-pyrrolidin-2-ylmethyl}-amid;
(S)-Chinolin-8-carbonsäure-{1-[2-cyclopropyl-5-(2-fluor-phenyl)-thiazol-4-carbonyl]-piperidin-2-ylmethyl}-amid;
(S)-Benzofuran-4-carbonsäure-{1-[2-cyclopropyl-5-(2-fluor-phenyl)-thiazol-4-carbonyl]-piperidin-2-ylmethyl -amid;
(S)-1-Methyl-1H-indazol-3-carbonsäure-{1-[2-cyclopropyl-5-(2-fluor-phenyl)-thiazol-4-carbonyl]-piperidin-2-ylmethyl}-amid;
(S)-6-Methyl-imidazo[2,1-b]thiazol-5-carbonsäure-{1-[2-cyclopropyl-5-(2-fluor-phenyl)-thiazol-4-carbonyl]-piperidin-2-ylmethyl}-amid;
(S)-Benzo[d]isoxazol-3-carbonsäure-{1-[2-cyclopropyl-5-(2-fluor-phenyl)-thiazol-4-carbonyl]-piperidin-2-ylmethyl}-amid;
(S)-6-Methyl-imidazo[2,1-b]thiazol-5-carbonsäure-{1-[2-cyelopropyl-5-(4-fluor-phenyl)-thiazol-4-carbonyl]-piperidin-2-ylmethyl}-amid;
(S)-6-Methyl-imidazo[2,1-b]thiazol-5-carbonsäure-[1-(2-cyclopropyl-5-p-tolyl-thiazol-4-carbonyl)-piperidin-2-ylmethyl]-amid;
(S)-1-Methyl-1H-indazol-3-carbonsäure-[1-(2-cyclopropyl-5-p-tolyl-thiazol-4-carbonyl)-piperidin-2-ylmethyl]-amid;
(S)-3-Brom-N-[1-(2-cyclopropyl-5-p-tolyl-thiazol-4-carbonyl)-piperidin-2-ylmethyl]-benzamid;
(S)-3-Chlor-N-[1-(2-cyclopropyl-5-p-tolyl-thiazol-4-carbonyl)-piperdin-2-ylmethyl]-benzamid;
(S)-N-[1-(2-Cyclopropyl-5-p-tolyl-thiazol-4-carbonyl)-piperidin-2-ylmethyl]-3-methyl-benzamid;
(S)-3-Chlor-N-[1-(2-cyclopropyl-5-m-tolyl-thiazol-4-carbonyl)-piperidin-2-ylmethyl]-benzamid;
(S)-1-Methyl-1H-indol-3-carbonsäure-[1-(2-cyclopropyl-5-m-tolyl-thiazol-4-carbonyl)-piperidin-2-ylmethyl]-amid;
(S)-6-Methyl-imidazo[2,1b]thiazol-5-carbonsäure-{1-[5-(3-chlor-phenyl)-2-cyclopropyl-thiazol-4-carbonyl]-piperidin-2-ylmethyl}-amid;
(S)-6-Methyl-imidazo[2,1-b]thiazol-5-carbonsäure-{1-[2-cyclopropyl-5-(3-trifluormethylphenyl)-thiazol-4-carbonyl]-piperidin-2-ylmethyl}-amid;
(S)-Thieno[2,3-b]pyrazin-6-carbonsäure-{1-[2-cyclopropyl-5-(4-fluor-phenyl)-thiazol-4-carbonyl]-piperidin-2-ylmethyl}-amid;
(S)-1-Methyl-1H-indol-6-carbonsäure-{1-[2-cyclopropyl-5-(4-fluor-phenyl)-thiazol-4-carbonyl]-piperidin-2-ylmethyl}-amid;
(S)-1-Methyl-1H-indol-4-carbonsäure-{1-[2-cyelopropyl-5-(4-fluor-phenyl)-thiazol-4-carbonyl]-piperidin-2-ylmethyl}-amid;
(S)-1-Methyl-1H-indol-5-carbonsäure-[1-(2-cyclopropyl-5-m-tolyl-thiazol-4-carbonyl)-piperidin-2-ylmethyl]-amid;
(S)-N-{1-[5-(3-Chlor-phenyl)-2-cyclopropyl-thiazol-4-carbonyl]-piperidin-2-ylmethyl}-3-methyl-benzamid;
(S)-Naphthalen-1-carbonsäure-{1-[5-(3-chlor-phenyl)-2-cyclopropyl-thiazol-4-carbonyl]-piperidin-2-ylmethyl}-amid;
(S)-4-Methyl-4H-furo[3,2-b]pyrrol-5-carbonsäure-{1-[5-(3-chlor-phenyl)-2-cyclopropyl-thiazol-4-carbonyl]-piperidin-2-ylmethyl}-amid;
(S)-1-Methyl-1H-indol-5-carbonsäure-{1-[5-(3-chlor-phenyl)-2-cyclopropyl-thiazol-4-carbonyl]-piperidin-2-ylmethyl}-amid;
(S)-Naphthalen-1-carbonsäure-{1-[2-cyclopropyl-5-(3-trifluormethyl-phenyl)-thiazol-4-carbonyl]-piperidin-2-ylmethyl}-amid;
(S)-Benzo[d]isoxazol-3-carbonsäure-{1-[2-cyclopropyl-5-(3-trifluormethyl-phenyl)-thiazol-4-carbonyl]-piperidin-2-ylmethyl}-amid;
(S)-3-Chlor-N-{1-[2-cyclopropyl-5-(4-fluor-phenyl)-thiazol-4-carbonyl]-pyrrolidin-2-ylmethyl}-benzamid;
(S)-Naphthalen-1-carbonsäure-{1-[2-cyclopropyl-5-(4-fluor-phenyl)-thiazol-4-carbonyl]-pyrrolidin-2-ylmethyl}-amid;
(S)-Imidazo[2,1-b]thiazol-5-carbonsäure-{1-[2-cyclopropyl-5-(4-fluor-phenyl)-thiazol-4-carbonyl]-pyrrolidin-2-ylmethyl}-amid;
(S)-4-Methyl-4H-furo[3,2-b]pyrrol-5-carbonsäure-{1-[2-cyclopropyl-5-(4-fluor-phenyl)-thiazol-4-carbonyl]-pyrrolidin-2-ylmethyl-amid;
(S)-1-Methyl-1H-indol-4-carbonsäure-{1-[2-cyclopropyl-5-(4-fluor-phenyl)-thiazol-4-carbonyl]-pyrrolidin-2-ylmethyl}-amid;
(S)-Imidazo[2,1-b]thiazol-5-carbonsäure-[1-(2-cyclopropyl-5-phenyl-thiazol-4-carbonyl)-piperidin-2-ylmethyl]-amid;
(S)-6-Methyl-imidazo[2,1-b]thiazol-5-carbonsäure-[1-(2-cyclopropyl-5-phenyl-thiazol-4-carbonyl)-piperidin-2-ylmethyl]-amid;
(S)-2,3-Dihydro-benzofuran-4-carbonsäure-[1-(2-cyclopropyl-5-phenyl-thiazol-4-carbonyl)-piperidin-2-ylmethyl]-amid;
(S)-Benzofuran-4-carbonsäure-[1-(2-cyclopropyl-5-phenyl-thiazol-4-carbonyl)-piperidin-2-ylmethyl]-amid;
(S)-1-Methyl-1H-indazol-3-carbonsäure-[1-(2-cyclopropyl-5-phenyl-thiazol-4-carbonyl)-piperidin-2-ylmethyl]-amid;
(S)-Chinolin-8-carbonsäure-[1-(2-cyclopropyl-5-phenyl-thiazol-4-carbonyl)-piperidin-2-ylmethyl]-amid;
(S)-N-[1-(2-Cyclopropyl-5-phenyl-thiazol-4-carbonyl)-piperidin-2-ylmethyl]-3,4-dimethyl-benzamid;
(S)-N-[1-(2-Cyclopropyl-5-phenyl-thiazol-4-carbonyl)-piperidin-2-ylmethyl]-4-methyl-3-trifluormethyl-benzamid;
(S)-3-Cyano-N-[1-(2-cyclopropyl-5-m-tolyl-thiazol-4-carbonyl)-piperidin-2-ylmethyl]-benzamid;
(S)-1-Methyl-1H-indol-3-carbonsäure-[1-(2-cyclopropyl-5-phenyl-thiazol-4-carbonyl)-piperidin-2-ylmethyl]-amid;
(S)-2,3-Dihydro-benzofuran-7-carbonsäure-[1-(2-cyclopropyl-5-m-tolyl-thiazol-4-carbonyl)-piperidin-2-ylmethyl]-amid;
(S)-2,3-Dihydro-benzofuran-4-carbonsäure-[1-(2-cyclopropyl-5-m-tolyl-thiazol-4-carbonyl)-piperidin-2-ylmethyl]-amid;
(S)-2-Ethyl-5-methyl-2H-pyrazol-3-carbonsäure-[1-(2-cyclopropyl-5-m-tolyl-thiazol-4-carbonyl)-piperidin-2-ylmethyl]-amid;
(S)-2,5-Dimethyl-2H-pyrazol-3-carbonsäure-[1-(2-cyclopropyl-5-m-tolyl-thiazol-4-carbonyl)-piperidin-2-ylmethyl]-amid;
(S)-3,4-Dihydro-2H-benzo[1,4]oxazin-5-carbonsäure-[1-(2-cyclopropyl-5-m-tolyl-thiazol-4-carbonyl)-piperidin-2-ylmethyl]-amid;
(S)-N-[1-(2-Cyclopropyl-5-m-tolyl-thiazol-4-carbonyl)-piperidin-2-ylmethyl]-3-methoxy-benzamid;
(S)-N-[1-(2-Cyclopropyl-5-m-tolyl-thiazol-4-carbonyl)-piperidin-2-ylmethyl]-3-trifluormethyl-benzamid;
(S)-2,3-Dihydro-thieno[3,4-b][1,4]dioxin-5-carbonsäure-[1-(2-cyclopropyl-5-m-tolyl-thiazol-4-carbonyl)-piperidin-2-ylmethyl]-amid;
(S)-N-[1-(2-Cyclopropyl-5-m-tolyl-thiazol-4-carbonyl)-piperidin-2-ylmethyl]-3-fluor-2-methyl-benzamid;
(S)-Benzo[d]isoxazol-3-carbonsäure-[1-(2-cyclopropyl-5-m-tolyl-thiazol-4-carbonyl)-piperidin-2-ylmethyl]-amid;
(S)-Benzo[d]isoxazol-3-carbonsäure-[1-(2-cyclopropyl-5-phenyl-thiazol-4-carbonyl)-piperidin-2-ylmethyl]-amid;
(S)-3,4-Dichlor-N-[1-(2-cyclopropyl-5-phenyl-thiazol-4-carbonyl)-piperidin-2-ylmethyl]-benzamid;
(S)-3-Chlor-N-[1-(2-cyclopropyl-5-m-tolyl-thiazol-4-carbonyl)-piperidin-2-ylmethyl]-2-methyl-benzamid;
(S)-2-Chlor-N-[1-(2-cyclopropyl-5-m-tolyl-thiazol-4-carbonyl)-piperidin-2-ylmethyl]-3-fluor-benzamid;
(S)-N-[1-(2-Cyclopropyl-5-phenyl-thiazol-4-carbonyl)-piperidin-2-ylmethyl]-3-methoxy-benzamid;
(S)-Chroman-8-carbonsäure-[1-(2-cyclopropyl-5-m-tolyl-thiazol-4-carbonyl)-piperidin-2-ylmethyl]-amid;
(S)-1H-Indazol-3-carbonsäure-[1-(2-cyclopropyl-5-m-tolyl-thiazol-4-carbonyl)-piperidin-2-ylmethyl]-amid; (S)-N-[1-(2-Cyclopropyl-5-phenyl-thiazol-4-carbonyl)-piperidin-2-ylmethyl]-3,5-dimethoxy-benzamid;
(S)-Isochinolin-1-carbonsäure-[1-(2-cyclopropyl-5-m-tolyl-thiazol-4-carbonyl)-piperidin-2-ylmethyl]-amid;
(S)-2,3-Dihydro-benzo[1,4]dioxin-5-carbonsäure-[1-(2-cyclopropyl-5-phenyl-thiazol-4-carbonyl)-piperidin-2-ylmethyl]-amid;
(S)-N-[1-(2-Cyclopropyl-5-phenyl-thiazol-4-carbonyl)-piperidin-2-ylmethyl]-benzamid;
(S)-N-[1-(2-Cyclopropyl-5-phenyl-thiazol-4-carbonyl)-piperidin-2-ylmethyl]-2,5-dimethoxy-benzamid;
(S)-3-Methyl-isoxazol-5-carbonsäure-[1-(2-cyclopropyl-5-m-tolyl-thiazol-4-carbonyl)-piperidin-2-ylmethyl]-amid;
(S)-1,2-Dimethyl-1H-indol-3-carbonsäure-[1-(2-cyclopropyl-5-m-tolyl-thiazol-4-carbonyl)-piperidin-2-ylmethyl]-amid;
(S)-3-Acetylamino-N-[1-(2-cyclopropyl-5-m-tolyl-thiazol-4-carbonyl)-piperidin-2-ylmethyl]-benzamid;
(S)-4-Methyl-4H-furo[3,2-b]pyrrol-5-carbonsäure-[1-(2-cyclopropyl-5-phenyl-thiazol-4-carbonyl)-piperidin-2-ylmethyl]-amid;
(S)-3-Methyl-1H-inden-2-carbonsäure-[1-(2-cyclopropyl-5-m-tolyl-thiazol-4-carbonyl)-piperidin-2-ylmethyl]-amid;
(S)-1-Methyl-1H-indol-4-carbonsäure-[1-(2-cyclopropyl-5-phenyl-thiazol-4-carbonyl)-piperidin-2-ylmethyl]-amid;
(S)-Imidazo[1,2-a]pyridin-3-carbonsäure-[1-(2-cyclopropyl-5-m-tolyl-thiazol-4-carbonyl)-piperidin-2-ylmethyl]-amid;
(S)-Naphthalen-1-carbonsäure-[1-(2-cyclopropyl-5-phenyl-thiazol-4-carbonyl)-piperidin-2-ylmethyl]-amid;
(S)-5-Fluor-1-methyl-1H-indol-2-carbonsäure-[1-(2-cyclopropyl-5-m-tolyl-thiazol-4-carbonyl)-piperidin-2-ylmethyl]-amid;
(S)-2,3-Dihydro-benzo[1,4]dioxin-6-carbonsäure-[1-(2-cyclopropyl-5-m-tolyl-thiazol-4-carbonyl)-piperidin-2-ylmethyl]-amid;
(S)-1-Methyl-1H-indol-5-carbonsäure-[1-(2-cyclopropyl-5-phenyl-thiazol-4-carbonyl)-piperidin-2-ylmethyl]-amid;
(S)-N-[1-(2-Cyclopropyl-5-m-tolyl-thiazol-4-carbonyl)-piperidin-2-ylmethyl]-4-methyl-nicotinamid;
(S)-2-Brom-N-[1-(2-cyclopropyl-5-m-tolyl-thiazol-4-carbonyl)-piperidin-2-ylmethyl]-3-methyl-benzamid;
(S)-N-[1-(2-Cyclopropyl-5-phenyl-thiazol-4-carbonyl)-piperidin-2-ylmethyl]-3,4-dimethoxy-benzamid;
(S)-N-[1-(2-Cyclopropyl-5-m-tolyl-thiazol-4-carbonyl)-piperidin-2-ylmethyl]-2,3-dimethyl-benzamid;
(S)-Thieno[2,3-b]pyrazin-6-carbonsäure-{1-[5-(3-chlor-phenyl)-2-cyclopropyl-thiazol-4-carbonyl]-piperidin-2-ylmethyl}-amid;
(S)-1-Methyl-1H-indol-4-carbonsäure-{1-[2-cyclopropyl-5-(3-trifluormethyl-phenyl)-thiazol-4-carbonyl]-piperidin-2-ylmethyl}-amid; und
(S)-2-Chlor-N-[1-(2-cyclopropyl-5-m-tolyl-thiazol-4-carbonyl)-piperidin-2-ylmethyl]-3-methyl-benzamid;
oder ein Salz einer solchen Verbindung.

10. Pharmazeutische Zusammensetzung, die eine Verbindung der Formel (I) nach einem der Ansprüche 1 bis 9 oder ein pharmazeutisch akzeptables Salz davon und einen pharmazeutisch akzeptablen Träger umfasst.

11. Verbindung nach einem der Ansprüche 1 bis 9 oder ein pharmazeutisch akzeptables Salz davon zur Verwendung als Medikament.

12. Verbindung nach einem der Ansprüche 1 bis 9 oder ein pharmazeutisch akzeptables Salz davon zur Vorbeugung gegen oder Behandlung von eine(r) Krankheit, die ausgewählt ist aus der Gruppe bestehend aus allen Arten von Schlafstörungen, stressbedingten Syndromen, Psychotropikumgebrauch und -missbrauch, kognitiven Fehlfunktionen bei der gesunden Bevölkerung und bei psychiatrischen und neurologischen Störungen, Ess- und Trinkstörungen.

13. Verwendung einer Verbindung nach einem der Ansprüche 1 bis 9 oder eines pharmazeutisch akzeptablen Salzes davon zur Herstellung einer pharmazeutischen Zusammensetzung zur Behandlung von Krankheiten oder Störungen, die ausgewählt sind aus der Gruppe bestehend aus Schlafstörungen, die alle Arten von Insomnien, Narkolepsie und andere exzessive Schläfrigkeitsstörungen, schlafbedingte Dystonien, das Restless-Legs-Syndrom, Schlafapnoen, das Jet-Lag-Syndrom, Schichtarbeiter-Syndrom, verzögerte oder vorgelagerte Schlafphasen-Syndrom und Insomnien im Zusammenhang mit psychiatrischen Störungen umfasst.

## Revendications

1. Composé de formule (I) 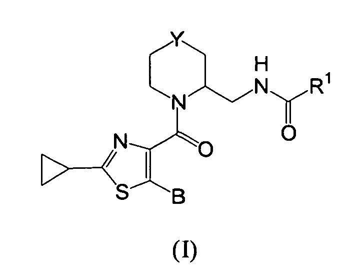 où
Y représente (CH₂)ₙ, où n représente 0 ou 1 ;
B représente un phényle, où le noyau phényle est non substitué ou mono-, di- ou trisubstitué, les substituants étant indépendamment sélectionnés dans le groupe consistant en un alkyle en (C₁₋₄), alkoxy en (C₁₋₄), trifluorométhyle et halogène ; et
R¹ représente un aryle ou un hétérocyclyle, où l'aryle ou l'hétérocyclyle est indépendamment non substitué ou mono-, di- ou trisubstitué, les substituants étant indépendamment sélectionnés dans le groupe consistant en un alkyle en (C₁₋₄), alkoxy en (C₁₋₄), halogène, cyano, trifluorométhyle et -NH-CO-alkyle en (C₁₋₄) ;
ou R¹ représente un groupement 2,3-dihydro-benzofuranyle, benzo[1,3]dioxolyle, 2,3-dihydro-benzo[1,4]dioxinyle, 4*H*-benzo[1,3]dioxinyle, 2*H*-chroményle, chromanyle, 2,3-dihydro-thiéno[3,4-b][1,4]dioxinyle, 3,4-dihydro-2*H*-benzo[1,4]oxazinyle ou indényle; lesdits groupements étant non substitués ou mono- ou disubstitués et les substituants étant indépendamment sélectionnés dans le groupe consistant en un alkyle en (C₁₋₄), alkoxy en (C₁₋₄) et halogène ;
ou sels de celui-ci.

2. Composé selon la revendication 1, où
Y représente (CH₂)ₙ, où n représente 0 ou 1 ;
B représente un phényle, où le noyau phényle est non substitué ou mono-, di- ou trisubstitué, les substituants étant indépendamment sélectionnés dans le groupe consistant en un alkyle en (C₁₋₄), alkoxy en (C₁₋₄), trifluorométhyle et halogène ; et
R¹ représente un aryle ou un hétérocyclyle, où l'aryle ou l'hétérocyclyle est indépendamment non substitué ou mono-, di- ou trisubstitué, les substituants étant indépendamment sélectionnés dans le groupe consistant en un alkyle en (C₁₋₄), alkoxy en (C₁₋₄), halogène, cyano et trifluorométhyle ;
ou R¹ représente un groupement 2,3-dihydro-benzofuranyle, benzo[1,3]dioxolyle, 2,3-dihydro-benzo[1,4]dioxinyle ou 4*H*-benzo[1,3]dioxinyle, lesdits groupements étant non substitués ou mono- ou disubstitués et les substituants étant indépendamment sélectionnés dans le groupe consistant en un alkyle en (C₁₋₄), alkoxy en (C₁₋₄) et halogène ;
ou sel de celui-ci.

3. Composé de formule (I) selon la revendication 1 ou 2, qui est également un composé de formule (Ia), où le centre stéréogène indiqué est en configuration (S) 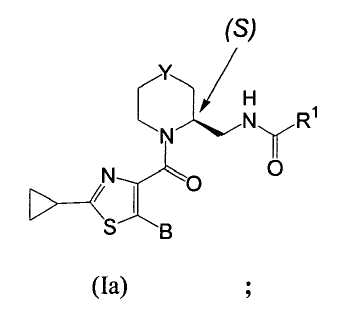 ou sel de celui-ci.

4. Composé selon l'une quelconque des revendications 1 à 3 où B représente un phényle, où le noyau phényle est non substitué ou mono- ou disubstitué, les substituants étant indépendamment sélectionnés dans le groupe consistant en un alkyle en (C₁₋₄), alkoxy en (C₁₋₄), trifluorométhyle et halogène ;
ou sel de celui-ci.

5. Composé selon l'une quelconque des revendications 1 à 4 où B représente un phényle, où le noyau phényle est non substitué ou monosubstitué, le substituant étant sélectionné dans le groupe consistant en un méthyle, chloro, fluoro et trifluorométhyle ;
ou sel de celui-ci.

6. Composé selon l'une quelconque des revendications 1 à 5, où
R¹ représente un phényle qui est monosubstitué, le substituants étant sélectionné dans le groupe consistant en un chloro, bromo et méthyle ;
ou R¹ représente un hétérocyclyle, où l'hétérocyclyle est sélectionné dans le groupe consistant en un indolyle, benzofuranyle, indazolyle, benzisoxazolyle, quinolinyle et imidazo[2,1-b]thiazolyle, ledit hétérocyclyle étant non substitué ou monosubstitué par un substituant sélectionné parmi des alkyles en (C₁₋₄) ;
ou R¹ représente un groupement 2,3-dihydro-benzofuranyle ou 2,3-dihydro-benzo[1,4]dioxinyle, lesdits groupements étant non substitués ;
ou sel de celui-ci.

7. Composé selon l'une quelconque des revendications 1 à 6 ou sel de celui-ci, où
R¹ représente un groupement l'un des groupements suivants : 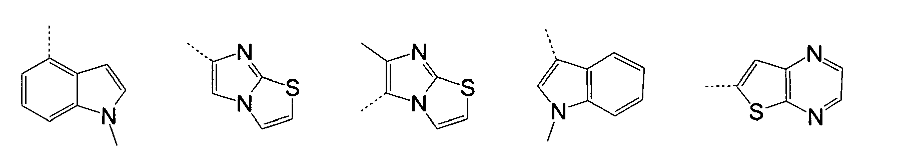 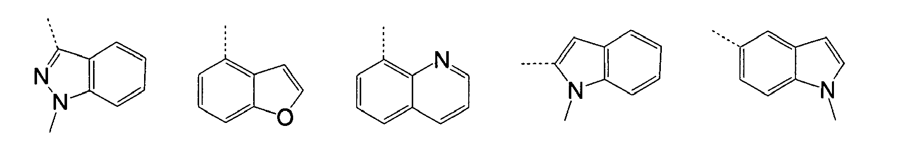 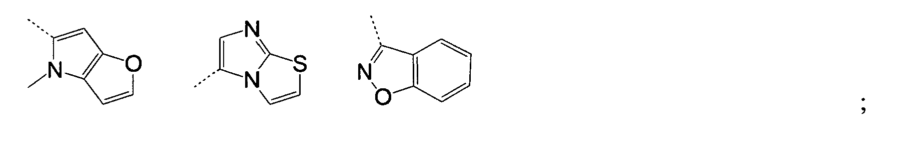 ou sel de celui-ci.

8. Composé selon l'une quelconque des revendications 1 à 6 ou sel de celui-ci, où
R¹ représente un groupement l'un des groupements suivants : 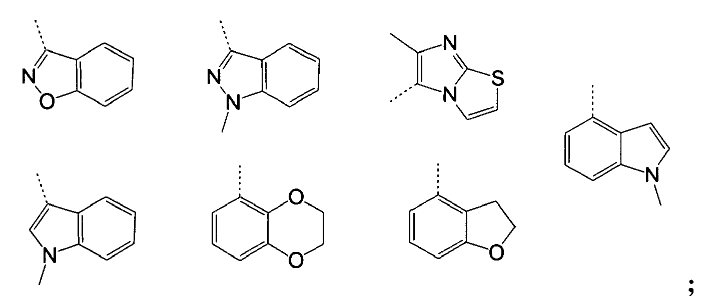 ou sel de celui-ci.

9. Composé selon la revendication 1, qui est sélectionné dans le groupe consistant en les suivants :
{1-[2-Cyclopropyl-5-(2-fluoro-phényl)-thiazole-4-carbonyl]-pipéridin-2-ylméthyl}-amide de l'acide (S)-4-méthyl-4*H*-furo[3,2-b]pyrrole-5-carboxylique ;
{1-[2-Cyclopropyl-5-(2-fluoro-phényl)-thiazole-4-carbonyl]-pipéridin-2-ylméthyl}-amide de l'acide (S)-thiéno[2,3-b]pyrazine-6-carboxylique ;
(S)-3-Bromo-*N*-{1-[2-cyclopropyl-5-(4-fluoro-phényl)-thiazole-4-carbonyl]-pipéridin-2-ylméthyl}-benzamide ;
{1-[2-Cyclopropyl-5-(4-fluoro-phényl)-thiazole-4-carbonyl]-pipéridin-2-ylméthyl}-amide de l'acide (S)-naphtalène-1-carboxylique ;
{1-[2-Cyclopropyl-5-(4-fluoro-phényl)-thiazole-4-carbonyl]-pipéridin-2-ylméthyl}-amide de l'acide (S)-quinoline-8-carboxylique ;
{1-[2-Cyclopropyl-5-(4-fluoro-phényl)-thiazole-4-carbonyl]-pipéridin-2-ylméthyl}-amide de l'acide (S)-2,3-dihydro-benzo[1,4]dioxine-5-carboxylique ;
{1-[2-Cyclopropyl-5-(4-fluoro-phényl)-thiazole-4-carbonyl]-pipéridin-2-ylméthyl}-amide de l'acide (S)-benzofuran-4-carboxylique ;
{1-[2-Cyclopropyl-5-(4-fluoro-phényl)-thiazole-4-carbonyl]-pipéridin-2-ylméthyl}-amide de l'acide (S)-4-méthyl-4*H-*furo[3,2-b]pyrrole-5-carboxylique ;
[1-(2-Cyclopropyl-5-*p*-tolyl-thiazole-4-carbonyl)-pipéridin-2-ylméthyl]-amide de l'acide (S)-imidazo[2,1-b]thiazole-5-carboxylique ;
[1-(2-Cyclopropyl-5-*p*-tolyl-thiazole-4-carbonyl)-pipéridin-2-ylméthyl]-amide de l'acide (S)-imidazo[2,1-b]thiazole-6-carboxylique ;
[1-(2-Cyclopropyl-5-*p*-tolyl-thiazole-4-carbonyl)-pipéridin-2-ylméthyl]-amide de l'acide (S)-1-méthyl-1*H*-indole-2-carboxylique ;
[1-(2-Cyclopropyl-5-*m*-tolyl-thiazole-4-carbonyl)-pipéridin-2-ylméthyl]-amide de l'acide (S)-naphtalène-1-carboxylique ;
[1-(2-Cyclopropyl-5-*m*-tolyl-thiazole-4-carbonyl)-pipéridin-2-ylméthyl]-amide de l'acide (S)-quinoline-8-carboxylique ;
[1-(2-Cyclopropyl-5-*m*-tolyl-thiazole-4-carbonyl)-pipéridin-2-ylméthyl]-amide de l'acide (S)-2,3-dihydro-benzo[1,4]dioxine-5-carboxylique ;
[1-(2-Cyclopropyl-5-*m*-tolyl-thiazole-4-carbonyl)-pipéridin-2-ylméthyl]-amide de l'acide (S)-benzofuran-4-carboxylique ;
[1-(2-Cyclopropyl-5-*m*-tolyl-thiazole-4-carbonyl)-pipéridin-2-ylméthyl]-amide de l'acide (S)-1-méthyl-1*H*-indazole-3-carboxylique;
[1-(2-Cyclopropyl-5-*m*-tolyl-thiazole-4-carbonyl)-pipéridin-2-ylméthyl]-amide de l'acide (S)-6-méthyl-imidazo[2,1-b]thiazole-5-carboxylique ;
[1-(2-Cyclopropyl-5-*m*-tolyl-thiazole-4-carbonyl)-pipéridin-2-ylméthyl]-amide de l'acide (S)-imidazo[2,1-b]thiazole-5-carboxylique ;
[1-(2-Cyclopropyl-5-*m*-tolyl-thiazole-4-carbonyl)-pipéridin-2-ylméthyl]-amide de l'acide (S)-1-méthyl-1*H*-indole-4-carboxylique ;
(S)-3-Chloro-*N*-{1-[5-(3-chloro-phényl)-2-cyclopropyl-thiazole-4-carbonyl]-pipéridin-2-ylméthyl}-benzamide ;
{1-[5-(3-Chloro-phényl)-2-cyclopropyl-thiazole-4-carbonyl]-pipéridin-2-ylméthyl}-amide de l'acide (S)-quinoline-8-carboxylique ;
{1-[5-(3-Chloro-phényl)-2-cyclopropyl-thiazole-4-carbonyl]-pipéridin-2-ylméthyl}-amide de l'acide (S)-benzofuran-4-carboxylique ;
{1-[5-(3-Chloro-phényl)-2-cyclopropyl-thiazole-4-carbonyl]-pipéridin-2-ylméthyl}-amide de l'acide (S)-1-méthyl-1*H*-indole-3-carboxylique ;
{1-[5-(3-Chloro-phényl)-2-cyclopropyl-thiazole-4-carbonyl]-pipéridin-2-ylméthyl}-amide de l'acide (S)-imidazo[2,1-b]thiazole-5-carboxylique ;
{1-[5-(3-Chloro-phényl)-2-cyclopropyl-thiazole-4-carbonyl]-pipéridin-2-ylméthyl}-amide de l'acide (S)-4-méthyl-4*H*-furo[3,2-b]pyrrole-5-carboxylique ;
{1-[5-(3-Chloro-phényl)-2-cyclopropyl-thiazole-4-carbonyl]-pipéridin-2-ylméthyl}-amide de l'acide (S)-1-méthyl-1*H*-indole-4-carboxylique ;
{1-[5-(3-Chloro-phényl)-2-cyclopropyl-thiazole-4-carbonyl]-pipéridin-2-ylméthyl}-amide de l'acide (S)-benzo[d]isoxazole-3-carboxylique ;
(S)-*N*-{1-[2-Cyclopropyl-5-(3-trifluorométhyl-phényl)-thiazole-4-carbonyl]-pipéridin-2-ylméthyl}-3-méthyl-benzamide ;
{1-[2-Cyclopropyl-5-(3-trifluorométhyl-phényl)-thiazole-4-carbonyl]-pipéridin-2-ylméthyl}-amide de l'acide (S)-quinoline-8-carboxylique ;
{1-[2-Cyclopropyl-5-(3-trifluorométhyl-phényl)-thiazole-4-carbonyl]-pipéridin-2-ylméthyl}-amide de l'acide (S)-2,3-dihydro-benzo[1,4]dioxine-5-carboxylique ;
{1-[2-Cyclopropyl-5-(3-trifluorométhyl-phényl)-thiazole-4-carbonyl]-pipéridin-2-ylméthyl}-amide de l'acide (S)-benzofuran-4-carboxylique ;
{1-[2-Cyclopropyl-5-(3-trifluorométhyl-phényl)-thiazole-4-carbonyl]-pipéridin-2-ylméthyl}-amide de l'acide (S)-imidazo[2,1-b]thiazole-5-carboxylique ;
(S)-3-Bromo-*N*-{1-[2-cyclopropyl-5-(4-fluoro-phényl)-thiazole-4-carbonyl]-pyrrolidin-2-ylméthyl}-benzamide ;
{1-[2-Cyclopropyl-5-(4-fluoro-phényl)-thiazole-4-carbonyl]-pyrrolidin-2-ylméthyl}-amide de l'acide (S)-quinoline-8-carboxylique ;
{1-[2-Cyclopropyl-5-(4-fluoro-phényl)-thiazole-4-carbonyl]-pyrrolidin-2-ylméthyl}-amide de l'acide (S)-2,3-dihydro-benzo[1,4]dioxine-5-carboxylique ;
{1-[2-Cyclopropyl-5-(4-fluoro-phényl)-thiazole-4-carbonyl]-pyrrolidin-2-ylméthyl}-amide de l'acide (S)-benzofuran-4-carboxylique ;
{1-[2-Cyclopropyl-5-(4-fluoro-phényl)-thiazole-4-carbonyl]-pyrrolidin-2-ylméthyl}-amide de l'acide (S)-1-méthyl-1*H*-indole-3-carboxylique ;
{1-[2-Cyclopropyl-5-(4-fluoro-phényl)-thiazole-4-carbonyl]-pyrrolidin-2-ylméthyl}-amide de l'acide (S)-1-méthyl-1*H*-indazole-3-carboxylique ;
{1-[2-Cyclopropyl-5-(4-fluoro-phényl)-thiazole-4-carbonyl]-pyrrolidin-2-ylméthyl}-amide de l'acide (S)-benzo[d]isoxazole-3-carboxylique ;
(S)-*N*-{1-[2-Cyclopropyl-5-(2-fluoro-phényl)-thiazole-4-carbonyl]-pipéridin-2-ylméthyl}-3-méthyl-benzamide ;
(S)-3-Chloro-*N*-{1-[2-cyclopropyl-5-(2-fluoro-phényl)-thiazole-4-carbonyl]-pipéridin-2-ylméthyl}-benzamide ;
(S)-3-Bromo-*N*-{1-[2-cyclopropyl-5-(2-fluoro-phényl)-thiazole-4-carbonyl]-pipéridin-2-ylméthyl}-benzamide ;
{1-[2-Cyclopropyl-5-(2-fluoro-phényl)-thiazole-4-carbonyl]-pipéridin-2-ylméthyl}-amide de l'acide (S)-naphtalène-1-carboxylique ;
{1-[2-Cyclopropyl-5-(2-fluoro-phényl)-thiazole-4-carbonyl]-pipéridin-2-ylméthyl}-amide de l'acide (S)-2,3-dihydro-benzo[1,4]dioxine-5-carboxylique ;
{1-[2-Cyclopropyl-5-(2-fluoro-phényl)-thiazole-4-carbonyl]-pipéridin-2-ylméthyl}-amide de l'acide (S)-1-méthyl-1*H*-indole-3-carboxylique ;
{1-[2-Cyclopropyl-5-(2-fluoro-phényl)-thiazole-4-carbonyl]-pipéridin-2-ylméthyl}-amide de l'acide (S)-1-méthyl-1*H*-indole-6-carboxylique ;
{1-[2-Cyclopropyl-5-(2-fluoro-phényl)-thiazole-4-carbonyl]-pipéridin-2-ylméthyl}-amide de l'acide (S)-1-méthyl-1*H*-indole-4-carboxylique ;
(S)-*N*-{1-[2-Cyclopropyl-5-(4-fluoro-phényl)-thiazole-4-carbonyl]-pipéridin-2-ylméthyl}-3-méthyl-benzamide ;
(S)-3-Chloro-*N-*{1-[2-cyclopropyl-5-(4-fluoro-phényl)-thiazole-4-carbonyl]-pipéridin-2-ylméthyl}-benzamide ;
{1-[2-Cyclopropyl-5-(4-fluoro-phényl)-thiazole-4-carbonyl]-pipéridin-2-ylméthyl}-amide de l'acide (S)-1-méthyl-1*H*-indole-3-carboxylique ;
{1-[2-Cyclopropyl-5-(4-fluoro-phényl)-thiazole-4-carbonyl]-pipéridin-2-ylméthyl}-amide de l'acide (S)-1-méthyl-1*H*-indazole-3-carboxylique ;
[1-(2-Cyclopropyl-5-*p*-tolyl-thiazole-4-carbonyl)-pipéridin-2-ylméthyl]-amide de l'acide (S)-2,3-dihydro-benzo[1,4]dioxine-5-carboxylique ;
(S)-*N*-[1-(2-Cyclopropyl-5-*p*-tolyl-thiazole-4-carbonyl)-pipéridin-2-ylméthyl]-3-méthyl-benzamide ;
(S)-3-Bromo-*N*-[1-(2-cyclopropyl-5-*m*-tolyl-thiazole-4-carbonyl)-pipéridin-2-ylméthyl]-benzamide ;
[1-(2-Cyclopropyl-5-*m*-tolyl-thiazole-4-carbonyl)-pipéridin-2-ylméthyl]-amide de l'acide (S)-4-méthyl-4H-furo[3,2-b]pyrrole-5 -carboxylique ;
(S)-*N*-{1-[5-(3-Chloro-phényl)-2-cyclopropyl-thiazole-4-carbonyl]-pipéridin-2-ylméthyl}-3-méthyl-benzamide ;
(S)-3-Bromo-*N*-{1-[5-(3-chloro-phényl)-2-cyclopropyl-thiazole-4-carbonyl]-pipéridin-2-ylméthyl}-benzamide ;
{1-[5-(3-Chloro-phényl)-2-cyclopropyl-thiazole-4-carbonyl]-pipéridin-2-ylméthyl}-amide de l'acide (S)-2,3-dihydro-benzo[1,4]dioxine-5-carboxylique ;
{1-[5-(3-Chloro-phényl)-2-cyclopropyl-thiazole-4-carbonyl]-pipéridin-2-ylméthyl}-amide de l'acide (S)-1-méthyl-1*H*-indazole-3-carboxylique ;
(S)-3-Chloro-*N*-{1-[2-cyclopropyl-5-(3-trifluorométhyl-phényl)-thiazole-4-carbonyl]-pipéridin-2-ylméthyl}-benzamide ;
(S)-3-Bromo-*N*-{1-[2-cyclopropyl-5-(3-trifluorométhyl-phényl)-thiazole-4-carbonyl]-pipéridin-2-ylméthyl}-benzamide ;
{1-[2-Cyclopropyl-5-(3-trifluorométhyl-phényl)-thiazole-4-carbonyl]-pipéridin-2-ylméthyl}-amide de l'acide (S)-1-méthyl-1*H*-indole-3-carboxylique ;
{1-[2-Cyclopropyl-5-(3-trifluorométhyl-phényl)-thiazole-4-carbonyl]-pipéridin-2-ylméthyl}-amide de l'acide (S)-1-méthyl-1*H*-indazole-3-carboxylique ;
{1-[2-Cyclopropyl-5-(4-fluoro-phényl)-thiazole-4-carbonyl]-pyrrolidin-2-ylméthyl}-amide de l'acide (S)-6-méthyl-imidazo[2,1-b]thiazole-5-carboxylique ;
{1-[2-Cyclopropyl-5-(4-fluoro-phényl)-thiazole-4-carbonyl]-pyrrolidin-2-ylméthyl}-amide de l'acide (S)-imidazo[2,1-b]thiazole-5-carboxylique ;
{1-[2-Cyclopropyl-5-(2-fluoro-phényl)-thiazole-4-carbonyl]-pipéridin-2-ylméthyl}-amide de l'acide (S)-quinoline-8-carboxylique ;
(1-[2-Cyclopropyl-5-(2-fluoro-phényl)-thiazole-4-carbonyl]-pipéridin-2-ylméthyl)-amide de l'acide (S)-benzofuran-4-carboxylique ;
{1-[2-Cyclopropyl-5-(2-fluoro-phényl)-thiazole-4-carbonyl]-pipéridin-2-ylméthyl}-amide de l'acide (S)-1-méthyl-1*H*-indazole-3-carboxylique ;
{1-[2-Cyclopropyl-5-(2-fluoro-phényl)-thiazole-4-carbonyl]-pipéfidin-2-ylméthyl}-amide de l'acide (S)-6-méthyl-imidazo[2,1-b]thiazole-5-carboxylique ;
{1-[2-Cyclopropyl-5-(2-fluoro-phényl)-thiazole-4-carbonyl]-pipéridin-2-ylméthyl}-amide de l'acide (S)-benzo[d]isoxazole-3-carboxylique ;
{1-[2-Cyclopropyl-5-(4-fluoro-phényl)-thiazole-4-carbonyl]-pipéridin-2-ylméthyl}-amide de l'acide (S)-6-méthyl-imidazo[2,1-b]thiazole-5-carboxylique;
[1-(2-Cyclopropyl-5-*p*-tolyl-thiazole-4-carbonyl)-pipéridin-2-ylméthyl]-amide de l'acide (S)-6-méthyl-imidazo[2,1-b]thiazole-5-carboxylique ;
[1-(2-Cyclopropyl-5-*p*-tolyl-thiazole-4-carbonyl)-pipéridin-2-ylméthyl]-amide de l'acide (S)-1-méthyl-1*H*-indazole-3-carboxylique ;
(S)-3-Bromo-*N*-[1-(2-cyclopropyl-5-*p*-tolyl-thiazole-4-carbonyl)-pipéridin-2-ylméthyl]-benzamide ;
(S)-3-Chloro-*N*-[1-(2-cyclopropyl-5-*p*-tolyl-thiazole-4-carbonyl)-pipéridin-2-ylméthyl]-benzamide ;
(S)-*N*-[1-(2-Cyclopropyl-5-*p*-tolyl-thiazole-4-carbonyl)-pipéridin-2-ylméthyl]-3-méthyl-benzamide ;
(S)-3-Chloro-*N*-[1-(2-cyclopropyl-5-*m*-tolyl-thiazole-4-carbonyl)-pipéridin-2-ylméthyl]-benzamide ;
[1-(2-Cyclopropyl-5-*m*-tolyl-thiazole-4-carbonyl)-pipéridin-2-ylméthyl]-amide de l'acide (S)-1-méthyl-1*H*-indole-3-carboxylique ;
{1-[5-(3-Chloro-phényl)-2-cyclopropyl-thiazole-4-carbonyl]-pipéridin-2-ylméthyl}-amide de l'acide (S)-6-méthyl-imidazo[2,1-b]thiazole-5-carboxylique ;
{1-[2-Cyclopropyl-5-(3-trifluorométhyl-phényl)-thiazole-4-carbonyl]-pipéridin-2-ylméthyl}-amide de l'acide (S)-6-méthyl-imidazo[2,1-b]thiazole-5-carboxylique ;
{1-[2-Cyclopropyl-5-(4-fluoro-phényl)-thiazole-4-carbonyl]-pipéridin-2-ylméthyl}-amide de l'acide (S)-thiéno[2,3-b]pyrazine-6-carboxylique ;
{1-[2-Cyclopropyl-5-(4-fluoro-phényl)-thiazole-4-carbonyl]-pipéridin-2-ylméthyl}-amide de l'acide (S)-1-méthyl-1*H*-indole-6-carboxylique ;
{1-[2-Cyclopropyl-5-(4-fluoro-phényl)-thiazole-4-carbonyl]-pipéridin-2-ylméthyl}-amide de l'acide (S)-1-méthyl-1*H*-indole-4-carboxylique ;
[1-(2-Cyclopropyl-5-*m*-tolyl-thiazole-4-carbonyl)-pipéridin-2-ylméthyl]-amide de l'acide (S)-1-méthyl-1*H*-indole-5-carboxylique ;
(S)-*N*-{1-[5-(3-Chloro-phényl)-2-cyclopropyl-thiazole-4-carbonyl]-pipéridin-2-ylméthyl}-3-méthyl-benzamide ;
{1-[5-(3-Chloro-phényl)-2-cyclopropyl-thiazole-4-carbonyl]-pipéridin-2-ylméthyl}-amide de l'acide (S)-naphtalène-1-carboxylique ;
{1-[5-(3-Chloro-phényl)-2-cyclopropyl-thiazole-4-carbonyl]-pipéridin-2-ylméthyl}-amide de l'acide (S)-4-méthyl-4*H*-furo[3,2-b]pyrrole-5-carboxylique ;
{1-[5-(3-Chloro-phényl)-2-cyclopropyl-thiazole-4-carbonyl]-pipéridin-2-ylméthyl}-amide de l'acide (S)-1-méthyl-1*H*-indole-5-carboxylique ;
{1-[2-Cyclopropyl-5-(3-trifluorométhyl-phényl)-thiazole-4-carbonyl]-pipéridin-2-ylméthyl}-amide de l'acide (S)-naphtalène-1-carboxylique ;
{1-[2-Cyclopropyl-5-(3-trifluorométhyl-phényl)-thiazole-4-carbonyl]-pipéridin-2-ylméthyl}-amide de l'acide (S)-benzo[d]isoxazole-3-carboxylique ;
(S)-3-Chloro-*N*-{1-[2-cyclopropyl-5-(4-fluoro-phényl)-thiazole-4-carbonyl]-pyrrolidin-2-ylméthyl}-benzamide ;
{1-[2-Cyclopropyl-5-(4-fluoro-phényl)-thiazole-4-carbonyl]-pyrrolidin-2-ylméthyl}-amide de l'acide (S)-naphtalène-1-carboxylique ;
{1-[2-Cyclopropyl-5-(4-fluoro-phényl)-thiazole-4-carbonyl]-pyrrolidin-2-ylméthyl}-amide de l'acide (S)-imidazo[2,1-b]thiazole-5-carboxylique ;
{1-[2-Cyclopropyl-5-(4-fluoro-phényl)-thiazole-4-carbonyl]-pyrrolidin-2-ylméthyl}-amide de l'acide (S)-4-méthyl-4*H*-furo[3,2-b]pyrrole-5-carboxylique ;
{1-[2-Cyclopropyl-5-(4-fluoro-phényl)-thiazole-4-carbonyl]-pyrrolidin-2-ylméthyl}-amide de l'acide (S)-1-méthyl-1*H*-indole-4-carboxylique ;
[1-(2-Cyclopropyl-5-phényl-thiazole-4-carbonyl)-pipéridin-2-ylméthyl]-amide de l'acide (S)-imidazo[2,1-b]thiazole-5-carboxylique ;
[1-(2-Cyclopropyl-5-phényl-thiazole-4-carbonyl)-pipéridin-2-ylméthyl]-amide de l'acide (S)-6-méthyl-imidazo[2,1-b]thiazole-5-carboxylique ;
[1-(2-Cyclopropyl-5-phényl-thiazole-4-carbonyl)-pipéridin-2-ylméthyl]-amide de l'acide (S)-2,3-dihydro-benzofuran-4-carboxylique ;
[1-(2-Cyclopropyl-5-phényl-thiazole-4-carbonyl)-pipéridin-2-ylméthyl]-amide de l'acide (S)-benzofuran-4-carboxylique ;
[1-(2-Cyclopropyl-5-phényl-thiazole-4-carbonyl)-pipéridin-2-ylméthyl]-amide de l'acide (S)-1-méthyl-1*H*-indazole-3-carboxylique ;
[1-(2-Cyclopropyl-5-phényl-thiazole-4-carbonyl)-pipéridin-2-ylméthyl]-amide de l'acide (S)-quinoline-8-carboxylique ;
(S)-*N*-[1-(2-Cyclopropyl-5-phényl-thiazole-4-carbonyl)-pipéridin-2-ylméthyl]-3,4-diméthyl-benzamide ;
(S)-*N*-[1-(2-Cyclopropyl-5-phényl-thiazole-4-carbonyl)-pipéridin-2-ylméthyl]-4-méthyl-3-trifluorométhyl-benzamide ;
(S)-3-Cyano-*N*-[1-(2-cyclopropyl-5-*m*-tolyl-thiazole-4-carbonyl)-pipéridin-2-ylméthyl]-benzamide ;
[1-(2-Cyclopropyl-5-phényl-thiazole-4-carbonyl)-pipéridin-2-ylméthyl]-amide de l'acide (S)-1-méthyl-1*H*-indole-3-carboxylique ;
[1-(2-Cyclopropyl-5-*m*-tolyl-thiazole-4-carbonyl)-pipéridin-2-ylméthyl]-amide de l'acide (S)-2,3-dihydro-benzofuran-7-carboxylique ;
[1-(2-Cyclopropyl-5-*m*-tolyl-thiazole-4-carbonyl)-pipéridin-2-ylméthyl]-amide de l'acide (S)-2,3-dihydro-benzofuran-4-carboxylique ;
[1-(2-Cyclopropyl-5-*m*-tolyl-thiazole-4-carbonyl)-pipéridin-2-ylméthyl]-amide de l'acide (S)-2-éthyl-5-méthyl-2*H*-pyrazole-3-carboxylique ;
[1-(2-Cyclopropyl-5-*m*-tolyl-thiazole-4-carbonyl)-pipéridin-2-ylméthyl]-amide de l'acide (S)-2,5-diméthyl-2*H*-pyrazole-3 -carboxylique ;
[1-(2-Cyclopropyl-5-*m*-tolyl-thiazole-4-carbonyl)-pipéridin-2-ylméthyl]-amide de l'acide (S)-3,4-dihydro-2*H*-benzo[1,4]oxazine-5-carboxylique ;
(S)-*N*-[1-(2-Cyclopropyl-5-*m*-tolyl-thiazole-4-carbonyl)-pipéridin-2-ylméthyl]-3-méthoxy-benzamide ;
(S)-*N*-[1-(2-Cyclopropyl-5-*m*-tolyl-thiazole-4-carbonyl)-pipéridin-2-ylméthyl]-3-trifluorométhyl-benzamide ;
[1-(2-Cyclopropyl-5-*m*-tolyl-thiazole-4-carbonyl)-pipéridin-2-ylméthyl]-amide de l'acide (S)-2,3-dihydro-thiéno[3,4-b][1,4]dioxine-5-carboxylique ;
(S)-*N*-1-(2-Cyclopropyl-5-*m*-tolyl-thiazole-4-carbonyl)-pipéridin-2-ylméthyl]-3-fluoro-2-méthyl-benzamide ;
[1-(2-Cyclopropyl-5-*m*-tolyl-thiazole-4-carbonyl)-pipéridin-2-ylméthyl]-amide de l'acide (S)-benzo[d]isoxazole-3-carboxylique ;
[1-(2-Cyclopropyl-5-phényl-thiazole-4-carbonyl)-pipéridin-2-ylméthyl]-amide de l'acide (S)-benzo[d]isoxazole-3-carboxylique ;
(S)-3,4-Dichloro-*N*-[1-(2-cyclopropyl-5-phényl-thiazole-4-carbonyl)-pipéridin-2-ylméthyl]-benzamide ;
(S)-3-Chloro-*N*-[1-(2-cyclopropyl-5-*m*-tolyl-thiazole-4-carbonyl)-pipéridin-2-ylméthyl]-2-méthyl-benzamide ;
(S)-2-Chloro-*N*-[1-(2-cyclopropyl-5-*m*-tolyl-thiazole-4-carbonyl)-pipéridin-2-ylméthyl]-3-fluoro-benzamide ;
(S)-*N*-[1-(2-Cyclopropyl-5-phényl-thiazole-4-carbonyl)-pipéridin-2-ylméthyl]-3-méthoxy-benzamide ;
[1-(2-Cyclopropyl-5-*m*-tolyl-thiazole-4-carbonyl)-pipéridin-2-ylméthyl]-amide de l'acide (S)-chroman-8-carboxylique ;
[1-(2-Cyclopropyl-5-*m*-tolyl-thiazole-4-carbonyl)-pipéridin-2-ylméthyl]-amide de l'acide (S)-1*H*-indazole-3-carboxylique ;
(S)-*N*-[1-(2-Cyclopropyl-5-phényl-thiazole-4-carbonyl)-pipéridin-2-ylméthyl]-3,5-diméthoxy-benzamide ;
[1-(2-Cyclopropyl-5-*m*-tolyl-thiazole-4-carbonyl)-pipéridin-2-ylméthyl]-amide de l'acide (S)-isoquinoline-1-carboxylique ;
[1-(2-Cyclopropyl-5-phényl-thiazole-4-carbonyl)-pipéridin-2-ylméthyl]-amide de l'acide (S)-2,3-dihydro-benzo[1,4]dioxine-5-carboxylique ;
(S)-*N*-[1-(2-Cyclopropyl-5-phényl-thiazole-4-carbonyl)-pipéridin-2-ylméthyl]-benzamide ;
(S)-*N*-[1-(2-Cyclopropyl-5-phényl-thiazole-4-carbonyl)-pipéridin-2-ylméthyl]-2,5-diméthoxy-benzamide ;
[1-(2-Cyclopropyl-5-*m*-tolyl-thiazole-4-carbonyl)-pipéridin-2-ylméthyl]-amide de l'acide (S)-3-méthyl-isoxazole-5-carboxylique ;
[1-(2-Cyclopropyl-5-*m*-tolyl-thiazole-4-carbonyl)-pipéridin-2-ylméthyl]-amide de l'acide (S)-1,2-diméthyl-1*H*-indole-3-carboxylique ;
(S)-3-Acétylamino-*N*-[1-(2-cyclopropyl-5-*m*-tolyl-thiazole-4-carbonyl)-pipéridin-2-ylméthyl]-benzamide ;
[1-(2-Cyclopropyl-5-phényl-thiazole-4-carbonyl)-pipéridin-2-ylméthyl]-amide de l'acide (S)-4-méthyl-4*H*-furo[3,2-b]pyrrole-5-carboxylique ;
[1-(2-Cyclopropyl-5-*m*-tolyl-thiazole-4-carbonyl)-pipéridin-2-ylméthyl]-amide de l'acide (S)-3-méthyl-1*H*-indène-2-carboxylique ;
[1-(2-Cyclopropyl-5-phényl-thiazole-4-carbonyl)-pipéridin-2-ylméthyl]-amide de l'acide (S)-1-méthyl-1*H*-indole-4-carboxylique ;
[1-(2-Cyclopropyl-5-*m*-tolyl-thiazole-4-carbonyl)-pipéridin-2-ylméthyl]-amide de l'acide (S)-imidazo[1,2-a]pyridine-3-carboxylique ;
[1-(2-Cyclopropyl-5-phényl-thiazole-4-carbonyl)-pipéridin-2-ylméthyl]-amide de l'acide (S)-naphtalène-1-carboxylique ;
[1-(2-Cyclopropyl-5-*m*-tolyl-thiazole-4-carbonyl)-pipéridin-2-ylméthyl]-amide de l'acide (S)-5-fluoro-1-méthyl-1*H*-indole-2-carboxylique ;
[1-(2-Cyclopropyl-5-*m*-tolyl-thiazole-4-carbonyl)-pipéridin-2-ylméthyl]-amide de l'acide (S)-2,3-dihydro-benzo[1,4]dioxine-6-carboxylique ;
[1-(2-Cyclopropyl-5-phényl-thiazole-4-carbonyl)-pipéridin-2-ylméthyl]-amide de l'acide (S)-1-méthyl-1*H*-indole-5-carboxylique ;
(S)-*N*-[1-(2-Cyclopropyl-5-*m*-tolyl-thiazole-4-carbonyl)-pipéridin-2-ylméthyl]-4-méthyl-nicotinamide ;
(S)-2-Bromo-*N*-[1-(2-cyclopropyl-5-*m*-tolyl-thiazole-4-carbonyl)-pipéfidin-2-ylméthyl]-3-méthyl-benzamide ;
*(*S)-*N*-[1-(2-Cyclopropyl-5-phényl-thiazole-4-carbonyl)-pipéridin-2-ylméthyl]-3,4-diméthoxy-benzamide ;
(S)-*N*-[1-(2-Cyclopropyl-5-*m*-tolyl-thiazole-4-carbonyl)-pipéridin-2-ylméthyl]-2,3-diméthyl-benzamide ;
{1-[5-(3-Chloro-phényl)-2-cyclopropyl-thiazole-4-carbonyl]-pipéridin-2-ylméthyl}-amide de l'acide (S)-thiéno[2,3-b]pyrazine-6-carboxylique ;
{1-[2-Cyclopropyl-5-(3-trifluorométhyl-phényl)-thiazole-4-carbonyl]-pipéridin-2-ylméthyl}-amide de l'acide (S)-1-méthyl-1*H*-indole-4-carboxylique ; et
(S)-2-Chloro-*N*-[1-(2-cyclopropyl-5-*m*-tolyl-thiazole-4-carbonyl)-pipéridin-2-ylméthyl]-3-méthyl-benzamide ;
ou sel d'un tel composé.

10. Composition pharmaceutique qui comprend un composé de formule (I) selon l'une quelconque des revendications 1 à 9 ou un sel pharmaceutiquement acceptable de celui-ci, et un véhicule pharmaceutiquement acceptable.

11. Composé selon l'une quelconque des revendications 1 à 9 ou sel pharmaceutiquement acceptable de celui-ci, en vue d'une utilisation en tant que médicament.

12. Composé selon l'une quelconque des revendications 1 à 9 ou sel pharmaceutiquement acceptable de celui-ci, pour la prévention ou le traitement d'une maladie sélectionnée dans le groupe consistant en tous les types de troubles du sommeil, de syndromes liés à un stress, d'usage ou d'abus de substances psychoactives, de dysfonctionnements cognitifs dans la population saine et chez des patients atteints de maladies psychiatriques et neurologiques et de troubles du comportement alimentaire ou perturbations des apports de liquides.

13. Utilisation d'un composé selon l'une quelconque des revendications 1 à 9 ou d'un sel pharmaceutiquement acceptable de celui-ci dans la préparation d'une composition pharmaceutique pour le traitement de maladies ou d'affections sélectionnées dans le groupe consistant en des troubles du sommeil qui comprend tous les types d'insomnie, la narcolepsie et d'autres affections associées à une torpeur excessive, des dystonies nocturnes, le syndrome des jambes sans repos, les apnées du sommeil, le syndrome du décalage horaire, la fatigue et les divers autres malaises attribués au travail en équipes, le syndrome de retard ou d'avance de phase du sommeil ou les insomnies liées à des troubles psychiatriques.
